# EUROPEAN PATENT APPLICATION

(11) **EP 4 552 643 A2**
(43) Date of publication of application: **14.05.2025**
(21) Application number: 24214548.0
(22) Date of filing: 02.07.2019
(51) Int. Cl.: A61K 31/501

(54) **SOLID FORMS OF 2-(3,5-DICHLORO-4-((5-ISOPROPYL-6-OXO-1,6-DIHYDROPYRIDAZIN-3-YL)OXY)PHENYL)-3,5-DIOXO-2,3,4,5-TETRAHYDRO-1,2,4-TRIAZINE-6-CARBONITRILE**

(30) Priority: 02.07.2018 US 201862692914 P
(62) Divisional of application: 19745399.6
(71) Applicant: Madrigal Pharmaceuticals, Inc., West Conshohocken, PA 19428 (US)
(72) Inventor: MIRMEHRABI, Mahmoud, Halifax, B3J 3M5 (CA); JONAS, Marco, Cohoes, 12047 (US); BATCHU, Pavan Karthik, Halifax, B3J 3M5 (CA)
(74) Representative: Carpmaels & Ransford LLP

(57) **Abstract**

The present invention is directed to morphic forms, co-crystals, salts, and amorphous solid dispersions of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile.

## Description

### Cross-Reference to Related Applications

This application claims the benefit of priority to U.S. Provisional Application No. 62/692,914, filed on July 2, 2018, the contents of which are hereby incorporated by reference in their entireties.

### Incorporation by Reference of Sequence Listing

The contents of the text file named "MDRI-024_SEQ_LISTING.txt", which was created on May 21, 2019 and is 3.66 KB in size, are hereby incorporated by reference in their entireties.

### Field of the Invention

The present invention relates to morphic forms, co-crystals, salts, and amorphous solid dispersions of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (Compound A).

### Background of the Invention

Thyroid hormones are critical for normal growth and development and for maintaining metabolic homeostasis (Paul M. Yen, Physiological reviews, Vol. 81(3): pp. 1097-1126 (2001)). Circulating levels of thyroid hormones are tightly regulated by feedback mechanisms in the hypothalamus/pituitary/thyroid (HPT) axis. Thyroid dysfunction leading to hypothyroidism or hyperthyroidism clearly demonstrates that thyroid hormones exert profound effects on cardiac function, body weight, metabolism, metabolic rate, body temperature, cholesterol, bone, muscle and behavior.

The biological activity of thyroid hormones is mediated by thyroid hormone receptors (TRs or THRs) (M. A. Lazar, Endocrine Reviews, Vol. 14: pp. 348-399 (1993)). TRs belong to the superfamily known as nuclear receptors. TRs form heterodimers with the retinoid receptor that act as ligand-inducible transcription factors. TRs have a ligand binding domain, a DNA binding domain, and an amino terminal domain, and regulate gene expression through interactions with DNA response elements and with various nuclear co-activators and co-repressors. The thyroid hormone receptors are derived from two separate genes, α and β. These distinct gene products produce multiple forms of their respective receptors through differential RNA processing. The major thyroid receptor isoforms are α1, α2, β1, and β2. Thyroid hormone receptors α1, β1, and β2 bind thyroid hormone. It has been shown that the thyroid hormone receptor subtypes can differ in their contribution to particular biological responses. Recent studies suggest that TRβ1 plays an important role in regulating TRH (thyrotropin releasing hormone) and on regulating thyroid hormone actions in the liver. TRβ2 plays an important role in the regulation of TSH (thyroid stimulating hormone) (Abel et. al., J. Clin. Invest., Vol 104: pp. 291-300 (1999)). TRβ1 plays an important role in regulating heart rate (B. Gloss et. al. Endocrinology, Vol. 142: pp. 544-550 (2001); C. Johansson et. al., Am. J. Physiol., Vol. 275: pp. R640-R646 (1998)).

Efforts have been made to synthesize thyroid hormone analogs which exhibit increased thyroid hormone receptor beta selectivity and/or tissue selective action. Such thyroid hormone mimetics may yield desirable reductions in body weight, lipids, cholesterol, and lipoproteins, with reduced impact on cardiovascular function or normal function of the hypothalamus/pituitary/thyroid axis (see, e.g., Joharapurkar et al., J. Med. Chem., 2012, 55 (12), pp 5649-5675). The development of thyroid hormone analogs which avoid the undesirable effects of hyperthyroidism and hypothyroidism while maintaining the beneficial effects of thyroid hormones would open new avenues of treatment for patients with metabolic disease such as obesity, hyperlipidemia, hypercholesterolemia, diabetes and other disorders and diseases such as liver steatosis and NASH, atherosclerosis, cardiovascular diseases, hypothyroidism, thyroid cancer, thyroid diseases, a resistance to thyroid hormone (RTH) syndrome, and related disorders and diseases.

### Summary of the Invention

The present disclosure provides morphic forms, co-crystals, salts, and amorphous solid dispersions of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (Compound A).

One aspect of the present disclosure relates to a crystalline salt of Compound A. Another aspect of the present disclosure relates to a pharmaceutical composition comprising the crystalline salt disclosed herein.

In some embodiments, the crystalline salt is characterized as having a counter-ion, wherein the counter-ion is selected from L-lysine, L-arginine, 2-hydroxy-N,N,N-trimethylethan-1-aminium, diethylamine, ethanolamine, ethanol-2-diethylamine, Na⁺, Mg²⁺, K⁺, Ca²⁺, diethanolamine, triethanolamine, L-histidine, and meglumine.

In some embodiments, the counter-ion is L-lysine.

In some embodiments, the counter-ion is L-arginine.

In some embodiments, the counter-ion is 2-hydroxy-N,N,N-trimethylethan-1-aminium.

In some embodiments, the crystalline salt (L-lysine salt) is characterized by an X-ray powder diffraction pattern including peaks at about 8.70, 9.22, 11.3, 17.0, and 24.8 degrees 2θ, wherein the x-ray powder diffraction pattern is obtained using a Cu Kα radiation source (1.54 Å).

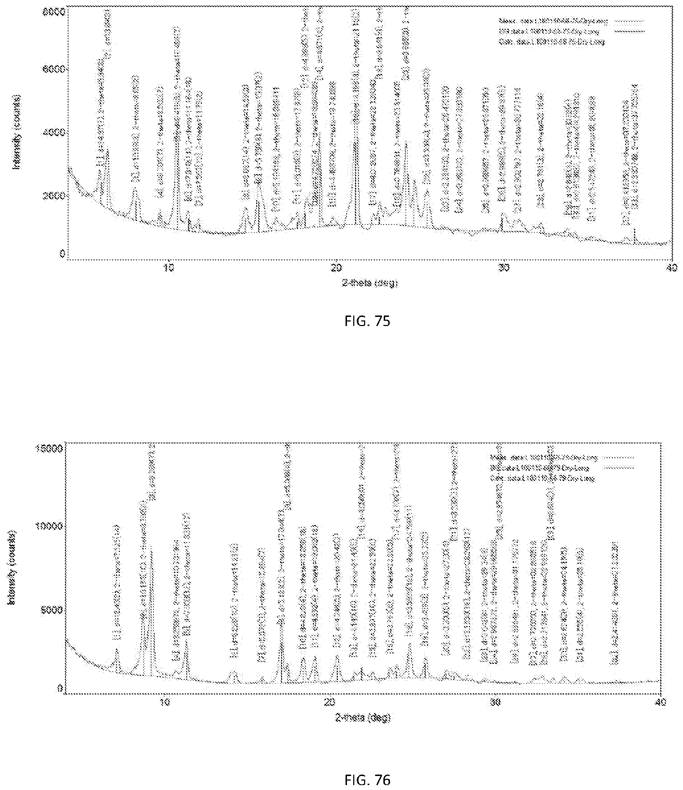

In some embodiments, the crystalline salt has an X-ray diffraction pattern substantially similar to that set forth in FIG. 76.

In some embodiments, the crystalline salt has a melting point of about 250 °C.

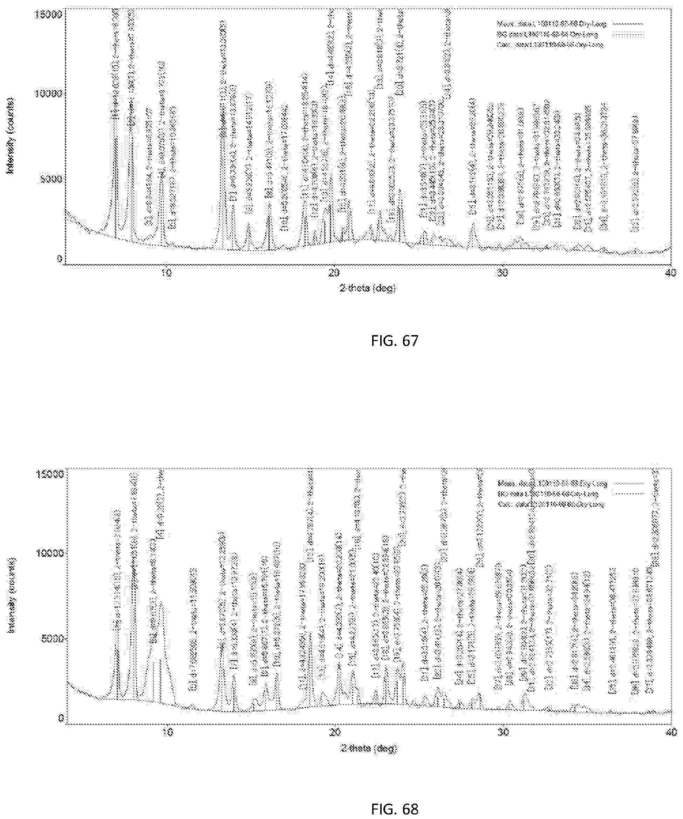

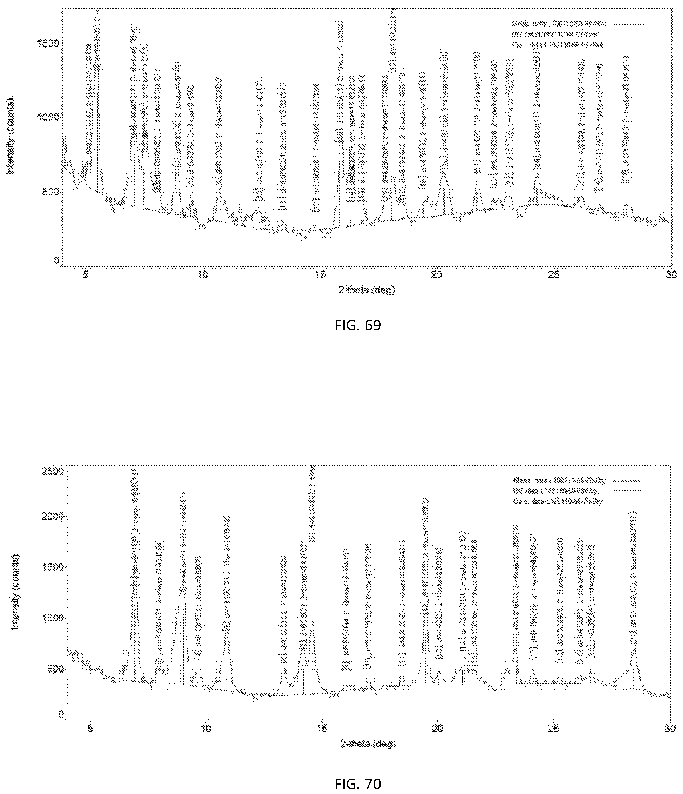

In some embodiments, the crystalline salt has an X-ray diffraction pattern substantially similar to that set forth in any one of FIGs. 67-70.

In some embodiments, the crystalline salt has a melting point of about 200 °C.

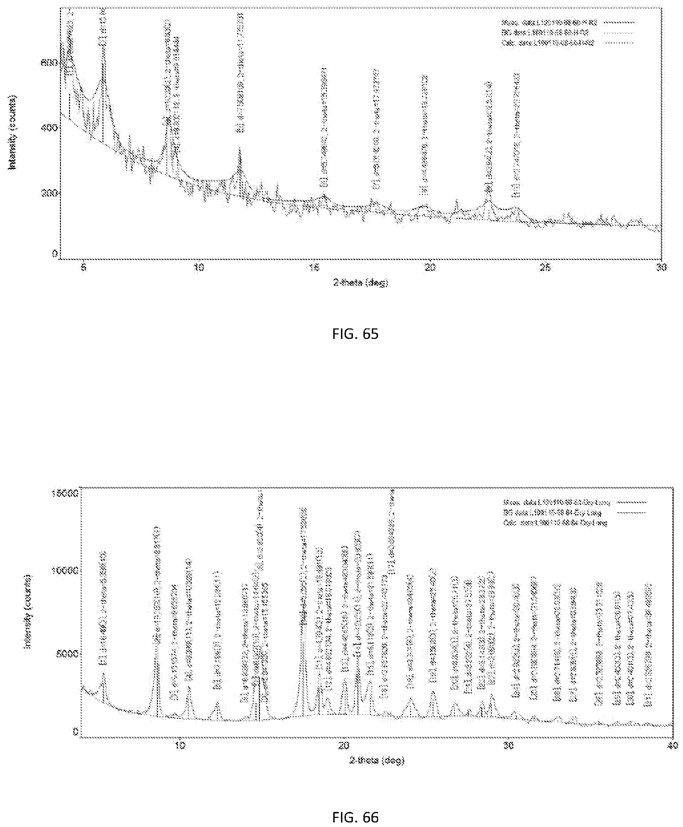

In some embodiments, the crystalline salt has an X-ray diffraction pattern substantially similar to that set forth in FIG. 66.

In some embodiments, the crystalline salt has a melting point of about 229 °C.

In some embodiments, the crystalline salt has purity of Compound A of greater than 90% by weight.

In some embodiments, the crystalline salt has purity of Compound A of greater than 95% by weight.

In some embodiments, the crystalline salt has purity of Compound A of greater than 99% by weight.

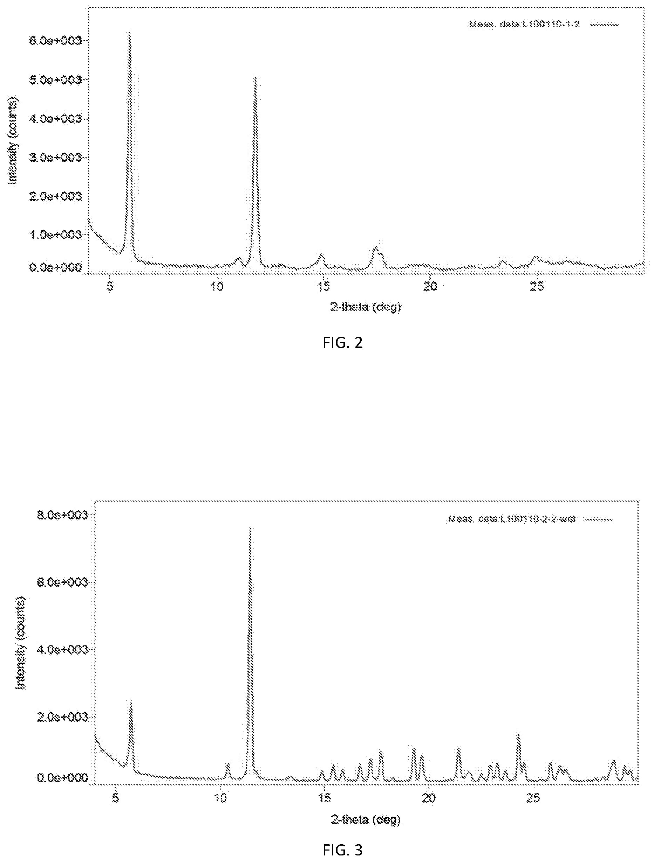

Another aspect of the present disclosure relates to a morphic form (Form B) of Compound A, characterized by an X-ray powder diffraction pattern including peaks at about 5.92, 11.8, and 17.5 degrees 2θ, wherein the X-ray powder diffraction pattern is obtained using a Cu Kα radiation source (1.54 Å). In some embodiments, Form B has an X-ray diffraction pattern substantially similar to that set forth in FIG. 2.

Another aspect of the present disclosure relates to a morphic form (Form C) of Compound A, characterized by an X-ray powder diffraction pattern including peaks at about 5.74, 11.5, 17.7, 19.3, 19.7, 21.4, 24.3 degrees 2θ, wherein the x-ray powder diffraction pattern is obtained using a Cu Kα radiation source (1.54 Å). In some embodiments, Form C has an X-ray diffraction pattern substantially similar to that set forth in FIG. 3.

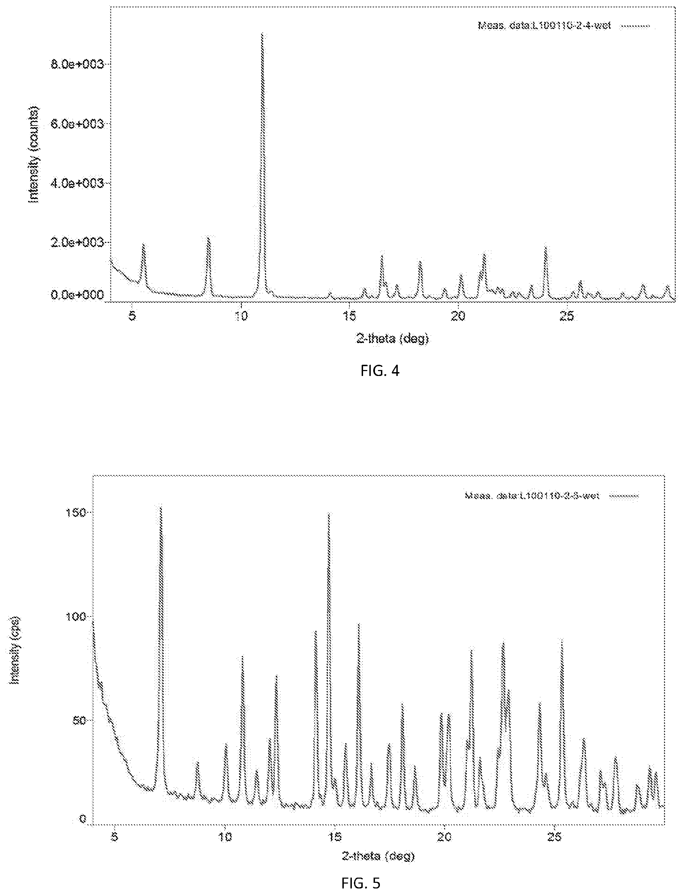

Another aspect of the present disclosure relates to a morphic form (Form D) of Compound A, characterized by an X-ray powder diffraction pattern including peaks at about 5.52, 8.52, 11.0, 16.5, 18.3, 21.0, 21.2, and 24.0 degrees 2θ, wherein the x-ray powder diffraction pattern is obtained using a Cu Kα radiation source (1.54 Å). In some embodiments, Form D has an X-ray diffraction pattern substantially similar to that set forth in FIG. 4.

Another aspect of the present disclosure relates to a morphic form (Form E) of Compound A, characterized by an X-ray powder diffraction pattern including peaks at about 7.13, 10.8, 12.3, 14.1, 14.7, 15.5, 16.1, 17.5, 18.1, 19.9, 20.2, 21.0, 21.2, 22.7, 22.9, 24.4, 25.3, and 26.3 degrees 2θ, wherein the x-ray powder diffraction pattern is obtained using a Cu Kα radiation source (1.54 Å). In some embodiments, Form E has an X-ray diffraction pattern substantially similar to that set forth in FIG. 5.

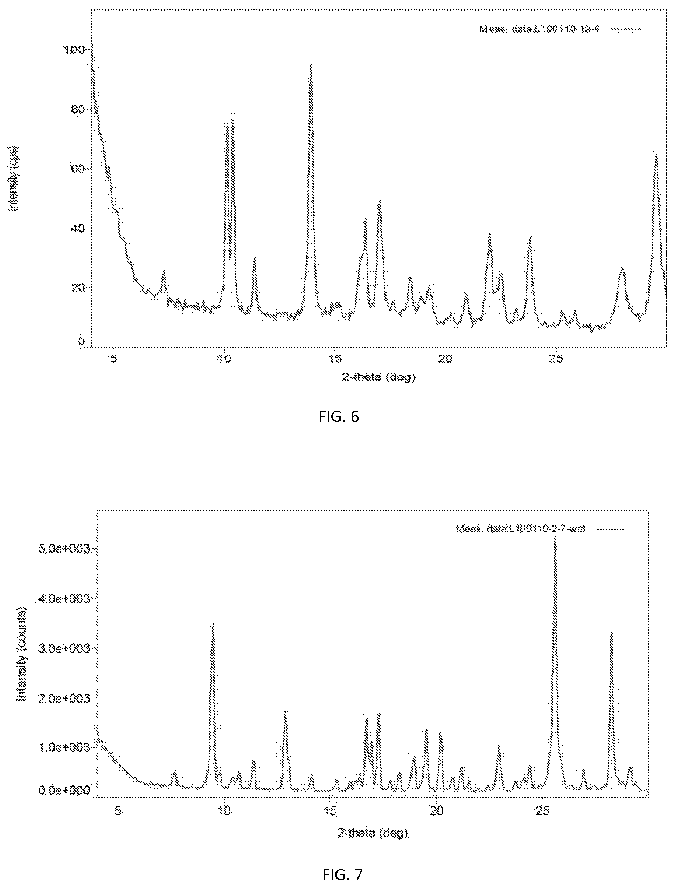

Another aspect of the present disclosure relates to a morphic form (Form F) of Compound A, characterized by an X-ray powder diffraction pattern including peaks at about 10.1, 10.4, 11.4, 13.9, 16.2, 16.4, 17.1, 22.0, 23.8, and 29.5 degrees 2θ, wherein the x-ray powder diffraction pattern is obtained using a Cu Kα radiation source (1.54 Å). In some embodiments, Form F has an X-ray diffraction pattern substantially similar to that set forth in FIG. 6.

Another aspect of the present disclosure relates to a morphic form (Form G) of Compound A, characterized by an X-ray powder diffraction pattern including peaks at about 9.50, 12.9, 16.7, 17.3, 19.5, 20.2, 25.6, and 28.3 degrees 2θ, wherein the x-ray powder diffraction pattern is obtained using a Cu Kα radiation source (1.54 Å). In some embodiments, Form G has an X-ray diffraction pattern substantially similar to that set forth in FIG. 7.

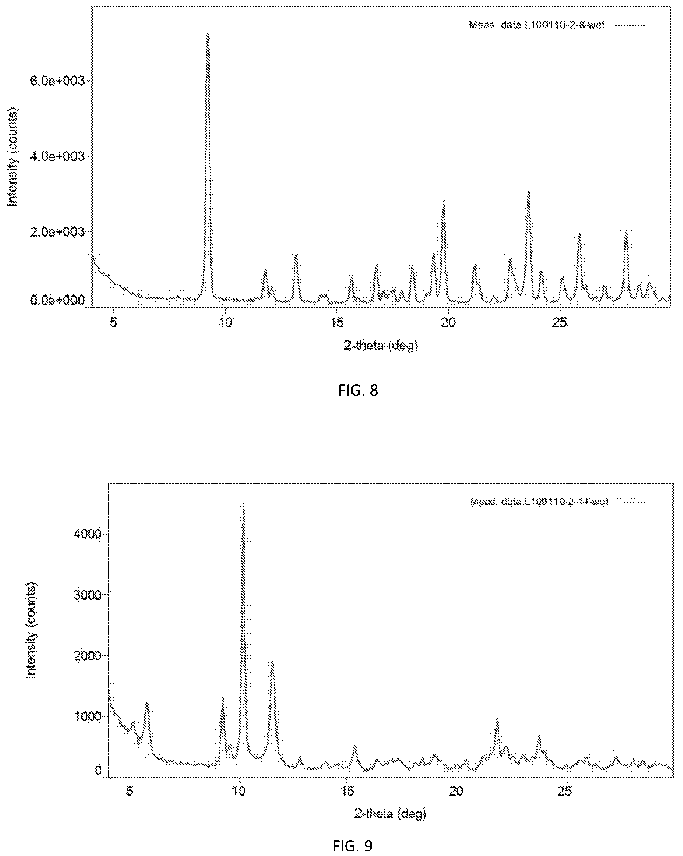

Another aspect of the present disclosure relates to a morphic form (Form H) of Compound A, characterized by an X-ray powder diffraction pattern including peaks at about 9.22, 19.8, 23.6, 25.9, and 28.0 degrees 2θ, wherein the x-ray powder diffraction pattern is obtained using a Cu Kα radiation source (1.54 Å). In some embodiments, Form H has an X-ray diffraction pattern substantially similar to that set forth in FIG. 8.

Another aspect of the present disclosure relates to a morphic form (Form I) of Compound A, characterized by an X-ray powder diffraction pattern including peaks at about 5.77, 9.30, 10.2, 11.6, and 21.9 degrees 2θ, wherein the x-ray powder diffraction pattern is obtained using a Cu Kα radiation source (1.54 Å). In some embodiments, Form I has an X-ray diffraction pattern substantially similar to that set forth in FIG. 9.

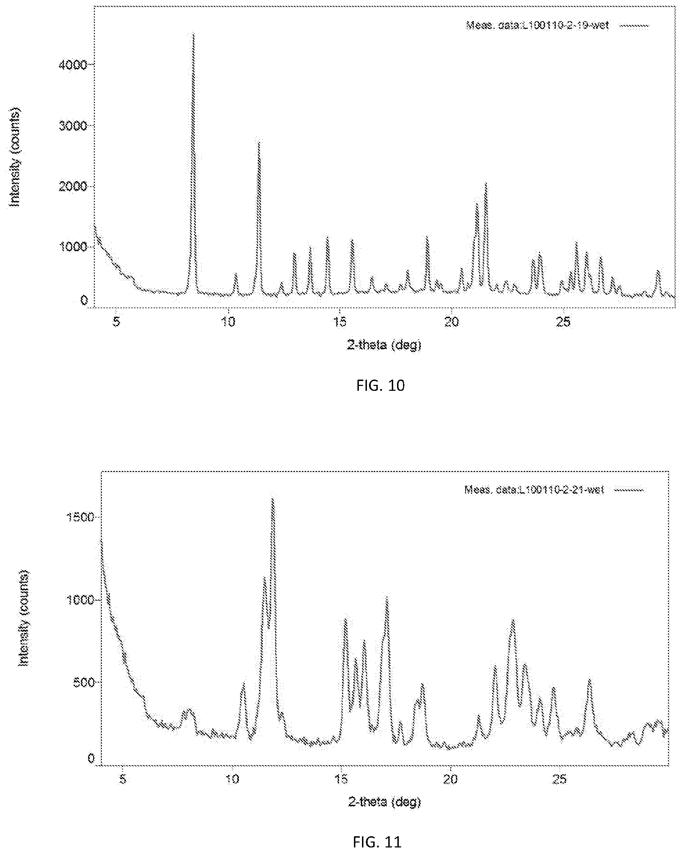

Another aspect of the present disclosure relates to a morphic form (Form K) of Compound A, characterized by an X-ray powder diffraction pattern including peaks at about 8.42, 11.4, 14.5, 18.9, 21.1, and 21.6 degrees 2θ, wherein the x-ray powder diffraction pattern is obtained using a Cu Kα radiation source (1.54 Å). In some embodiments, Form K has an X-ray diffraction pattern substantially similar to that set forth in FIG. 10.

Another aspect of the present disclosure relates to a morphic form (Form L) of Compound A, characterized by an X-ray powder diffraction pattern including peaks at about 10.5, 11.5, 11.9, 15.2, 15.7, 16.0, 16.9, 17.1, 18.4, 18.7, 22.0, 22.8, 23.5, and 26.4 degrees 2θ, wherein the x-ray powder diffraction pattern is obtained using a Cu Kα radiation source (1.54 Å). In some embodiments, Form L has an X-ray diffraction pattern substantially similar to that set forth in FIG. 11.

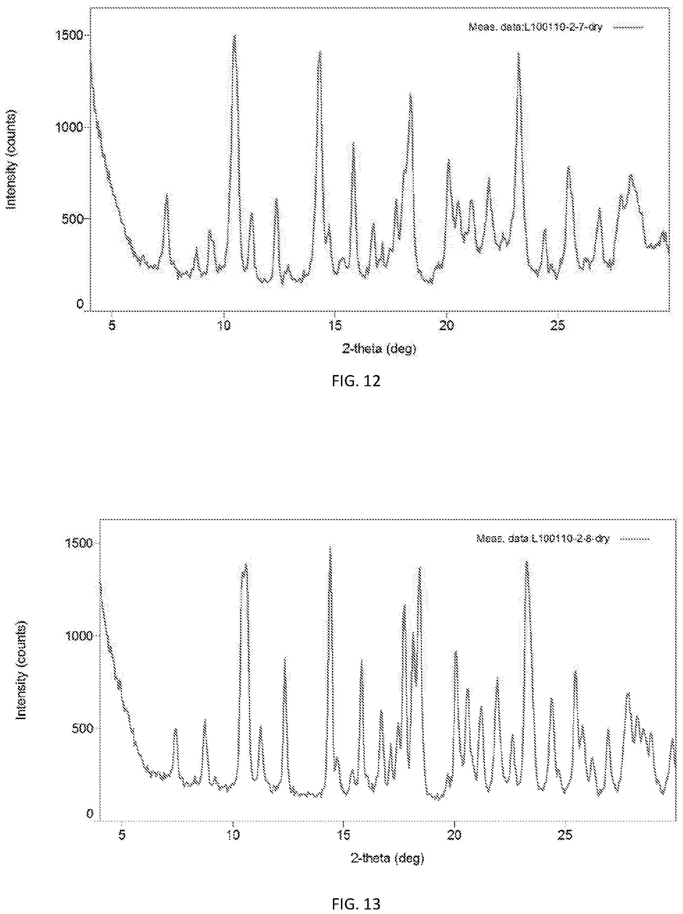

Another aspect of the present disclosure relates to a morphic form (Form S+T) of Compound A, characterized by an X-ray powder diffraction pattern including peaks at about 7.42, 10.5, 11.3, 12.4, 14.3, 15.8, 16.8, 17.7, 18.1, 18.4, 20.1, 20.5, 21.1, 21.9, 23.2, 25.5, 26.9, and 28.3 degrees 2θ, wherein the x-ray powder diffraction pattern is obtained using a Cu Kα radiation source (1.54 Å). In some embodiments, Form S+T has an X-ray diffraction pattern substantially similar to that set forth in FIG. 12.

Another aspect of the present disclosure relates to a morphic form (Form S) of Compound A, characterized by an X-ray powder diffraction pattern including peaks at about 10.5, 12.3, 14.4, 15.8, 16.7, 17.7, 18.1, 18.4, 20.1, 20.6, 21.2, 21.9, 23.3, 24.4, 25.5, and 27.8 degrees 2θ, wherein the x-ray powder diffraction pattern is obtained using a Cu Kα radiation source (1.54 Å). In some embodiments, Form S has an X-ray diffraction pattern substantially similar to that set forth in FIG. 13.

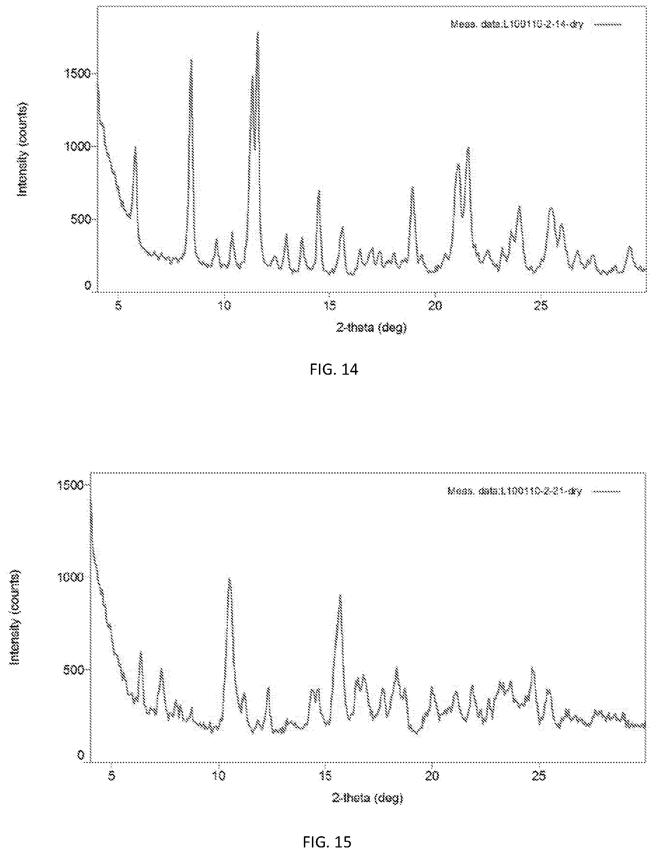

Another aspect of the present disclosure relates to a morphic form (Form U) of Compound A, characterized by an X-ray powder diffraction pattern including peaks at about 5.79, 8.43, 11.4, 11.6, 14.5, 18.9, 21.1, and 21.6 degrees 2θ, wherein the x-ray powder diffraction pattern is obtained using a Cu Kα radiation source (1.54 Å). In some embodiments, Form U has an X-ray diffraction pattern substantially similar to that set forth in FIG. 14.

Another aspect of the present disclosure relates to a morphic form (Form V) of Compound A, characterized by an X-ray powder diffraction pattern including peaks at about 6.35, 10.6, 15.6, 16.5, 16.8, and 18.3 degrees 2θ, wherein the x-ray powder diffraction pattern is obtained using a Cu Kα radiation source (1.54 Å). In some embodiments, Form V has an X-ray diffraction pattern substantially similar to that set forth in FIG. 15.

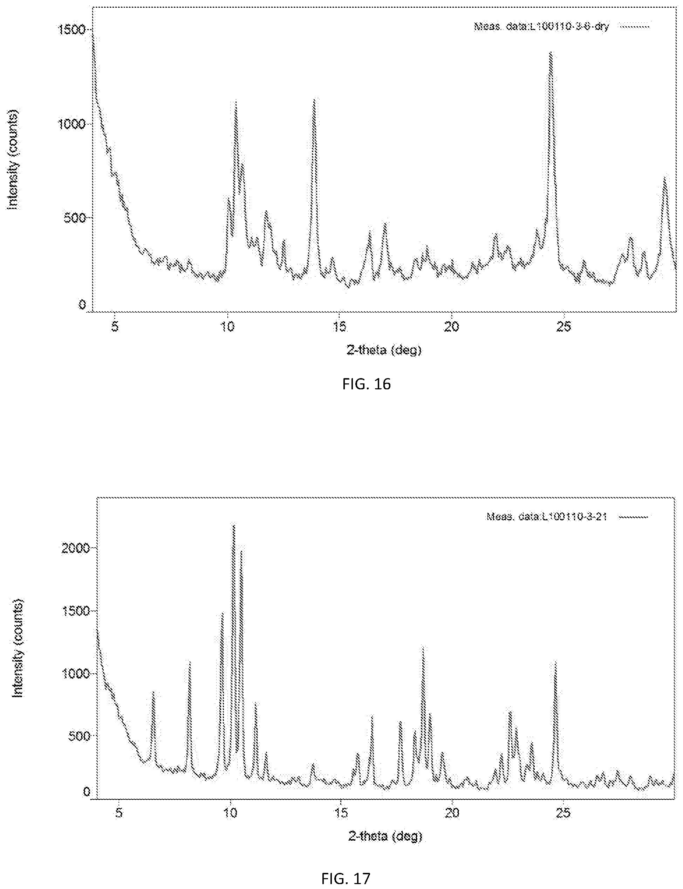

Another aspect of the present disclosure relates to a morphic form (Form W) of Compound A, characterized by an X-ray powder diffraction pattern including peaks at about 10.1, 10.4, 10.7, 11.7, 13.9, 24.4, and 29.5 degrees 2θ, wherein the x-ray powder diffraction pattern is obtained using a Cu Kα radiation source (1.54 Å). In some embodiments, Form W has an X-ray diffraction pattern substantially similar to that set forth in FIG. 16.

Another aspect of the present disclosure relates to a morphic form (Form X) of Compound A, characterized by an X-ray powder diffraction pattern including peaks at about 9.66, 10.2, 10.5, 11.2, 18.7, and 24.7 degrees 2θ, wherein the x-ray powder diffraction pattern is obtained using a Cu Kα radiation source (1.54 Å). In some embodiments, Form X has an X-ray diffraction pattern substantially similar to that set forth in FIG. 17.

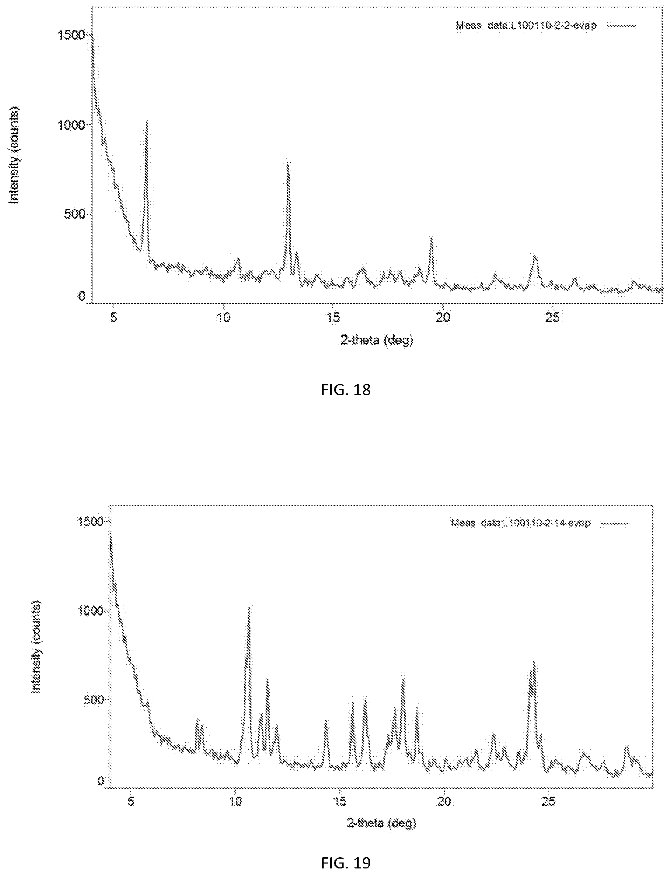

Another aspect of the present disclosure relates to a morphic form (Form Y) of Compound A, characterized by an X-ray powder diffraction pattern including peaks at about 6.51, 13.0, 13.3, 19.5, and 24.2 degrees 2θ, wherein the x-ray powder diffraction pattern is obtained using a Cu Kα radiation source (1.54 Å). In some embodiments, Form Y has an X-ray diffraction pattern substantially similar to that set forth in FIG. 18.

Another aspect of the present disclosure relates to a morphic form (Form Z) of Compound A, characterized by an X-ray powder diffraction pattern including peaks at about 10.6, 11.2, 11.6, 12.0, 14.3, 15.6, 16.2, 17.6, 18.1, 18.7, 24.1, and 24.3 degrees 2θ, wherein the x-ray powder diffraction pattern is obtained using a Cu Kα radiation source (1.54 Å). In some embodiments, Form Z has an X-ray diffraction pattern substantially similar to that set forth in FIG. 19.

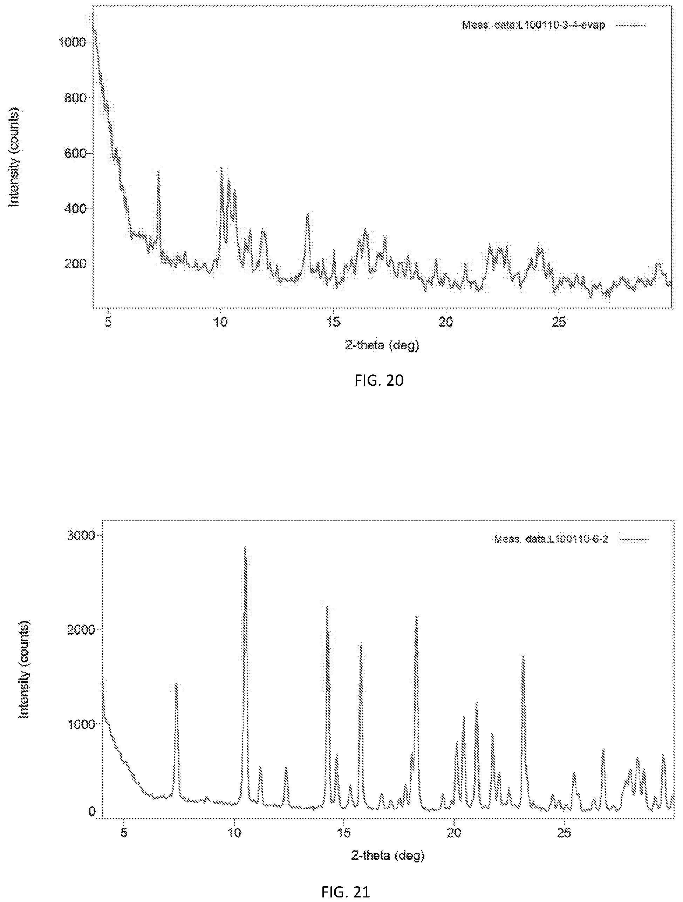

Another aspect of the present disclosure relates to a morphic form (Form α) of Compound A, characterized by an X-ray powder diffraction pattern including peaks at about 7.26, 10.1, 10.4, 10.6, 11.9, 13.9, 16.5, 21.9, 22.4, and 24.1 degrees 2θ, wherein the x-ray powder diffraction pattern is obtained using a Cu Kα radiation source (1.54 Å). In some embodiments, Form α has an X-ray diffraction pattern substantially similar to that set forth in FIG. 20.

Another aspect of the present disclosure relates to a morphic form (Form β) of Compound A, characterized by an X-ray powder diffraction pattern including peaks at about 7.36, 10.5, 14.3, 15.7, 18.3, 20.4, 21.0, 21.8, and 23.2 degrees 2θ, wherein the x-ray powder diffraction pattern is obtained using a Cu Kα radiation source (1.54 Å). In some embodiments, Form β has an X-ray diffraction pattern substantially similar to that set forth in FIG. 21.

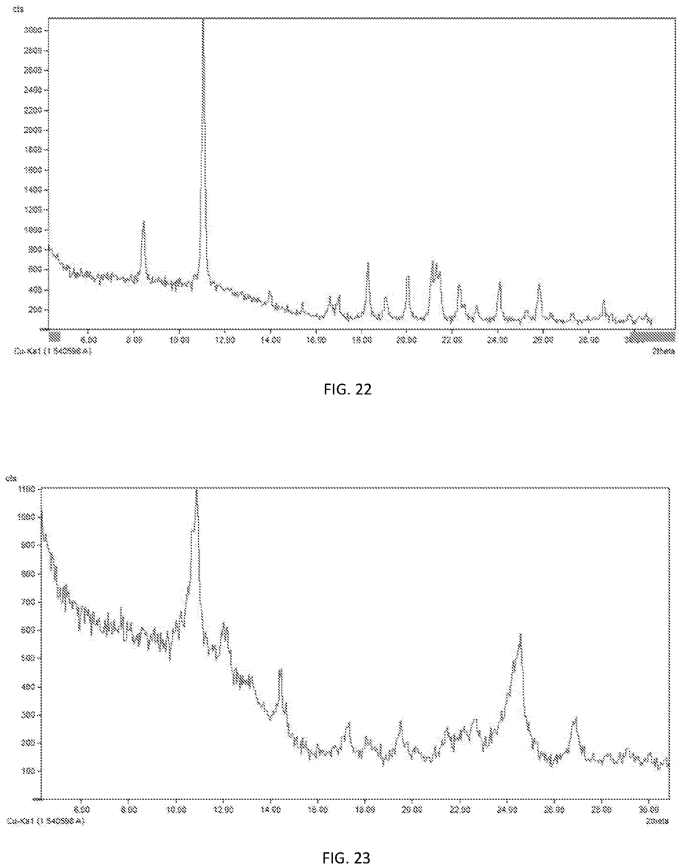

Another aspect of the present disclosure relates to a morphic form (Form χ) of Compound A, characterized by an X-ray powder diffraction pattern including peaks at about 8.53, 11.2, 18.4, 20.1, and 21.4 degrees 2θ, wherein the x-ray powder diffraction pattern is obtained using a Cu Kα radiation source (1.54 Å). In some embodiments, Form χ has an X-ray diffraction pattern substantially similar to that set forth in FIG. 22.

Another aspect of the present disclosure relates to a morphic form (Form δ) of Compound A, characterized by an X-ray powder diffraction pattern including peaks at about 10.9, 12.1, 14.4, 18.1, 19.6, 24.5, and 27.0 degrees 2θ, wherein the x-ray powder diffraction pattern is obtained using a Cu Kα radiation source (1.54 Å). In some embodiments, Form δ has an X-ray diffraction pattern substantially similar to that set forth in FIG. 23.

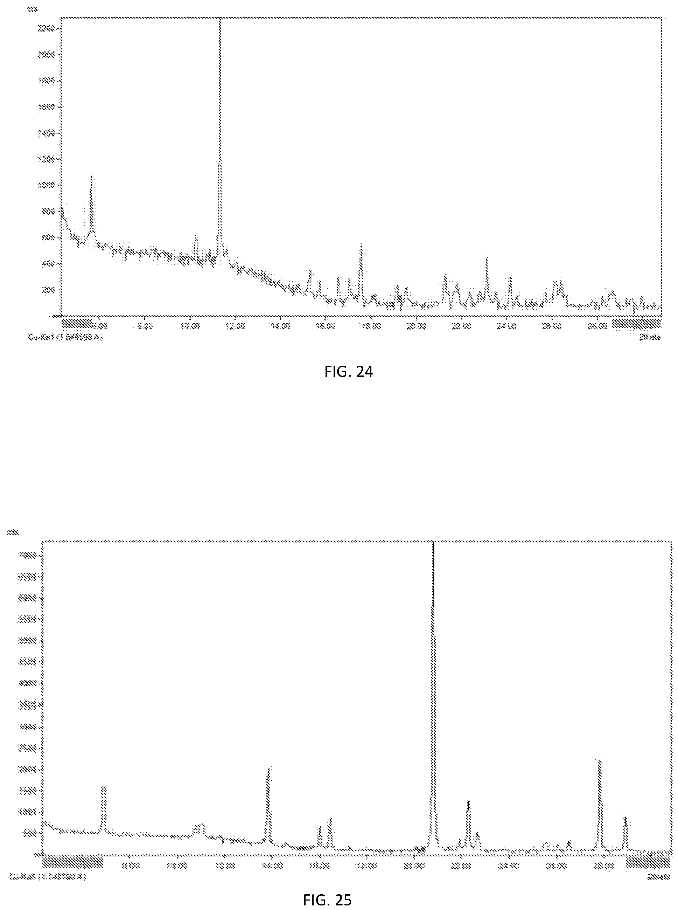

Another aspect of the present disclosure relates to a morphic form (Form ε) of Compound A, characterized by an X-ray powder diffraction pattern including peaks at about 5.73, 11.4, 16.6, 17.6, 23.2, and 24.2 degrees 2θ, wherein the x-ray powder diffraction pattern is obtained using a Cu Kα radiation source (1.54 Å). In some embodiments, Form ε has an X-ray diffraction pattern substantially similar to that set forth in FIG. 24.

Another aspect of the present disclosure relates to a morphic form (Form ϕ) of Compound A, characterized by an X-ray powder diffraction pattern including peaks at about 6.95, 13.9, 20.9, 22.3, and 27.9 degrees 2θ, wherein the x-ray powder diffraction pattern is obtained using a Cu Kα radiation source (1.54 Å). In some embodiments, Form ϕ has an X-ray diffraction pattern substantially similar to that set forth in FIG. 25.

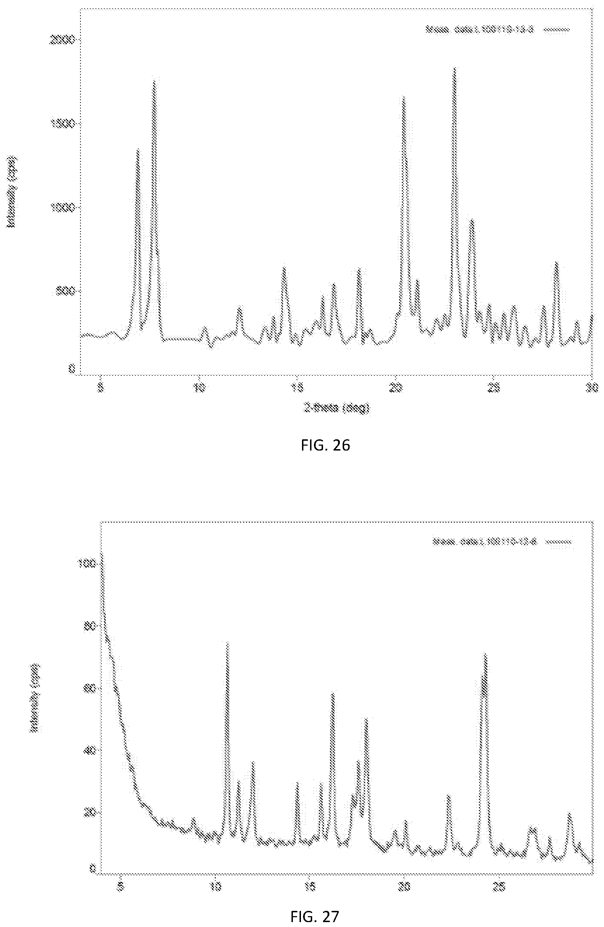

Another aspect of the present disclosure relates to a morphic form (Form η) of Compound A, characterized by an X-ray powder diffraction pattern including peaks at about 6.87, 7.69, 20.5, 23.0, 23.9, and 28.2 degrees 2θ, wherein the x-ray powder diffraction pattern is obtained using a Cu Kα radiation source (1.54 Å). In some embodiments, Form η has an X-ray diffraction pattern substantially similar to that set forth in FIG. 26.

Another aspect of the present disclosure relates to a morphic form (Form λ) of Compound A, characterized by an X-ray powder diffraction pattern including peaks at about 10.6, 12.0, 14.3, 16.2, 17.6, 18.0, and 24.3 degrees 2θ, wherein the x-ray powder diffraction pattern is obtained using a Cu Kα radiation source (1.54 Å). In some embodiments, Form λ has an X-ray diffraction pattern substantially similar to that set forth in FIG. 27.

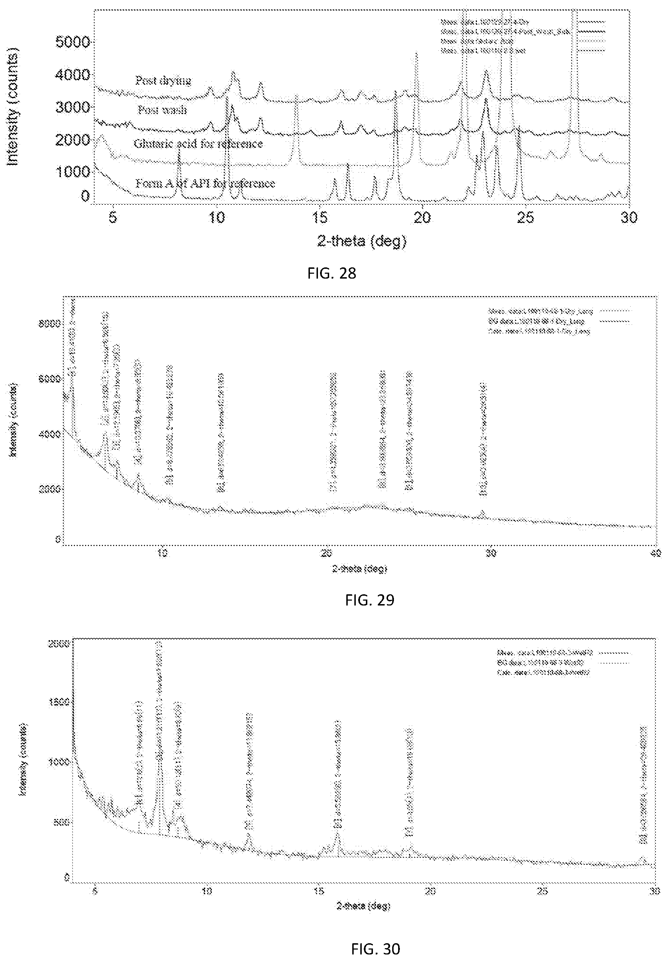

Another aspect of the present disclosure relates to a co-crystal of Compound A and glutaric acid, characterized by an X-ray powder diffraction pattern including peaks at about 9.74, 10.8, 11.0, 12.2, 16.1, 17.0, 19.2, 21.9, and 23.1 degrees 2θ, wherein the x-ray powder diffraction pattern is obtained using a Cu Kα radiation source (1.54 Å). In some embodiments, the co-crystal has an X-ray diffraction pattern substantially similar to that set forth in FIG. 28.

Another aspect of the present disclosure relates to an amorphous solid dispersion of Compound A, wherein the amorphous solid dispersion comprises a polymer. In some embodiments, the polymer is polyvinylpyrrolidone. In some embodiments, the weight ratio of Compound A over the polymer is about 1:2 or 1:4.

The crystalline salt, morphic form, co-crystal, or amorphous solid dispersion of the present disclosure can be used in (a) a method for treating a resistance to thyroid hormone (RTH) syndrome; (b) a method for treating non-alcoholic steatohepatitis; (c) a method for treating familial hypercholesterolemia; (d) a method for treating fatty liver disease; and (e) a method for treating dyslipidemia. In some embodiments, the crystalline salt, morphic form, co-crystal, or amorphous solid dispersion is administered daily.

Another aspect of the disclosure relates to crystalline salt, morphic form, co-crystal, or amorphous solid dispersion of the present disclosure for the manufacture of a medicament for treating: (a) resistance to thyroid hormone (RTH) syndrome; (b) non-alcoholic steatohepatitis; (c) familial hypercholesterolemia; (d) fatty liver disease; and (e) treating dyslipidemia, wherein the crystalline salt, morphic form, co-crystal, or amorphous solid dispersion of the present disclosure is for administration to the subject in at least one therapeutically effective amount. In some embodiments, the the crystalline salt, morphic form, co-crystal, or amorphous solid dispersion is administered daily.

Another aspect of the disclosure relates to crystalline salt, morphic form, co-crystal, or amorphous solid dispersion of the present disclosure for use in treating: (a) resistance to thyroid hormone (RTH) syndrome; (b) non-alcoholic steatohepatitis; (c) familial hypercholesterolemia; (d) fatty liver disease; and (e) treating dyslipidemia, wherein the crystalline salt, morphic form, co-crystal, or amorphous solid dispersion of the present disclosure is for administration to the subject in at least one therapeutically effective amount. In some embodiments, the the crystalline salt, morphic form, co-crystal, or amorphous solid dispersion is administered daily.

### Brief Description of the Drawings

FIG. 1 is an X-ray powder diffraction (XRPD) pattern of an anhydrous crystalline form (Form A) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (Compound A).
FIG. 2 is an XRPD pattern of a methanol solvate (Form B) of Compound A.
FIG. 3 is an XRPD pattern of an ethanol solvate (Form C) of Compound A.
FIG. 4 is an XRPD pattern of an acetone solvate (Form D) of Compound A.
FIG. 5 is an XRPD pattern of a tetrahydrofuran solvate (Form E) of Compound A.
FIG. 6 is an XRPD pattern of an ethyl acetate desolvate (Form F) of Compound A.
FIG. 7 is an XRPD pattern of a methyl isobutyl ketone (MIBK) solvate (Form G) of Compound A.
FIG. 8 is an XRPD pattern of an isopropyl acetate (IPAc) solvate (Form H) of Compound A.
FIG. 9 is an XRPD pattern of an acetic acid solvate (Form I) of Compound A.
FIG. 10 is an XRPD pattern of a dimethyl acetamide solvate (Form K) of Compound A.
FIG. 11 is an XRPD pattern of an acetonitrile solvate (Form L) of Compound A.
FIG. 12 is an XRPD pattern of a MIBK desolvate (Form S+T) of Compound A.
FIG. 13 is an XRPD pattern of an IPAc desolvate (Form S) of Compound A.
FIG. 14 is an XRPD pattern of an acetic acid desolvate (Form U) of Compound A.
FIG. 15 is an XRPD pattern of an acetonitrile desolvate (Form V) of Compound A.
FIG. 16 is an XRPD pattern of an ethyl acetate desolvate (Form W) of Compound A.
FIG. 17 is an XRPD pattern of an acetonitrile solvate (Form X) of Compound A.
FIG. 18 is an XRPD pattern of an ethanol desolvate (Form Y) of Compound A.
FIG. 19 is an XRPD pattern of an acetic acid desolvate (Form Z) of Compound A.
FIG. 20 is an XRPD pattern of an acetone desolvate (Form α) of Compound A.
FIG. 21 is an XRPD pattern of an N-methylpyrrolidone (NMP) solvate (Form β) of Compound A.
FIG. 22 is an XRPD pattern of a dimethyl sulfoxide (DMSO) solvate (Form χ) of Compound A.
FIG. 23 is an XRPD pattern of a possible THF solvate (Form δ) of Compound A.
FIG. 24 is an XRPD pattern of a mixture of Form C and a possible acetone solvate (Form ε) of Compound A.
FIG. 25 is an XRPD pattern of an acetone solvate (Form ϕ) of Compound A.
FIG. 26 is an XRPD pattern of an IPA solvate (Form η) of Compound A.
FIG. 27 is an XRPD pattern of an IPAc desolvate (Form λ) of Compound A.
FIG. 28 is an XRPD pattern of the cocrystal obtained from heating a mixture of Form A and glutaric acid showing no change before and after drying. The patterns of Form A and the co-former glutaric acid are provided for comparison.
FIG. 29 is an XRPD pattern of the Calcium salt of Compound A from the experiment L100110-68-1 along with peak positions (Form 1-A).
FIG. 30 is an XRPD pattern of the Calcium salt of Compound A from the experiment L100110-68-3-Wet along with peak positions (Form 1-B).
FIG. 31 is an XRPD pattern of the Calcium salt of Compound A from the experiment L100110-68-8 along with peak positions (Form 2-B).
FIG. 32 is an XRPD pattern of the Calcium salt of Compound A from the experiment L100110-68-10 after exposing to saturated humidity environment along with peak positions (Form 2-D).
FIG. 33 is an XRPD pattern of the Magnesium salt of Compound A from the experiment L100110-68-11-Dry along with peak positions (3-A).
FIG. 34 is an XRPD pattern of the Magnesium salt of Compound A from the experiment L100110-68-11 upon exposing it to saturated humidity environment at room temperature along with peak positions (3-C).
FIG. 35 is an XRPD pattern of the Magnesium salt of Compound A from the experiment L100110-68-13 before drying (3-B).
FIG. 36 is an XRPD pattern of the Magnesium salt of Compound A from the experiment L100110-68-13, dried after deliquescing (3-D).
FIG. 37 is an XRPD pattern of the Magnesium salt of Compound A from the experiment L100110-68-17, before drying (Form 4-B).
FIG. 38 is an XRPD pattern of the Magnesium salt of Compound A from the experiment L100110-68-20, before drying (Form 4-D).
FIG. 39 is an XRPD pattern of the Magnesium salt of Compound A from the experiment L100110-68-20, after drying (Form 4-E).
FIG. 40 is an XRPD pattern of the Sodium salt of Compound A from the experiment L100110-68-21 along with peak positions (Form 5-A).
FIG. 41 is an XRPD pattern of the Sodium salt of Compound A from the experiment L100110-68-24 along with peak positions (Form 5-B).
FIG. 42 is an XRPD pattern of the Sodium salt of Compound A from the experiment L100110-68-25 along with peak positions (Form 5-C).
FIG. 43 is an XRPD pattern of the Sodium salt of Compound A from the experiment L100110-68-25 after humidity exposure, that resulted in substantial improvement in crystallinity. along with peak positions (Form 5-D).
FIG. 44 is an XRPD pattern of the Potassium salt of Compound A from the experiment L100110-68-26 after drying along with peak positions (Form 6-A).
FIG. 45 is an XRPD pattern of the Potassium salt of Compound A from the experiment L100110-68-29 along with peak positions (Form 6-B).
FIG. 46 is an XRPD pattern of the Potassium salt of Compound A from the experiment L100110-68-30 after drying along with peak positions (Form 6-C).
FIG. 47 is an XRPD pattern of the Potassium salt of Compound A from the experiment L100110-68-30-H along with peak positions (Form 6-D).
FIG. 48 is an XRPD pattern of the Ethanolamine salt of Compound A from the experiment L100110-68-31 along with peak positions (Form 7-A).
FIG. 49 is an XRPD pattern of the Ethanolamine salt of Compound A from the experiment L100110-68-32 after drying along with peak positions (Form 7-B).
FIG. 50 is an XRPD pattern of the Diethanolamine salt of Compound A from the experiment L100110-68-36 (Form 8-A) along with peak positions.
FIG. 51 is an XRPD pattern of the Diethanolamine salt of Compound A from the experiment L100110-68-38 (Form 8-B) along with peak positions.
FIG. 52 is an XRPD pattern of the Diethanolamine salt of Compound A from the experiment L100110-68-36 after subjecting to saturated humidity environment at RT (Form 8-C) along with peak positions.
FIG. 53 is an XRPD pattern of the Diethanolamine salt of Compound A from the experiment L100110-68-40 before drying (Form 8-D) along with peak positions.
FIG. 54 is an XRPD pattern of the Diethanolamine salt of Compound A from the experiment L100110-68-40 after drying followed by exposure to saturated humidity environment (Form 8-E) (Essentially Form 8-B with extra peaks) along with peak positions.
FIG. 55 is an XRPD pattern of the Triethanolamine salt of Compound A from the experiment L100110-68-42 (Form 9-A) along with peak positions.
FIG. 56 is an XRPD pattern of the Triethanolamine salt of Compound A from the experiment L100110-68-44 (Form 9-B) along with peak positions.
FIG. 57 is an XRPD pattern of the Triethanolamine salt of Compound A from the experiment L100110-68-41 after drying and subjecting it to saturated humidity environment at room temperature (RT) (Form 9-C) along with peak positions.
FIG. 58 is an XRPD pattern of the Triethanolamine salt of Compound A from the experiment L100110-68-44 after drying, subjecting it to saturated humidity environment at RT (Form 9-D) along with peak positions.
FIG. 59 is an XRPD pattern of the Triethanolamine salt of Compound A from the experiment L100110-68-45 (Form 9-E) along with peak positions.
FIG. 60 is an XRPD pattern of the Diethylamine salt of Compound A from the scale-up experiment L100110-85-9 (Form 10-A) along with peak positions.
FIG. 61 is an XRPD pattern of the Diethylamine salt of Compound A from the experiment L100110-68-46 followed by drying (Form 10-C) along with peak positions.
FIG. 62 is an XRPD pattern of the Diethylamine salt of Compound A from the experiment L100110-68-49 (Form 10-B + extra minor peaks) along with their peak positions.
FIG. 63 is an XRPD pattern of the Ethanol-2-diethylamine salt of Compound A from the experiment L100110-68-56 (Form 12-A) along with their peak positions.
FIG. 64 is an XRPD pattern of the Ethanol-2-diethylamine salt of Compound A from the experiment L100110-68-60 (Form 12-B) along with their peak positions.
FIG. 65 is an XRPD pattern of the Ethanol-2-diethylamine salt of Compound A from the experiment L100110-68-60 after subjecting it to saturated humidity environment at RT (Form 12-C) along with their peak positions.
FIG. 66 is an XRPD pattern of the Choline hydroxide salt of Compound A from the experiment L100110-68-64 (Form 13-A) along with their peak positions.
FIG. 67 is an XRPD pattern of the L-Arginine salt of Compound A from the experiment L100110-68-66 (Form 14-A) along with their peak positions.
FIG. 68 is an XRPD pattern of the L-Arginine salt of Compound A from the experiment L100110-68-68 (Form 14-B) along with their peak positions,
FIG. 69 is an XRPD pattern of the L-Arginine salt of Compound A from the experiment L100110-68-69 (Form 14-C) along with their peak positions.
FIG. 70 is an XRPD pattern of the L-Arginine salt of Compound A from the experiment L100110-68-70 after drying (Form 14-E) along with their peak positions.
FIG. 71 is an XRPD pattern of the L-Histidine salt of Compound A from the experiment L100110-68-71 after drying (Form 15-A) along with their peak positions.
FIG. 72 is an XRPD pattern of the L-Histidine salt of Compound A from the experiment L100110-68-71 after drying, followed by subjecting it to saturated humidity environment at RT (Form 15-B) along with its peak positions.
FIG. 73 is an XRPD pattern of the L-Histidine salt of Compound A from the experiment L100110-68-72 after drying (Form 15-C) along with its peak positions.
FIG. 74 is an XRPD pattern of the L-Histidine salt of Compound A from the experiment L100110-68-75 before drying (Form 15-D) along with its peak positions.
FIG. 75 is an XRPD pattern of the L-Histidine salt of Compound A from the experiment L100110-68-75 (Form 15-E) along with their peak positions.
FIG. 76 is an XRPD pattern of the L-Lysine salt of Compound A from the experiment L100110-68-79 (Form 16-A) along with their peak positions.
FIG. 77 is an XRPD pattern of the Meglumine salt of Compound A from the experiment L100110-68-82 (Form 17-A) along with their peak positions.
FIG. 78 is an XRPD pattern of the Meglumine salt of Compound A from the experiment L100110-68-85 (Form 17-B) along with their peak positions.

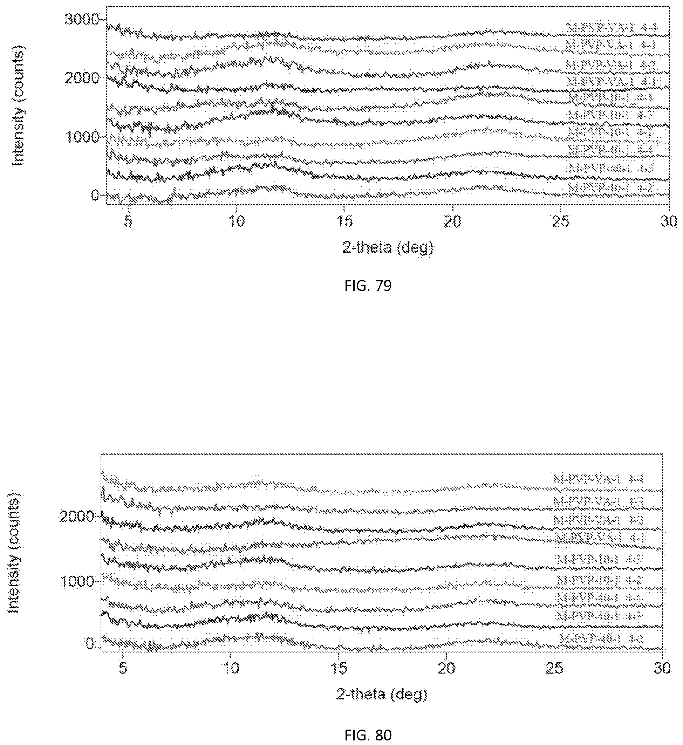

FIG. 79 is a graph showing a series of XRPD patterns of amorphous solids that were produced during the trials with 1:4 mixtures of Compound A with polymer.
FIG. 80 is a graph showing a series of XRPD patterns of amorphous solids after 1 month of storage that were produced during the trials with 1:4 mixtures of Compound A with polymer.

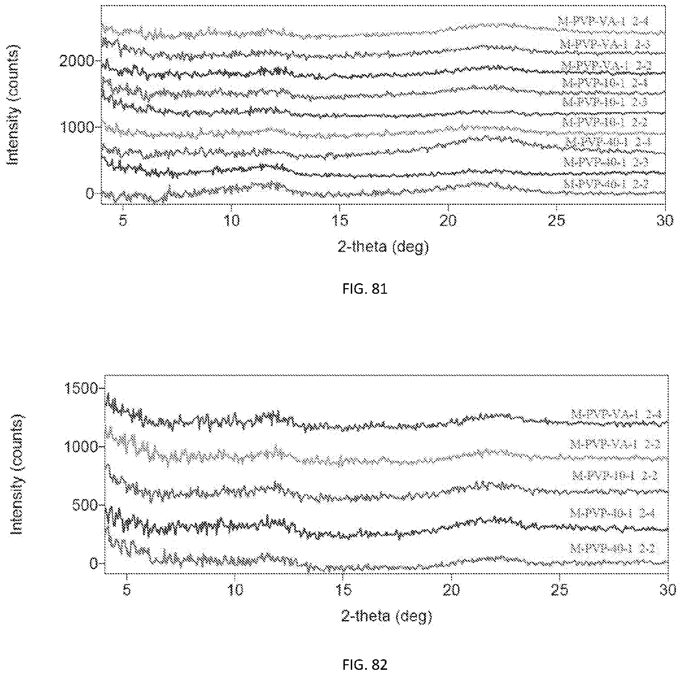

FIG. 81 is a graph showing a series of XRPD patterns of amorphous solids that were produced during the trials with 1:2 mixtures of Compound A with polymer.
FIG. 82 is a graph showing a series of XRPD patterns of amorphous solids after 1 month of storage that were produced during the trials with 1:2 mixtures of Compound A with polymer.
FIG. 83 is a dynamic vapor sorption (DVS) isotherm of the glutaric acid co-crystal of Compound A showing a total mass gain of 1.12% by weight between 2% and 95% relative humidity environments.
FIG. 84 is an XRPD pattern of the glutaric acid co-crystal after subjecting it to DVS analysis (L100129-24-Dry-Post_DVS), compared with the starting material, no change was observed.
FIG. 85 is an XRPD pattern of a mixture of Form B and Form M.
FIG. 86 is an XRPD pattern of a mixture of Form F and Form N.
FIG. 87 is an XRPD pattern of a mixture of Form A and Form O.
FIG. 88 is an XRPD pattern of a mixture of Form B and Form P.
FIG. 89 is an XRPD pattern of a mixture of Form C and Form Q.
FIG. 90 is an XRPD pattern of a mixture of Form F and Form R.

### Detailed Description of the Invention

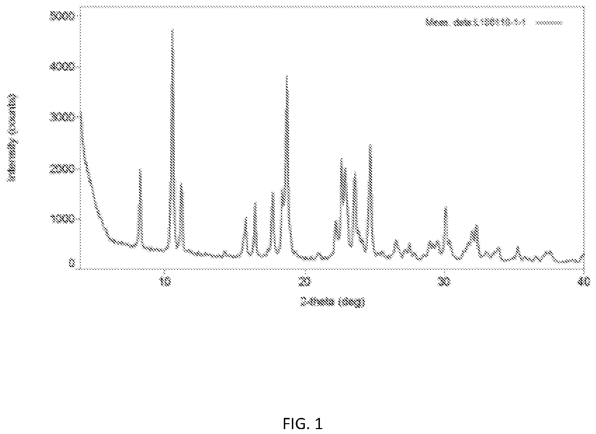

Provided herein are various morphic forms, co-crystals, salts, and amorphous solid dispersions of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (Compound A). The synthetic methods for Compound A can be found at U.S. Patent No. 7,452,882 and U.S. Patent No. 9,266,861, the contents of each of which are incorporated herein by reference. U.S. Patent No. 9,266,861 also discloses Form A (FIG. 1) of Compound A and methods of production thereof.

All the XRPD patterns described herein are based on a Cu Kα radiation wavelength (1.54 Å).

In one aspect, the present disclosure provides a morphic form of Compound A. In some embodiments, the morphic form is a solvate such as a methanol solvate, an ethanol solvate, an acetone solvate, a tetrahydrofuran solvate, an N-methylpyrrolidone solvate, a methyl isobutyl ketone solvate, an isopropyl acetate solvate, an acetic acid solvate, a dimethyl acetamide solvate, a dimethyl sulfoxide solvate, an isopropanol solvate, and an acetonitrile solvate.

In some embodiments, the morphic form is a desolvate such as an acetic acid desolvate, an acetonitrile desolvate, an ethyl acetate desolvate, an ethanol desolvate, and an acetone desolvate.

In some embodiments, the morphic form (Form B) of Compound A is characterized by an X-ray powder diffraction pattern including peaks at about 5.92, 11.8, and 17.5 degrees 2θ, wherein the X-ray powder diffraction pattern is obtained using a Cu Kα radiation source (1.54 Å). In some embodiments, the X-ray powder diffraction pattern of Form B can further include a peak at about 15.0 degrees 2θ. In some embodiments, Form B has an X-ray diffraction pattern substantially similar to that set forth in FIG. 2.

In some embodiments, the morphic form (Form C) of Compound A is characterized by an X-ray powder diffraction pattern including peaks at about 5.74, 11.5, 17.7, 19.3, 19.7, 21.4, 24.3 degrees 2θ, wherein the x-ray powder diffraction pattern is obtained using a Cu Kα radiation source (1.54 Å). In some embodiments, the X-ray powder diffraction pattern of Form C can further include one or more peaks from Table 5. In some embodiments, Form C has an X-ray diffraction pattern substantially similar to that set forth in FIG. 3.

In some embodiments, the morphic form (Form D) of Compound A is characterized by an X-ray powder diffraction pattern including peaks at about 5.52, 8.52, 11.0, 16.5, 18.3, 21.0, 21.2, and 24.0 degrees 2θ, wherein the x-ray powder diffraction pattern is obtained using a Cu Kα radiation source (1.54 Å). In some embodiments, the X-ray powder diffraction pattern of Form D can further include one or more peaks from Table 6. In some embodiments, Form D has an X-ray diffraction pattern substantially similar to that set forth in FIG. 4.

In some embodiments, the morphic form (Form E) of Compound A is characterized by an X-ray powder diffraction pattern including peaks at about 7.13, 10.8, 12.3, 14.1, 14.7, 15.5, 16.1, 17.5, 18.1, 19.9, 20.2, 21.0, 21.2, 22.7, 22.9, 24.4, 25.3, and 26.3 degrees 2θ, wherein the x-ray powder diffraction pattern is obtained using a Cu Kα radiation source (1.54 Å). In some embodiments, the X-ray powder diffraction pattern of Form E can further include one or more peaks from Table 7. In some embodiments, Form E has an X-ray diffraction pattern substantially similar to that set forth in FIG. 5.

In some embodiments, the morphic form (Form F) of Compound A is characterized by an X-ray powder diffraction pattern including peaks at about 10.1, 10.4, 11.4, 13.9, 16.2, 16.4, 17.1, 22.0, 23.8, and 29.5 degrees 2θ, wherein the x-ray powder diffraction pattern is obtained using a Cu Kα radiation source (1.54 Å). In some embodiments, the X-ray powder diffraction pattern of Form F can further include one or more peaks from Table 8. In some embodiments, Form F has an X-ray diffraction pattern substantially similar to that set forth in FIG. 6.

In some embodiments, the morphic form (Form G) of Compound A is characterized by an X-ray powder diffraction pattern including peaks at about 9.50, 12.9, 16.7, 17.3, 19.5, 20.2, 25.6, and 28.3 degrees 2θ, wherein the x-ray powder diffraction pattern is obtained using a Cu Kα radiation source (1.54 Å). In some embodiments, the X-ray powder diffraction pattern of Form G can further include one or more peaks from Table 9. In some embodiments, Form G has an X-ray diffraction pattern substantially similar to that set forth in FIG. 7.

In some embodiments, the morphic form (Form H) of Compound A is characterized by an X-ray powder diffraction pattern including peaks at about 9.22, 19.8, 23.6, 25.9, and 28.0 degrees 2θ, wherein the x-ray powder diffraction pattern is obtained using a Cu Kα radiation source (1.54 Å). In some embodiments, the X-ray powder diffraction pattern of Form H can further include one or more peaks from Table 10. In some embodiments, Form H has an X-ray diffraction pattern substantially similar to that set forth in FIG. 8.

In some embodiments, the morphic form (Form I) of Compound A is characterized by an X-ray powder diffraction pattern including peaks at about 5.77, 9.30, 10.2, 11.6, and 21.9 degrees 2θ, wherein the x-ray powder diffraction pattern is obtained using a Cu Kα radiation source (1.54 Å). In some embodiments, the X-ray powder diffraction pattern of Form I can further include one or more peaks from Table 11. In some embodiments, Form I has an X-ray diffraction pattern substantially similar to that set forth in FIG. 9.

In some embodiments, the morphic form (Form K) of Compound A is characterized by an X-ray powder diffraction pattern including peaks at about 8.42, 11.4, 14.5, 18.9, 21.1, and 21.6 degrees 2θ, wherein the x-ray powder diffraction pattern is obtained using a Cu Kα radiation source (1.54 Å). In some embodiments, the X-ray powder diffraction pattern of Form K can further include one or more peaks from Table 12. In some embodiments, Form K has an X-ray diffraction pattern substantially similar to that set forth in FIG. 10.

In some embodiments, the morphic form (Form L) of Compound A is characterized by an X-ray powder diffraction pattern including peaks at about 10.5, 11.5, 11.9, 15.2, 15.7, 16.0, 16.9, 17.1, 18.4, 18.7, 22.0, 22.8, 23.5, and 26.4 degrees 2θ, wherein the x-ray powder diffraction pattern is obtained using a Cu Kα radiation source (1.54 Å). In some embodiments, the X-ray powder diffraction pattern of Form L can further include one or more peaks from Table 13. In some embodiments, Form L has an X-ray diffraction pattern substantially similar to that set forth in FIG. 11.

In some embodiments, the morphic form (Form S+T) of Compound A is characterized by an X-ray powder diffraction pattern including peaks at about 7.42, 10.5, 11.3, 12.4, 14.3, 15.8, 16.8, 17.7, 18.1, 18.4, 20.1, 20.5, 21.1, 21.9, 23.2, 25.5, 26.9, and 28.3 degrees 2θ, wherein the x-ray powder diffraction pattern is obtained using a Cu Kα radiation source (1.54 Å). In some embodiments, the X-ray powder diffraction pattern of Form S+T can further include one or more peaks from Table 14. In some embodiments, Form S+T has an X-ray diffraction pattern substantially similar to that set forth in FIG. 12.

In some embodiments, the morphic form (Form S) of Compound A is characterized by an X-ray powder diffraction pattern including peaks at about 10.5, 12.3, 14.4, 15.8, 16.7, 17.7, 18.1, 18.4, 20.1, 20.6, 21.2, 21.9, 23.3, 24.4, 25.5, and 27.8 degrees 2θ, wherein the x-ray powder diffraction pattern is obtained using a Cu Kα radiation source (1.54 Å). In some embodiments, the X-ray powder diffraction pattern of Form S can further include one or more peaks from Table 15. In some embodiments, Form S has an X-ray diffraction pattern substantially similar to that set forth in FIG. 13.

In some embodiments, the morphic form (Form U) of Compound A is characterized by an X-ray powder diffraction pattern including peaks at about 5.79, 8.43, 11.4, 11.6, 14.5, 18.9, 21.1, and 21.6 degrees 2θ, wherein the x-ray powder diffraction pattern is obtained using a Cu Kα radiation source (1.54 Å). In some embodiments, the X-ray powder diffraction pattern of Form U can further include one or more peaks from Table 16. In some embodiments, Form U has an X-ray diffraction pattern substantially similar to that set forth in FIG. 14.

In some embodiments, the morphic form (Form V) of Compound A is characterized by an X-ray powder diffraction pattern including peaks at about 6.35, 10.6, 15.6, 16.5, 16.8, and 18.3 degrees 2θ, wherein the x-ray powder diffraction pattern is obtained using a Cu Kα radiation source (1.54 Å). In some embodiments, the X-ray powder diffraction pattern of Form V can further include one or more peaks from Table 17. In some embodiments, Form V has an X-ray diffraction pattern substantially similar to that set forth in FIG. 15.

In some embodiments, the morphic form (Form W) of Compound A is characterized by an X-ray powder diffraction pattern including peaks at about 10.1, 10.4, 10.7, 11.7, 13.9, 24.4, and 29.5 degrees 2θ, wherein the x-ray powder diffraction pattern is obtained using a Cu Kα radiation source (1.54 Å). In some embodiments, the X-ray powder diffraction pattern of Form W can further include one or more peaks from Table 18. In some embodiments, Form W has an X-ray diffraction pattern substantially similar to that set forth in FIG. 16.

In some embodiments, the morphic form (Form X) of Compound A is characterized by an X-ray powder diffraction pattern including peaks at about 9.66, 10.2, 10.5, 11.2, 18.7, and 24.7 degrees 2θ, wherein the x-ray powder diffraction pattern is obtained using a Cu Kα radiation source (1.54 Å). In some embodiments, the X-ray powder diffraction pattern of Form X can further include one or more peaks from Table 19. In some embodiments, Form X has an X-ray diffraction pattern substantially similar to that set forth in FIG. 17.

In some embodiments, the morphic form (Form Y) of Compound A is characterized by an X-ray powder diffraction pattern including peaks at about 6.51, 13.0, 13.3, 19.5, and 24.2 degrees 2θ, wherein the x-ray powder diffraction pattern is obtained using a Cu Kα radiation source (1.54 Å). In some embodiments, the X-ray powder diffraction pattern of Form Y can further include one or more peaks from Table 20. In some embodiments, Form Y has an X-ray diffraction pattern substantially similar to that set forth in FIG. 18.

In some embodiments, the morphic form (Form Z) of Compound A is characterized by an X-ray powder diffraction pattern including peaks at about 10.6, 11.2, 11.6, 12.0, 14.3, 15.6, 16.2, 17.6, 18.1, 18.7, 24.1, and 24.3 degrees 2θ, wherein the x-ray powder diffraction pattern is obtained using a Cu Kα radiation source (1.54 Å). In some embodiments, the X-ray powder diffraction pattern of Form Z can further include one or more peaks from Table 21. In some embodiments, Form Z has an X-ray diffraction pattern substantially similar to that set forth in FIG. 19.

In some embodiments, the morphic form (Form α) of Compound A is characterized by an X-ray powder diffraction pattern including peaks at about 7.26, 10.1, 10.4, 10.6, 11.9, 13.9, 16.5, 21.9, 22.4, and 24.1 degrees 2θ, wherein the x-ray powder diffraction pattern is obtained using a Cu Kα radiation source (1.54 Å). In some embodiments, the X-ray powder diffraction pattern of Form α can further include one or more peaks from Table 22. In some embodiments, Form α has an X-ray diffraction pattern substantially similar to that set forth in FIG. 20.

In some embodiments, the morphic form (Form β) of Compound A is characterized by an X-ray powder diffraction pattern including peaks at about 7.36, 10.5, 14.3, 15.7, 18.3, 20.4, 21.0, 21.8, and 23.2 degrees 2θ, wherein the x-ray powder diffraction pattern is obtained using a Cu Kα radiation source (1.54 Å). In some embodiments, the X-ray powder diffraction pattern of Form β can further include one or more peaks from Table 23. In some embodiments, Form β has an X-ray diffraction pattern substantially similar to that set forth in FIG. 21.

In some embodiments, the morphic form (Form χ) of Compound A is characterized by an X-ray powder diffraction pattern including peaks at about 8.53, 11.2, 18.4, 20.1, and 21.4 degrees 2θ, wherein the x-ray powder diffraction pattern is obtained using a Cu Kα radiation source (1.54 Å). In some embodiments, the X-ray powder diffraction pattern of Form χ can further include one or more peaks from Table 24. In some embodiments, Form χ has an X-ray diffraction pattern substantially similar to that set forth in FIG. 22.

In some embodiments, the morphic form (Form δ) of Compound A is characterized by an X-ray powder diffraction pattern including peaks at about 10.9, 12.1, 14.4, 18.1, 19.6, 24.5, and 27.0 degrees 2θ, wherein the x-ray powder diffraction pattern is obtained using a Cu Kα radiation source (1.54 Å). In some embodiments, the X-ray powder diffraction pattern of Form δ can further include one or more peaks from Table 25. In some embodiments, Form δ has an X-ray diffraction pattern substantially similar to that set forth in FIG. 23.

In some embodiments, the morphic form (Form ε) of Compound A is characterized by an X-ray powder diffraction pattern including peaks at about 5.73, 11.4, 16.6, 17.6, 23.2, and 24.2 degrees 2θ, wherein the x-ray powder diffraction pattern is obtained using a Cu Kα radiation source (1.54 Å). In some embodiments, the X-ray powder diffraction pattern of Form ε can further include one or more peaks from Table 26. In some embodiments, Form ε has an X-ray diffraction pattern substantially similar to that set forth in FIG. 24.

In some embodiments, the morphic form (Form ϕ) of Compound A is characterized by an X-ray powder diffraction pattern including peaks at about 6.95, 13.9, 20.9, 22.3, and 27.9 degrees 2θ, wherein the x-ray powder diffraction pattern is obtained using a Cu Kα radiation source (1.54 Å). In some embodiments, the X-ray powder diffraction pattern of Form ϕ can further include one or more peaks from Table 27. In some embodiments, Form ϕ has an X-ray diffraction pattern substantially similar to that set forth in FIG. 25.

In some embodiments, the morphic form (Form η) of Compound A is characterized by an X-ray powder diffraction pattern including peaks at about 6.87, 7.69, 20.5, 23.0, 23.9, and 28.2 degrees 2θ, wherein the x-ray powder diffraction pattern is obtained using a Cu Kα radiation source (1.54 Å). In some embodiments, the X-ray powder diffraction pattern of Form η can further include one or more peaks from Table 28. In some embodiments, Form η has an X-ray diffraction pattern substantially similar to that set forth in FIG. 26.

In some embodiments, the morphic form (Form λ) of Compound A is characterized by an X-ray powder diffraction pattern including peaks at about 10.6, 12.0, 14.3, 16.2, 17.6, 18.0, and 24.3 degrees 2θ, wherein the x-ray powder diffraction pattern is obtained using a Cu Kα radiation source (1.54 Å). In some embodiments, the X-ray powder diffraction pattern of Form λ can further include one or more peaks from Table 29. In some embodiments, Form λ has an X-ray diffraction pattern substantially similar to that set forth in FIG. 27.

In some embodiments, the morphic form has purity of greater than 85% by weight, e.g., greater than 86% by weight, greater than 90% by weight, greater than 92.5% by weight, greater than 95% by weight, greater than 96% by weight, greater than 97% by weight, greater than 97.5% by weight, greater than 98% by weight, greater than 98.5% by weight, greater than 99% by weight, greater than 99.2% by weight, greater than 99.5% by weight, or greater than 99.8% by weight. For example, the content of impurities (i.e., any components of the composition other than Compound A, such as byproducts, starting material, solvent residues, heavy metal, etc.) is less than 15% by weight, less than 14% by weight, less than 10% by weight, less than 8% by weight, less than 5% by weight, less than 4% by weight, less than 3% by weight, less than 2% by weight, less than 1.5% by weight, less than 1% by weight, less than 0.8% by weight, less than 0.5% by weight, or less than 0.2% by weight.

In another aspect, the present disclosure provides an amorphous solid dispersion of Compound A. As used herein, the term "solid dispersion" refers to a system in a solid state comprising at least two components, wherein one component is dispersed throughout the other component or components. The term "amorphous solid dispersion" as used herein, refers to a stable solid dispersion comprising an amorphous drug substance and a stabilizing polymer. Non-limiting examples of the stabilizing polymer are polyvinylpyrrolidone MW 10,000 (PVP-10), polyvinylpyrrolidone MW 40,000 (PVP-40), and poly(1-vinylpyrrolidone-co-vinyl acetate) (PVP-Co-VA), hydroxy-propyl methyl cellulose (Hypromellose), methylcellulose, hydroxy propyl cellulose, and poly ethylene glycol (PEG) 6000. In some embodiments, the stabilizing polymer is polyvinylpyrrolidone.

The amorphous solid dispersion of the present disclosure includes Compound A and a stabilizing polymer. The amount of Compound A in the amorphous solid dispersions of the present disclosure can range from about 0.1% to about 60% by weight relative to the stabilizing polymer. In some embodiments, the amount of Compound A in the amorphous solid dispersions of the present disclosure ranges from about 15% to about 50% by weight relative to the stabilizing polymer. In some embodiments, the amount of Compound A in the amorphous solid dispersions of the present disclosure can be about 50% by weight relative to the stabilizing polymer. In some embodiments, the amount of Compound A in the amorphous solid dispersions of the present disclosure can be about 25% by weight relative to the stabilizing polymer.

In another aspect, the present disclosure provides a co-crystal of Compound A. Co-crystal screening was performed on Compound A with 20 different potential co-formers. The co-formers tested include adipic acid, L-arginine, ascorbic acid, benzoic acid, citric acid, D (+) glucose, glutaric acid, L-histidine, 4-hydroxy benzoic acid, 3,4-dihydroxy benzoic acid, L-lysine, malic acid, salicyclic acid, succinic acid, tartaric acid, urea, vanillin, and vanillic acid. So far, only a Compound A/glutaric acid co-crystal has been observed.

In some embodiments, the co-crystal can be characterized by an XRPD pattern including peaks at about 9.74, 10.8, 11.0, 12.2, 16.1, 17.0, 19.2, 21.9, and 23.1 degrees 2θ. In some embodiments, the X-ray powder diffraction pattern of the co-crystal can further include one or more peaks from Table 30. In some embodiments, the co-crystal has an XRPD pattern substantially similar to that set forth in FIG. 28. In some embodiments, the co-crystal has purity of greater than 85% by weight, e.g., greater than 86% by weight, greater than 90% by weight, greater than 92.5% by weight, greater than 95% by weight, greater than 96% by weight, greater than 97% by weight, greater than 97.5% by weight, greater than 98% by weight, greater than 98.5% by weight, greater than 99% by weight, greater than 99.2% by weight, greater than 99.5% by weight, or greater than 99.8% by weight. For example, the content of impurities (i.e., any components of the composition other than Compound A, such as byproducts, starting material, solvent residues, heavy metal, etc.) is less than 15% by weight, less than 14% by weight, less than 10% by weight, less than 8% by weight, less than 5% by weight, less than 4% by weight, less than 3% by weight, less than 2% by weight, less than 1.5% by weight, less than 1% by weight, less than 0.8% by weight, less than 0.5% by weight, or less than 0.2% by weight.

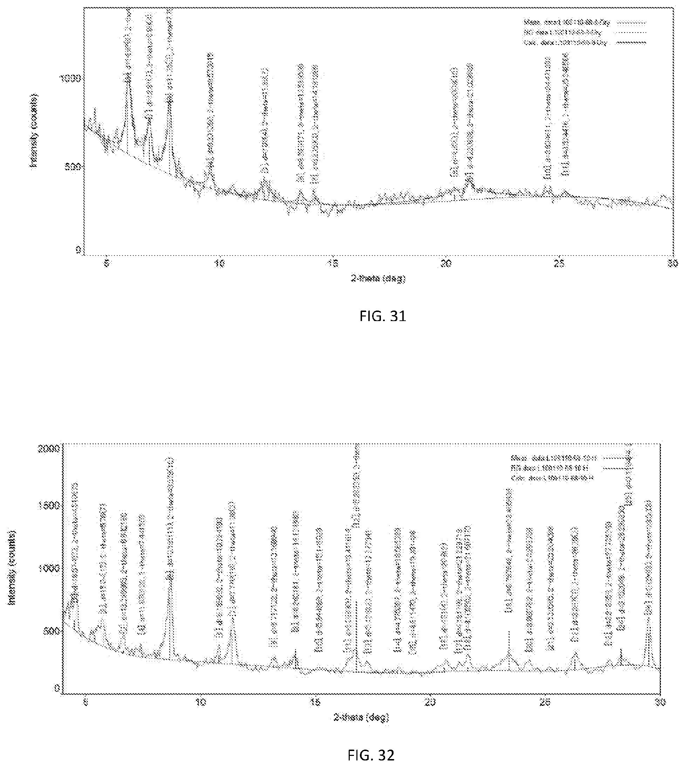

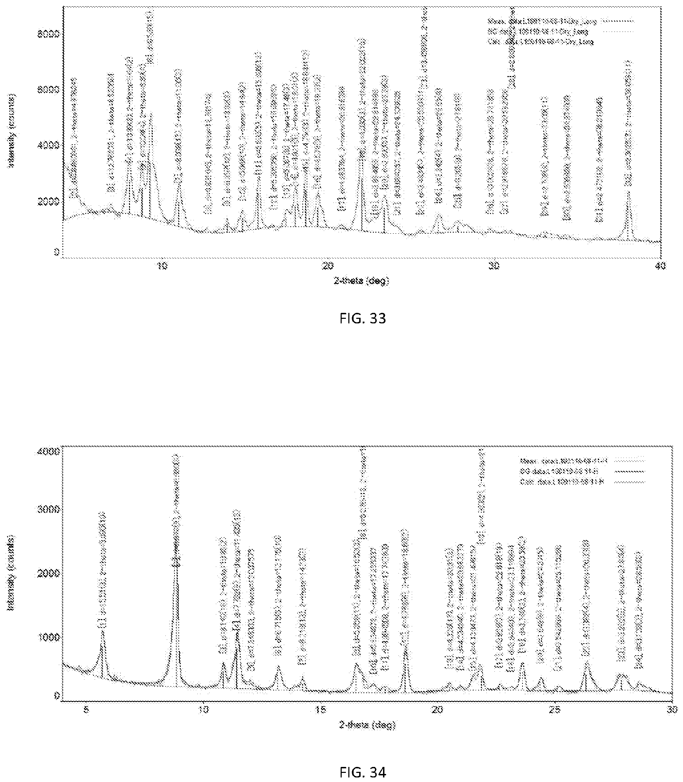

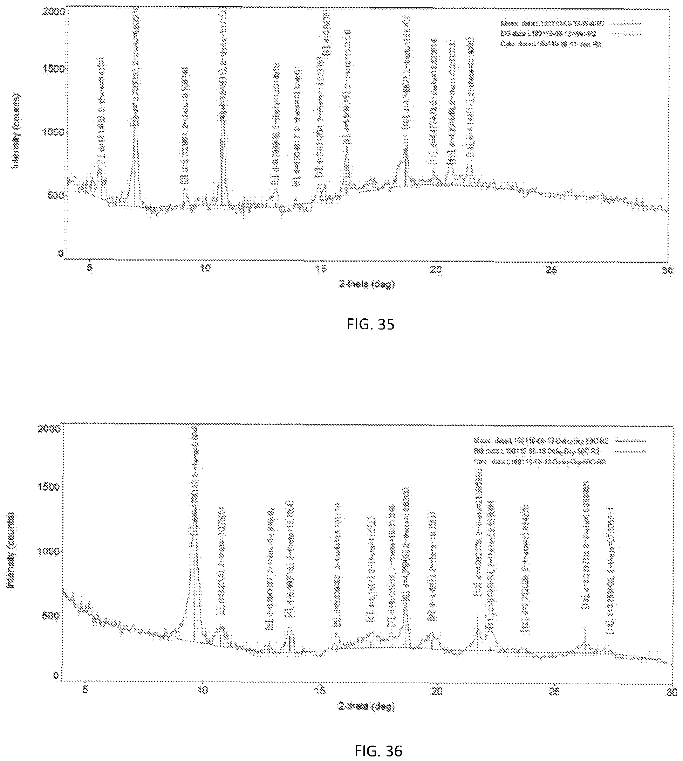

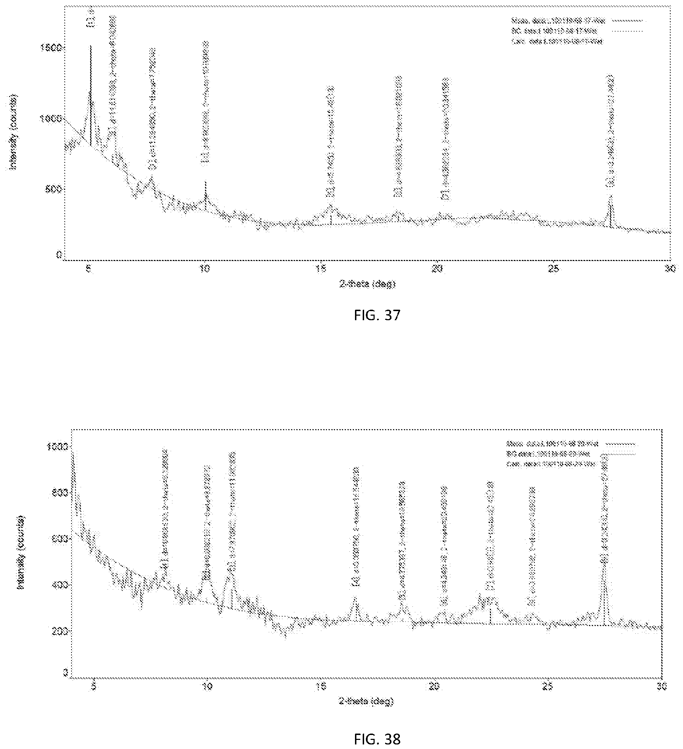

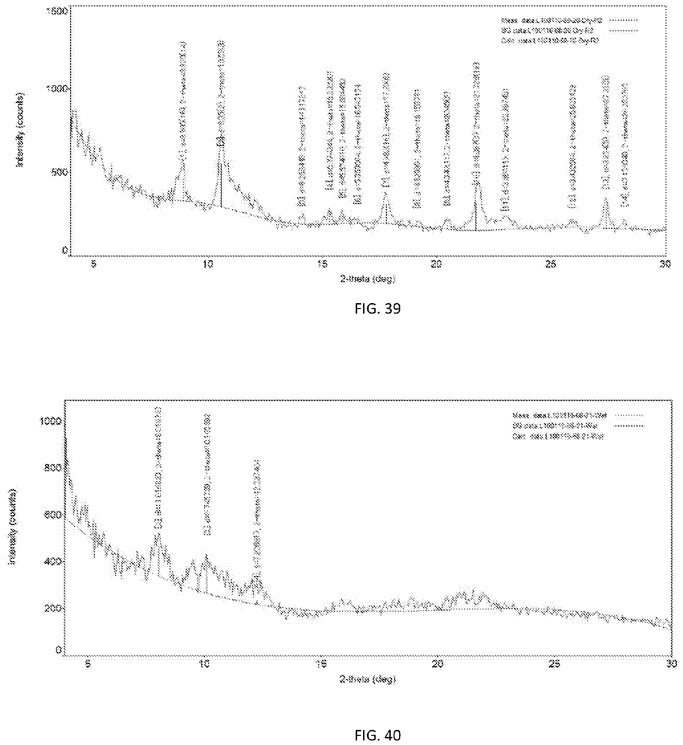

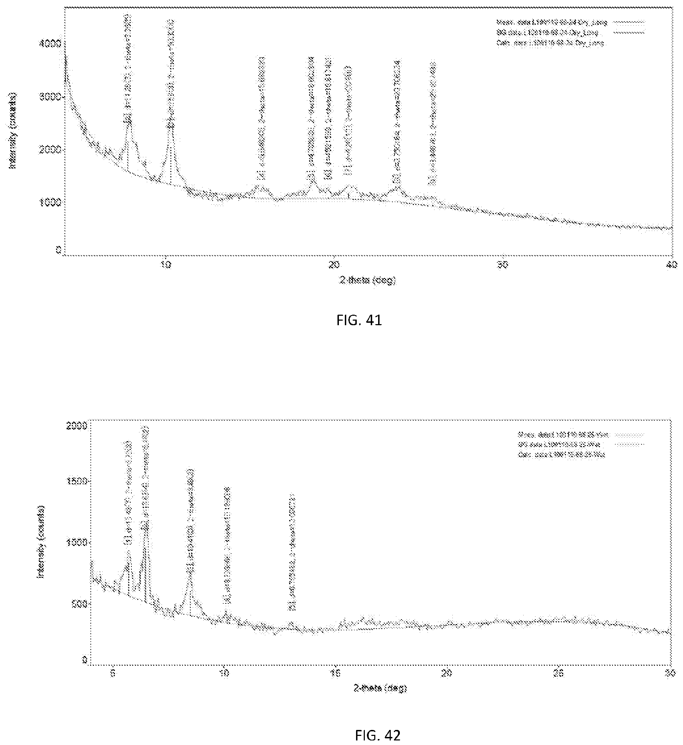

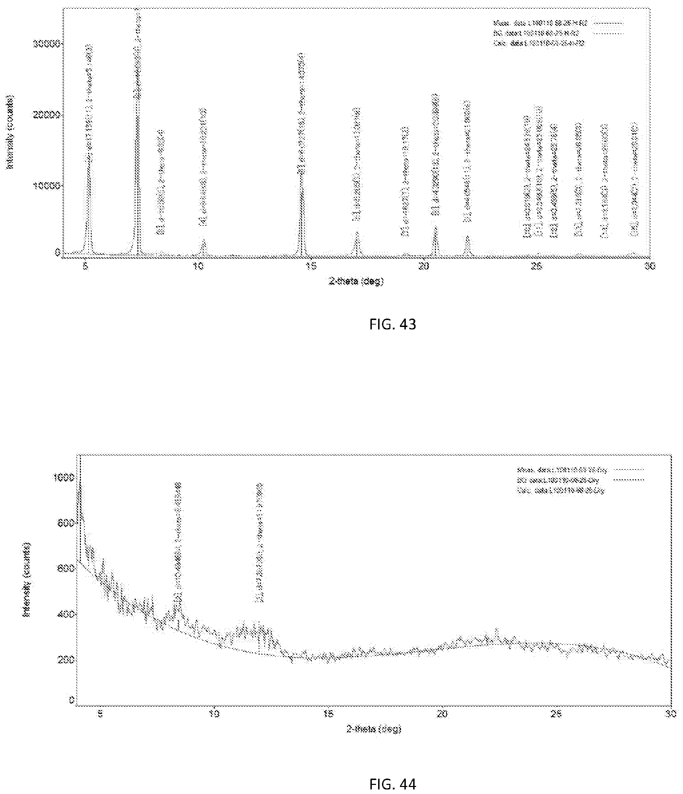

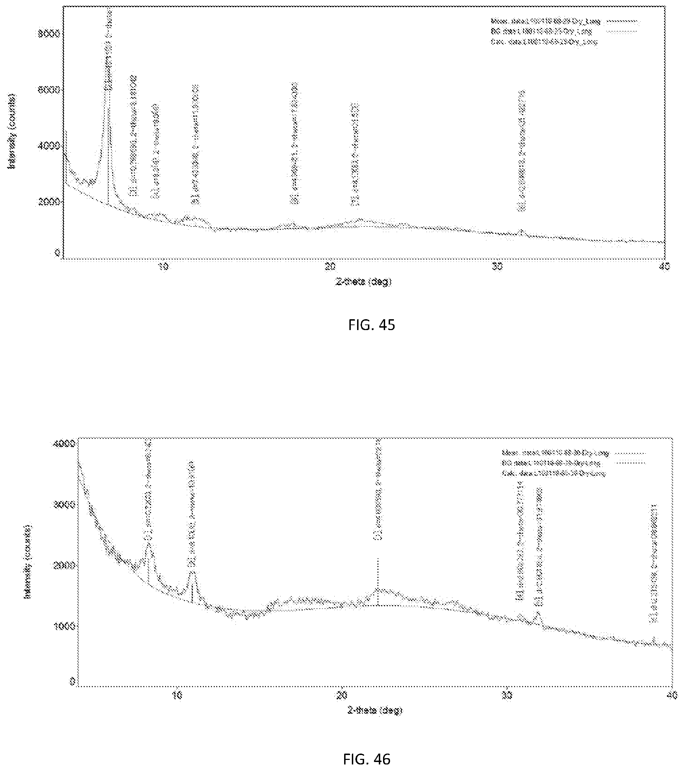

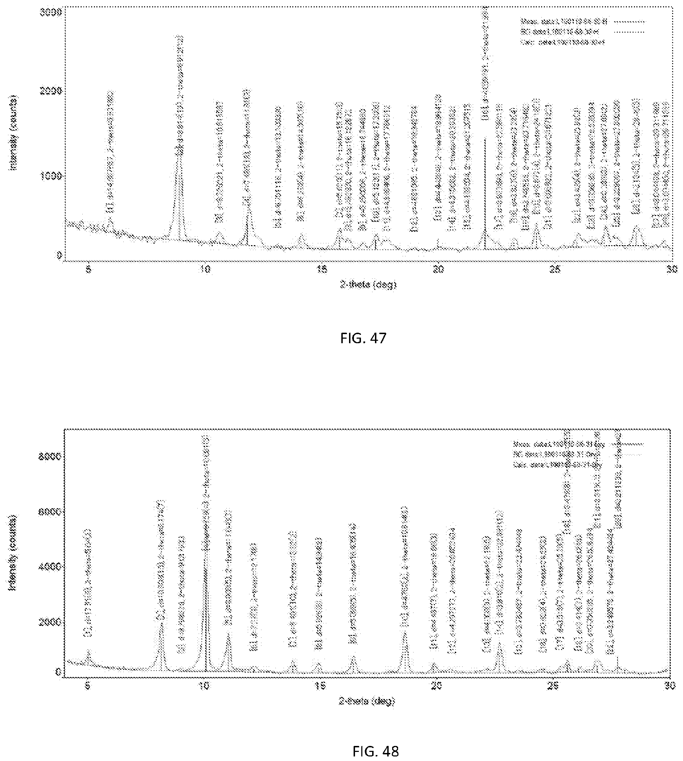

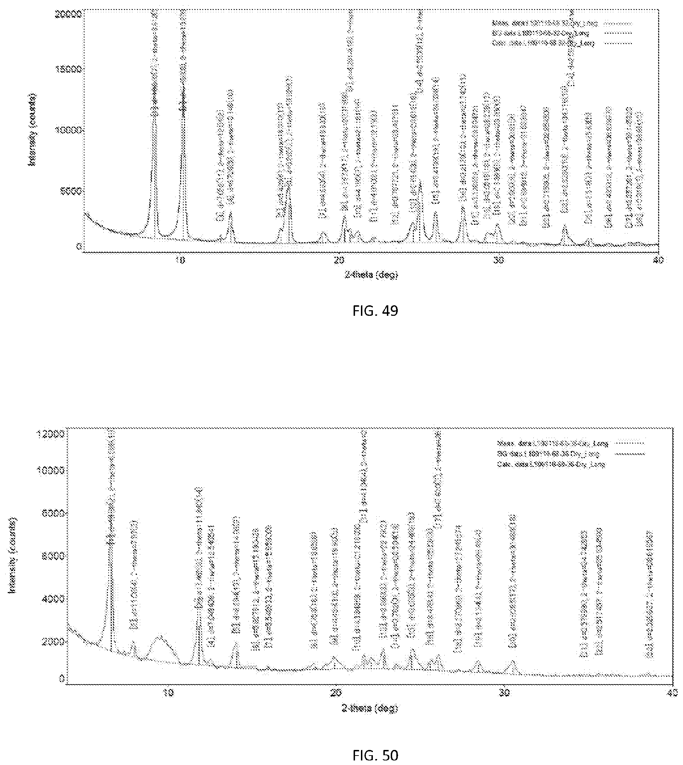

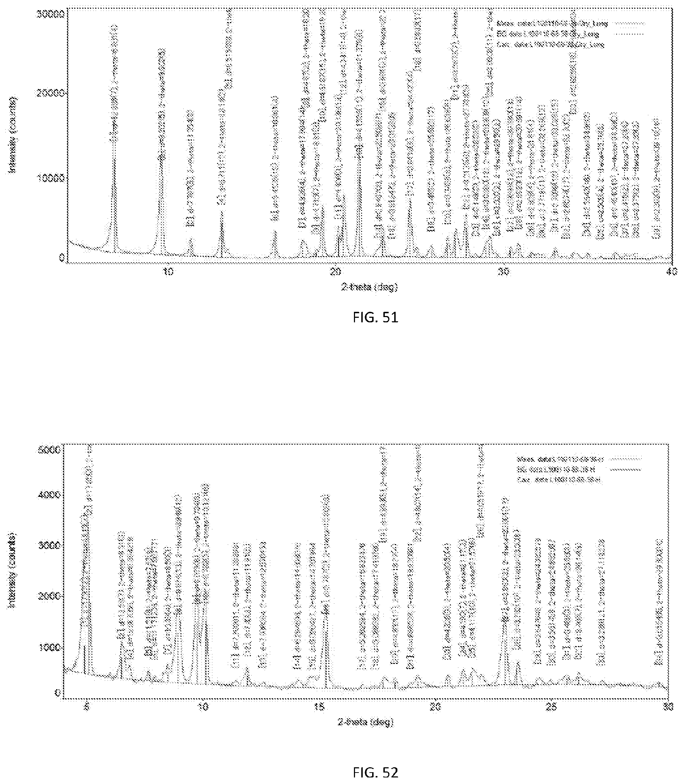

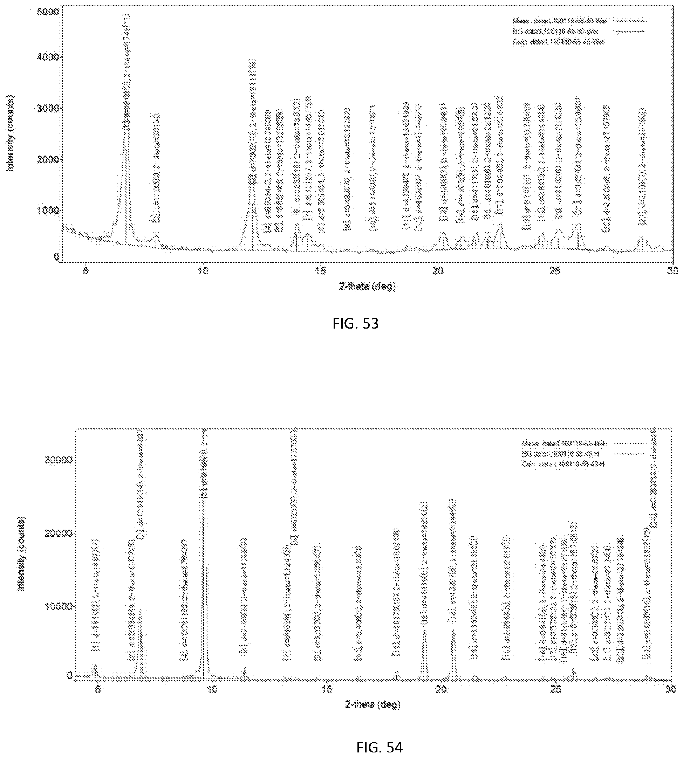

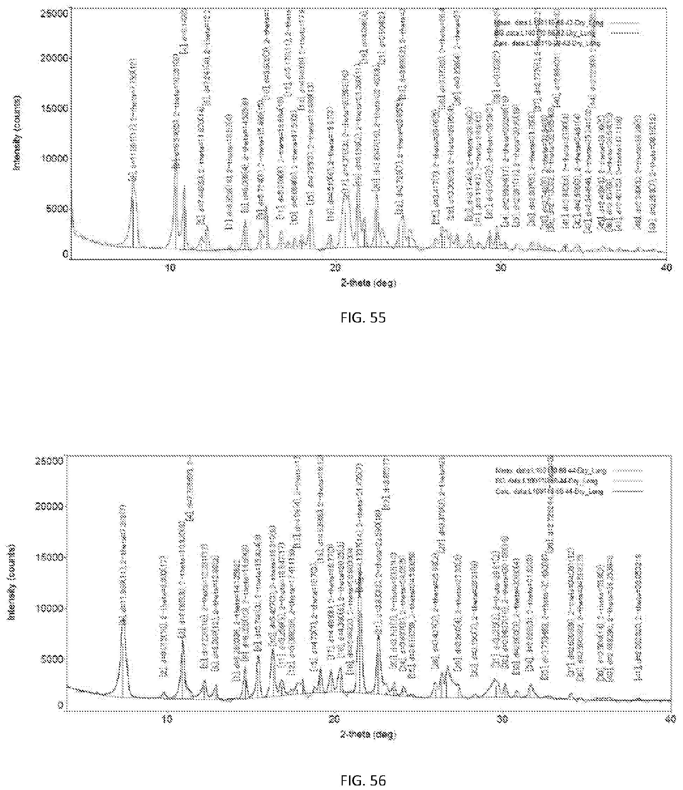

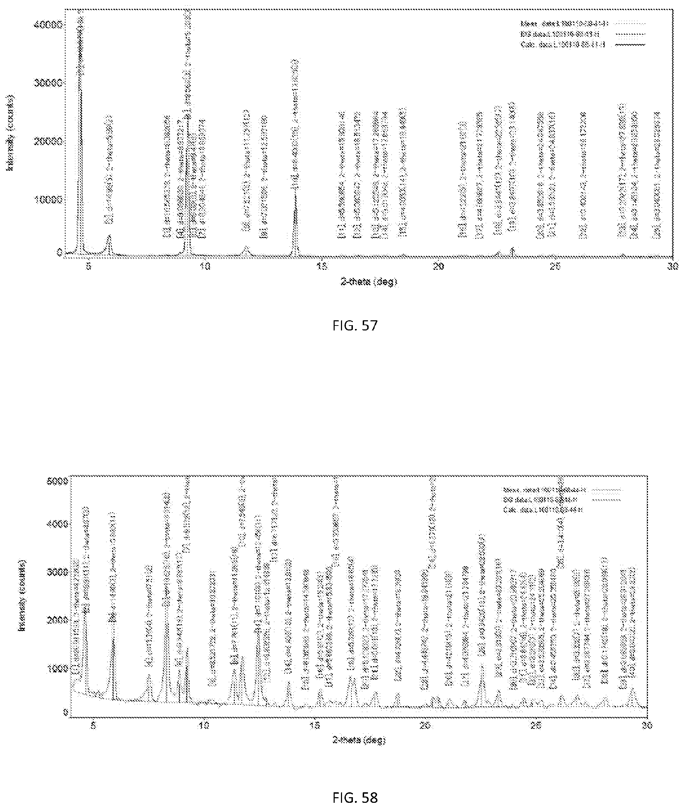

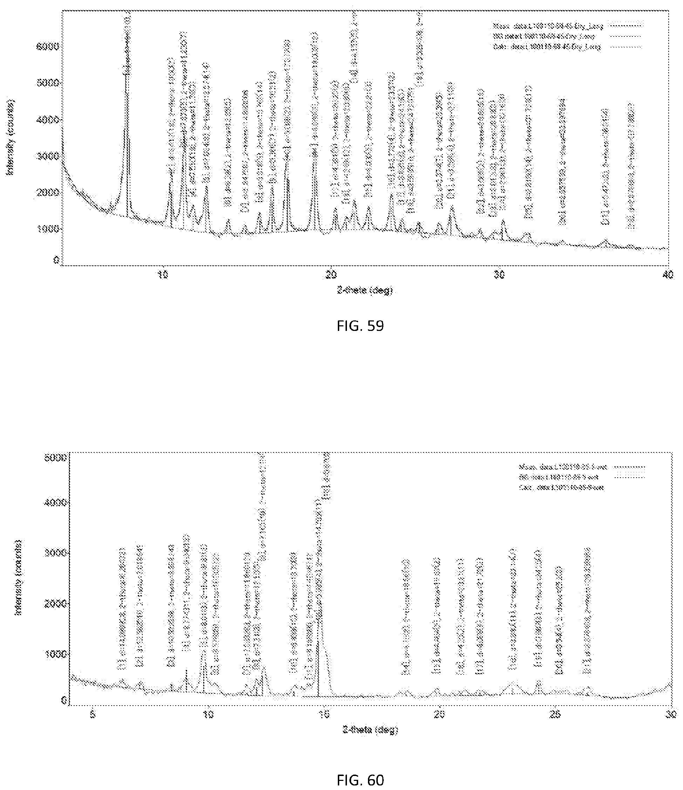

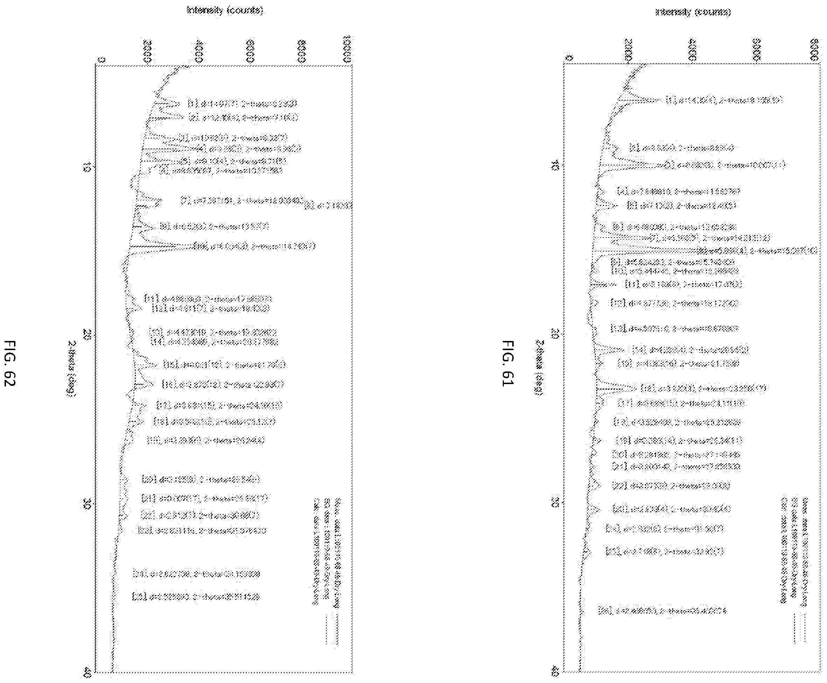

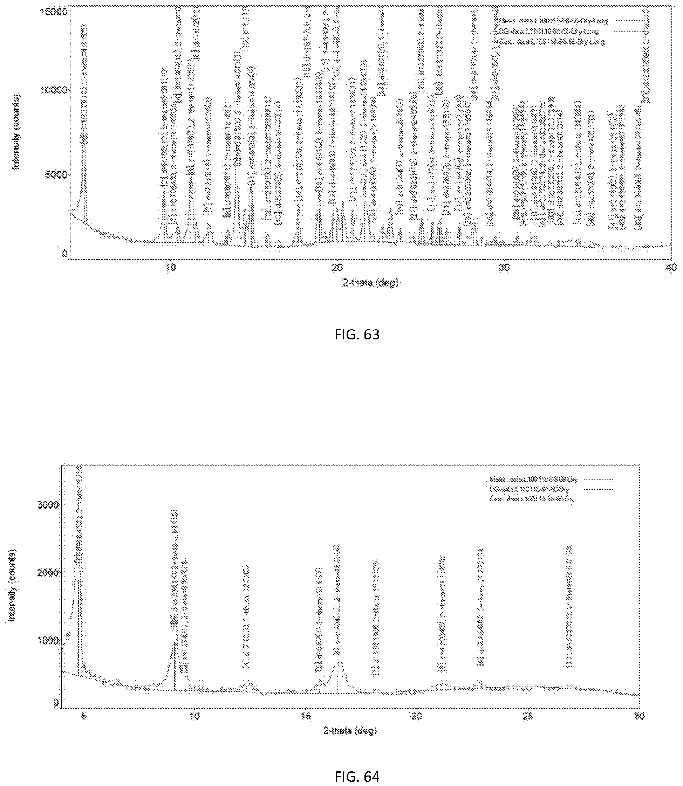

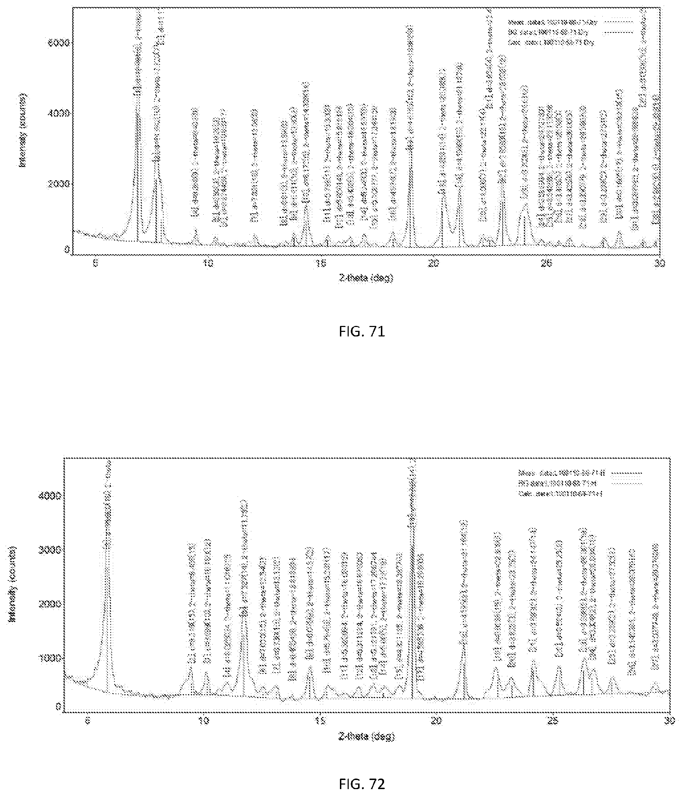

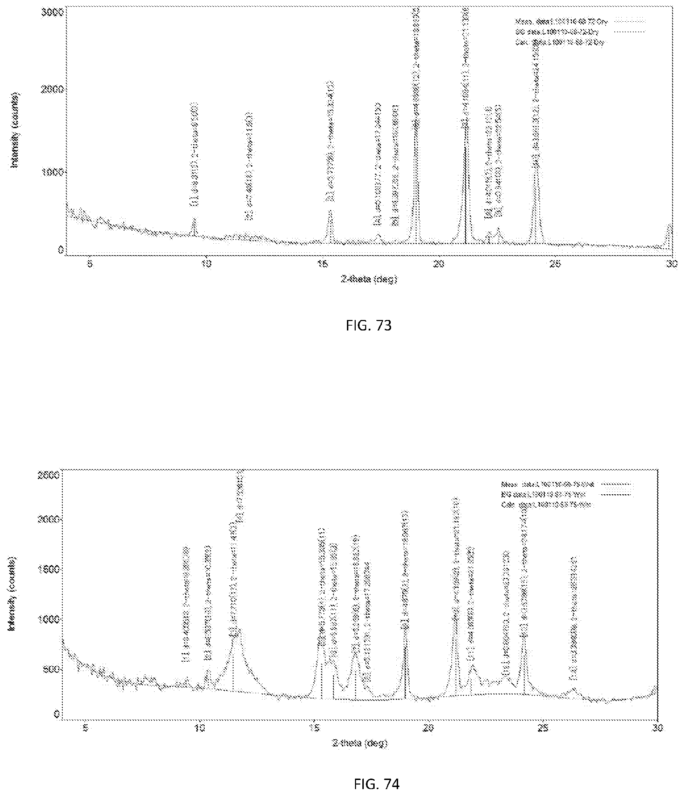

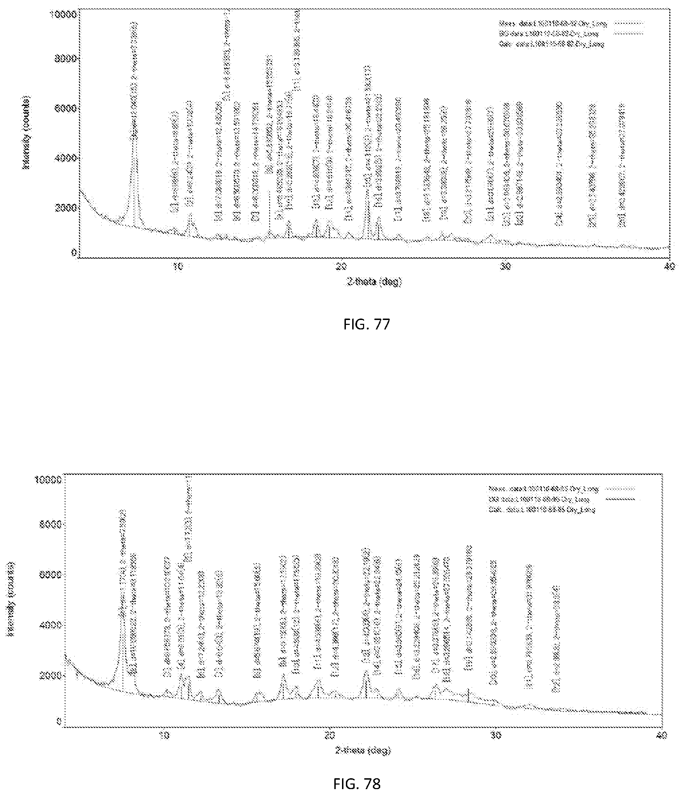

In yet another aspect, the present disclosure provides a crystalline salt of Compound A. The crystalline salt comprises Compound A and one or more counter-ions. The molar ratio of Compound A over the counter-ion can be 1:1 to 2:1. In some embodiments, the molar ratio of Compound A over the counter-ion is 1:1.1. In some embodiments, the molar ratio of Compound A over the counter-ion is 1:1. In some embodiments, the counter-ion is L-lysine, L-arginine, 2-hydroxy-N,N,N-trimethylethan-1-aminium, diethylamine, ethanolamine, ethanol-2-diethylamine, Na⁺, Mg²⁺, K⁺, Ca²⁺, diethanolamine, triethanolamine, L-histidine, meglumine, or a combination thereof. In some embodiments, the crystalline salt has an XRPD pattern substantially similar to that set forth in any one of FIGs. 29-78.

In some embodiments, the counter-ion is L-lysine. In some embodiments, when the counter-ion is L-lysine, the crystalline salt can be characterized by an XRPD pattern including peaks at about 8.70, 9.22, 11.3, 17.0, and 24.8 degrees 2θ. The XRPD pattern can further include peaks at about 7.12, 18.4, 19.1, 20.4, and 25.7 degrees 2θ. In some embodiments, the L-lysine salt has an XRPD pattern substantially similar to that set forth in FIG. 76. The L-lysine salt can have a melting point of about 250 °C.

In some embodiments, the counter-ion is L-arginine. The L-arginine salt can have an XRPD pattern substantially similar to that set forth in any one of FIGs. 67-70. The L-arginine salt can have a melting point of about 200 °C.

In some embodiments, the counter-ion is 2-hydroxy-N,N,N-trimethylethan-1-aminium. In some embodiments, the 2-hydroxy-N,N,N-trimethylethan-1-aminium salt can have an XRPD pattern substantially similar to that set forth in FIG. 66. The 2-hydroxy-N,N,N-trimethylethan-1-aminium salt can have a melting point of about 229 °C.

In yet another aspect, the present disclosure includes a salt of Compound A in the form of a solvate (referred to herein as "a salt solvate"). The salt solvate can contain one or more counter-ions. In some embodiments, the counter ion can be potassium, sodium, or magnesium. In some embodiments, the salt solvate is a salt of Compound A (e.g., potassium salt, sodium salt, or magnesium salt) in the form of an acetic acid solvate. In some embodiments, the salt solvate is a salt of Compound A in the form of a tetrahydrofuran solvate. In some embodiments, the salt solvate is a salt of Compound A that includes a solvent and water. Such morphic forms may include a solvent and water in a single chemical entity with Compound A and the counter-ion, or may comprise a physical mixture of Compound A as the salt in a hydrate and a solvate.

The potassium salt solvate of Compound A is useful for the removal of impurities, which may be removed during the isolation of the salt solvate.

In another aspect of the invention, a morphic form of one type may be converted to another. A morphic form comprising a solvate or hydrate may be converted to a form having a counter-ion.

In some embodiments, the crystalline salt has purity of greater than 85% by weight, e.g., greater than 86% by weight, greater than 90% by weight, greater than 92.5% by weight, greater than 95% by weight, greater than 96% by weight, greater than 97% by weight, greater than 97.5% by weight, greater than 98% by weight, greater than 98.5% by weight, greater than 99% by weight, greater than 99.2% by weight, greater than 99.5% by weight, or greater than 99.8% by weight. For example, the content of impurities (i.e., any components of the composition other than Compound A, such as byproducts, starting material, solvent residues, heavy metal, counter-ions, etc.) is less than 15% by weight, less than 14% by weight, less than 10% by weight, less than 8% by weight, less than 5% by weight, less than 4% by weight, less than 3% by weight, less than 2% by weight, less than 1.5% by weight, less than 1% by weight, less than 0.8% by weight, less than 0.5% by weight, or less than 0.2% by weight.

The present disclosure also provides a mixture of two or more of the forms disclosed herein. The forms can be present at any weight ratio in the mixture. For example, two or more of the morphic forms disclosed herein can be present in a mixture.

The present disclosure also provides a pharmaceutical composition comprising any one of the morphic forms, co-crystals, salts, and amorphous solid dispersions of Compound A as disclosed herein. The pharmaceutical composition can further include at least one pharmaceutically acceptable excipient or carrier.

A "pharmaceutical composition" is a formulation containing a compound of the present invention in a form suitable for administration to a subject. In some embodiments, the pharmaceutical composition is in bulk or in unit dosage form. The unit dosage form can be in any of a variety of forms, including, for example, a capsule, an IV bag, a tablet, a single pump on an aerosol inhaler or a vial. The quantity of active ingredient (e.g., a formulation of the disclosed morphic forms, co-crystals, salts, and amorphous solid dispersions) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for topical or transdermal administration include powders, sprays, ointments, pastes, creams, lotions, gels, solutions, patches and inhalants. In some embodiments, Compound A is mixed under sterile conditions with a pharmaceutically acceptable carrier, and with any preservatives, buffers or propellants that are required.

A pharmaceutical composition of the invention is formulated to be compatible with its intended route of administration. Examples of routes of administration include parenteral, *e.g.,* intravenous, intradermal, subcutaneous, oral (*e.g.,* inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

In the practice of the method of the present invention, an effective amount of any one of the morphic forms, co-crystals, salts, and amorphous solid dispersions of this invention is administered via any of the usual and acceptable methods known in the art, either singly or in combination. The compositions can thus be administered orally (e.g., buccal cavity), sublingually, parenterally (e.g., intramuscularly, intravenously, or subcutaneously), rectally (e.g., by suppositories or washings), transdermally (e.g., skin electroporation), or by inhalation (e.g., by aerosol), and in the form or solid, liquid or gaseous dosages, including tablets and suspensions. The administration can be conducted in a single unit dosage form with continuous therapy or in a single dose therapy ad libitum. The therapeutic composition can also be in the form of an oil emulsion or dispersion in conjunction with a lipophilic salt such as pamoic acid, or in the form of a biodegradable sustained-release composition for subcutaneous or intramuscular administration.

Useful pharmaceutical carriers for the preparation of the compositions hereof, can be solids, liquids or gases; thus, the compositions can take the form of tablets, pills, capsules, suppositories, powders, enterically coated or other protected formulations (e.g. binding on ionexchange resins or packaging in lipid-protein vesicles), sustained release formulations, solutions, suspensions, elixirs, aerosols, and the like. The carrier can be selected from the various oils including those of petroleum, animal, vegetable or synthetic origin, e.g., peanut oil, soybean oil, mineral oil, sesame oil, and the like. Water, saline, aqueous dextrose, and glycols are preferred liquid carriers, particularly (when isotonic with the blood) for injectable solutions. For example, formulations for intravenous administration comprise sterile aqueous solutions of the active ingredient(s) which are prepared by dissolving solid active ingredient(s) in water to produce an aqueous solution, and rendering the solution sterile. Suitable pharmaceutical excipients include starch, cellulose, talc, glucose, lactose, talc, gelatin, malt, rice, flour, chalk, silica, magnesium stearate, sodium stearate, glycerol monostearate, sodium chloride, dried skim milk, glycerol, propylene glycol, water, ethanol, and the like. The compositions may be subjected to conventional pharmaceutical additives such as preservatives, stabilizing agents, wetting or emulsifying agents, salts for adjusting osmotic pressure, buffers and the like. Suitable pharmaceutical carriers and their formulation are described in Remington's Pharmaceutical Sciences by E. W. Martin. Such compositions will, in any event, contain an effective amount of the active compound together with a suitable carrier so as to prepare the proper dosage form for proper administration to the recipient.

The pharmaceutical preparations can also contain preserving agents, solubilizing agents, stabilizing agents, wetting agents, emulsifying agents, sweetening agents, coloring agents, flavoring agents, salts for varying the osmotic pressure, buffers, coating agents or antioxidants. They can also contain other therapeutically valuable substances, including additional active ingredients other than those of Compound A.

The compositions disclosed herein are useful as medicaments for the treatment of a resistance to thyroid hormone (RTH) syndrome in a subject who has at least one TRβ mutation. Accordingly, the present disclosure provides the compositions disclosed herein for use in treating a RTH syndrome in a subject having at least one TRβ mutation. The present disclosure also provides a method for treating a RTH syndrome in a subject having at least one TRβ mutation, the method comprising administering to the subject a therapeutically effective amount of any one of the morphic forms, co-crystals, salts, and amorphous solid dispersions disclosed herein. The present disclosure also provides a crystalline salt, morphic form, co-crystal, or amorphous solid dispersion disclosed herein for the manufacture of a medicament for treating a RTH syndrome in a subject having at least one TRβ mutation in a subject in need thereof, wherein the crystalline salt, morphic form, co-crystal, or amorphous solid dispersion disclosed herein is for administration to the subject in at least one therapeutically effective amount. The present disclosure also provides a crystalline salt, morphic form, co-crystal, or amorphous solid dispersion disclosed herein for use in treating a RTH syndrome in a subject having at least one TRβ mutation in a subject in need thereof, wherein the crystalline salt, morphic form, co-crystal, or amorphous solid dispersion disclosed herein is for administration to the subject in at least one therapeutically effective amount.

The subject may exhibit one or more symptoms of the RTH syndrome, such as obesity, hyperlipidemia, hypercholesterolemia, heterozygous familial hypercholesterolemia, diabetes, non-alcoholic steatohepatitis, fatty liver, fatty liver disease, bone disease, dyslipidemia, thyroid axis alteration, atherosclerosis, a cardiovascular disorder, tachycardia, hyperkinetic behavior, hypothyroidism, goiter, attention deficit hyperactivity disorder, learning disabilities, mental retardation, hearing loss, delayed bone age, neurologic or psychiatric disease or thyroid cancer. Details about the RTH syndrome can be found at Weiss and Refetoff, "Resistance to Thyroid Hormone (RTH) in the Absence of Abnormal Thyroid Hormone Receptor (TR) (nonTR-RTH)," Hot Thyroidology 09/09, 11 pages in total, the contents of which are incorporated by reference in their entireties.

Thyroid hormone receptor nucleic acids and polypeptides from various species (e.g., human, rat, chicken, etc.) have previously been described. *See, e.g.,* R. L. Wagner et al. (2001), Molecular Endocrinology 15(3): 398-410; J. Sap et al. (1986), Nature 324:635-640; C. Weinberger et al. (1986), Nature 324:641-646; and C.C.Tompson et al. (1986), Science 237:1610-1614; each of which is incorporated herein by reference in its entirety. The amino acid sequence of human TRβ is provided, *e.g*., by Genbank Accession No. P10828.2, incorporated herein by reference.

| |
|---|
| Amino acid sequence of the ligand binding domain (residues 203-461) of human TRβ (SEQ ID NO: 1) |
| ELQKSIGHKPEPTDEEWELIKTVTEAHVATNAQGSHWKQKRKFLPEDIGQAPIVNAPEGGKVDLEAFSHFTKIIT PAITRVVDFAKKLPMFCELPCEDQIILLKGCCMEIMSLRAAVRYDPESETLTLNGEMAVTRGQLKNGGLGVVSDA IFDLGMSLSSFNLDDTEVALLQAVLLMSSDRPGLACVERIEKYQDSFLLAFEHYINYRKHHVTHFWPKLLMKVTD LRMIGACHASRFLHMKVECPTELFPPLFLEVFED |

The residues at the 234, 243, 316, and 317 positions of human TRβ are underlined in SEQ ID NO: 1. The portion of the human TRβ nucleotide sequence that encodes the above amino acid sequence is SEQ ID NO: 2. The nucleotide sequence of human TRβ is provided, *e.g*., by Genbank Accession No. NM_000461.4, incorporated herein by reference.

| |
|---|
| Nucleic acid sequence encoding the ligand binding domain of human TRβ (SEQ ID NO: 2) |
| GAGCTGCAGAAGTCCATCGGGCACAAGCCAGAGCCCACAGACGAGGAATGGGAGCTCATCAAAACTGTCACCGAA GCCCATGTGGCGACCAACGCCCAAGGCAGCCACTGGAAGCAAAAACGGAAATTCCTGCCAGAAGACATTGGACAA GCACCAATAGTCAATGCCCCAGAAGGTGGAAAGGTTGACTTGGAAGCCTTCAGCCATTTTACAAAAATCATCACA CCAGCAATTACCAGAGTGGTGGATTTTGCCAAAAAGTTGCCTATGTTTTGTGAGCTGCCATGTGAAGACCAGATC ATCCTCCTCAAAGGCTGCTGCATGGAGATCATGTCCCTTCGCGCTGCTGTGCGCTATGACCCAGAAAGTGAGACT TTAACCTTGAATGGGGAAATGGCAGTGACACGGGGCCAGCTGAAAAATGGGGGTCTTGGGGTGGTGTCAGACGCC ATCTTTGACCTGGGCATGTCTCTGTCTTCTTTCAACCTGGATGACACTGAAGTAGCCCTCCTTCAGGCCGTCCTG CTGATGTCTTCAGATCGCCCGGGGCTTGCCTGTGTTGAGAGAATAGAAAAGTACCAAGATAGTTTCCTGCTGGCC TTTGAACACTATATCAATTACCGAAAACACCACGTGACACACTTTTGGCCAAAACTCCTGATGAAGGTGACAGAT CTGCGGATGATAGGAGCCTGCCATGCCAGCCGCTTCCTGCACATGAAGGTGGAATGCCCCACAGAACTCTTCCCC CCTTTGTTCTTGGAAGTGTTCGAGGATTAG |

The TRβ mutation is selected from the group consisting of a substitution of threonine (T) for the wild type residue alanine (A) at amino acid position 234 of SEQ ID NO: 1 (A234T); a substitution of glutamine (Q) for the wild type residue arginine (R) at amino acid position 243 of SEQ ID NO: 1 (R243Q); a substitution of histidine (H) for the wild type residue arginine (R) at amino acid position 316 of SEQ ID NO: 1 (R316H); and a substitution of threonine (T) for the wild type residue alanine (A) at amino acid position 317 of SEQ ID NO: 1 (A317T).

The compositions disclosed herein are also useful as medicaments for the treatment of non-alcoholic steatohepatitis (NASH). NASH is liver inflammation and damage caused by a buildup of fat in the liver. Accordingly, the present disclosure provides the compositions disclosed herein for use in treating NASH in a subject in need thereof. The present disclosure also provides a method for treating NASH in a subject in need thereof, the method comprising administering to the subject a therapeutically effective amount of any one of the morphic forms, co-crystals, salts, and amorphous solid dispersions disclosed herein. The present disclosure also provides a crystalline salt, morphic form, co-crystal, or amorphous solid dispersion disclosed herein for the manufacture of a medicament for treating non-alcoholic steatohepatitis in a subject in need thereof, wherein the crystalline salt, morphic form, co-crystal, or amorphous solid dispersion disclosed herein is for administration to the subject in at least one therapeutically effective amount. The present disclosure also provides a crystalline salt, morphic form, co-crystal, or amorphous solid dispersion disclosed herein for use in treating non-alcoholic steatohepatitis in a subject in need thereof, wherein the crystalline salt, morphic form, co-crystal, or amorphous solid dispersion disclosed herein is for administration to the subject in at least one therapeutically effective amount.

The compositions disclosed herein are also useful as medicaments for the treatment of familial hypercholesterolemia (FH). FH is an inherited genetic disorder that causes dangerously high cholesterol levels, which can lead to heart disease, heart attack, or stroke at an early age if left untreated. There are two types of FH: homozygous FH (HoFH) and heterozygous FH (HeFH). Accordingly, the present disclosure provides the compositions disclosed herein for use in treating HoFH or HeFH in a subject in need thereof. The present disclosure also provides a method for treating HoFH or HeFH in a subject in need thereof, the method comprising administering to the subject a therapeutically effective amount of any one of the morphic forms, co-crystals, salts, and amorphous solid dispersions disclosed herein. The present disclosure also provides a crystalline salt, morphic form, co-crystal, or amorphous solid dispersion disclosed herein for the manufacture of a medicament for treating familial hypercholesterolemia in a subject in need thereof, wherein the crystalline salt, morphic form, co-crystal, or amorphous solid dispersion disclosed herein is for administration to the subject in at least one therapeutically effective amount. The present disclosure also provides a crystalline salt, morphic form, co-crystal, or amorphous solid dispersion disclosed herein for use in treating familial hypercholesterolemia in a subject in need thereof, wherein the crystalline salt, morphic form, co-crystal, or amorphous solid dispersion disclosed herein is for administration to the subject in at least one therapeutically effective amount.

The compositions disclosed herein are also useful as medicaments for the treatment of fatty liver disease. Fatty liver disease is a condition wherein large vacuoles of triglyceride fat accumulate in liver cells via the process of steatosis. Accordingly, the present disclosure provides the compositions disclosed herein for use in treating fatty liver disease in a subject in need thereof. The present disclosure also provides a method for treating fatty liver disease in a subject in need thereof, the method comprising administering to the subject a therapeutically effective amount of any one of the morphic forms, co-crystals, salts, and amorphous solid dispersions disclosed herein. The present disclosure also provides a crystalline salt, morphic form, co-crystal, or amorphous solid dispersion disclosed herein for the manufacture of a medicament for treating fatty liver disease in a subject in need thereof, wherein the crystalline salt, morphic form, co-crystal, or amorphous solid dispersion disclosed herein is for administration to the subject in at least one therapeutically effective amount. The present disclosure also provides a crystalline salt, morphic form, co-crystal, or amorphous solid dispersion disclosed herein for use in treating fatty liver disease in a subject in need thereof, wherein the crystalline salt, morphic form, co-crystal, or amorphous solid dispersion disclosed herein is for administration to the subject in at least one therapeutically effective amount.

The compositions disclosed herein are also useful as medicaments for the treatment of dyslipidemia. Dyslipidemia is a condition characterized by an abnormal amount of lipids (e.g. triglycerides, cholesterol and/or fat phospholipids) in the blood. Accordingly, the present disclosure provides the compositions disclosed herein for use in treating dyslipidemia in a subject in need thereof. The present disclosure also provides a method for treating dyslipidemia in a subject in need thereof, the method comprising administering to the subject a therapeutically effective amount of any one of the morphic forms, co-crystals, salts, and amorphous solid dispersions disclosed herein. The present disclosure also provides a crystalline salt, morphic form, co-crystal, or amorphous solid dispersion disclosed herein for the manufacture of a medicament for treating dyslipidemia in a subject in need thereof, wherein the crystalline salt, morphic form, co-crystal, or amorphous solid dispersion disclosed herein is for administration to the subject in at least one therapeutically effective amount. The present disclosure also provides a crystalline salt, morphic form, co-crystal, or amorphous solid dispersion disclosed herein for use in treating dyslipidemia in a subject in need thereof, wherein the crystalline salt, morphic form, co-crystal, or amorphous solid dispersion disclosed herein is for administration to the subject in at least one therapeutically effective amount.

The therapeutically effective amount or dosage according to this invention can vary within wide limits and may be determined in a manner known in the art. For example, the drug can be dosed according to body weight. Such dosage will be adjusted to the individual requirements in each particular case including the specific compound(s) being administered, the route of administration, the condition being treated, as well as the patient being treated. In another embodiment, the drug can be administered by fixed does, e.g., dose not adjusted according to body weight. In general, in the case of oral or parenteral administration to adult humans, a daily dosage of from about 0.5 mg to about 1000 mg should be appropriate, although the upper limit may be exceeded when indicated. The dosage is preferably from about 5 mg to about 400 mg per day. For example, the dosage is about 40 mg, about 50 mg, about 80 mg, about 100 mg, about 120 mg, about 140 mg, about 160 mg, about 180 mg, or about 200 mg. A preferred dosage may be from about 20 mg to about 200 mg per day. The daily dosage can be administered as a single dose or in divided doses, or for parenteral administration it may be given as continuous infusion.

An effective amount of a pharmaceutical agent is that which provides an objectively identifiable improvement as noted by the clinician or other qualified observer. As used herein, the term "dosage effective manner" refers to an amount of an active compound to produce the desired biological effect in a subject or cell.

The details of the invention are set forth in the accompanying description below. Although methods and materials similar or equivalent to those described herein can be used in the practice or testing of the present invention, illustrative methods and materials are now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs. All patents and publications cited in this specification are incorporated herein by reference in their entireties.

### Definitions

The articles "a" and "an" are used in this disclosure to refer to one or more than one (i.e., to at least one) of the grammatical object of the article. By way of example, "an element" means one element or more than one element.

The term "and/or" is used in this disclosure to mean either "and" or "or" unless indicated otherwise.

The term "solvate" is used herein to describe a morphic form that includes an organic solvent chemically incorporated with the parent molecule in various fractional or integral molar ratios.

The term "hydrate" is used herein to describe a morphic form that includes water chemically incorporated with the parent molecule in various fractional or integral molar ratios.

The term "desolvate" is used herein to describe a morphic form resulting from a solvent being substantially removed from a solvate, typically by heat, vacuum, or both. In some embodiments, at least 75% by weight of the solvent is removed from the solvate to form a desolvate. In some embodiments, at least 80% by weight of the solvent is removed from the solvate to form a desolvate. In some embodiments, at least 85% by weight of the solvent is removed from the solvate to form a desolvate. In some embodiments, at least 90% by weight of the solvent is removed from the solvate to form a desolvate. In some embodiments, at least 95% by weight of the solvent is removed from the solvate to form a desolvate. In some embodiments, at least 99% by weight of the solvent is removed from the solvate to form a desolvate.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient or carrier" means an excipient or carrier that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

The term "therapeutically effective amount", as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or condition to be treated is a metabolic disorder.

The term "subject" as used herein refers to a mammal, preferably a human.

"Treating" or "treatment" as used herein with regard to a condition may refer to preventing the condition, slowing the onset or rate of development of the condition, reducing the risk of developing the condition, preventing or delaying the development of symptoms associated with the condition, reducing or ending symptoms associated with the condition, generating a complete or partial regression of the condition, or some combination thereof.

As used herein, the term "about" when used in conjunction with numerical values and/or ranges generally refers to those numerical values and/or ranges near to a recited numerical value and/or range. In some instances, the term "about" can mean within ± 10% of the recited value. For example, in some instances, "about 100 [units]" can mean within ± 10% of 100 (e.g., from 90 to 110).

The term "substantially similar" used in reference to XRPD patterns means that the XRPD pattern of a polymorph may display "batch to batch" variations due to differences in the types of equipment used for the measurements, and fluctuations in both experimental conditions (e.g. purity and grain size of the sample) and instrumental settings (e.g. X-ray wavelengths; accuracy and sensitivity of the diffractometer; and "instrumental drift") normally associated with the X-ray diffraction measurement. Due to these variations, the same polymorph may not contain XRPD peaks at exactly the same positions or intensities shown in the figures disclosed herein. Accordingly, the term "about" used in reference to the peaks in an XRPD pattern takes into account these variations and a skilled artisan would readily appreciate the scope.

### Examples

The disclosure is further illustrated by the following examples and synthesis examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

### Example 1. Solvates and Desolvates of Compound A

A suspension of Compound A and methanol was stirred for 2 days then filtered. XRPD analysis indicated the formation of the methanol solvate (Form B, FIG. 2). In a similar manner, the ethanol solvate (Form C, FIG. 3), acetone solvate (Form D, FIG. 4), THF solvate (Form E, FIG. 5), MIBK solvate (Form G, FIG. 7), isopropyl acetate solvate (Form H, FIG. 8), acetic acid solvate (Form I, FIG. 9), dimethyl acetamide solvate (Form K, FIG. 10), and acetonitrile solvate (Form L, FIG. 11).

A suspension of Compound A and ethyl acetate was stirred for 2 days then filtered. Heating to 100°C for 1 hour generated the desolvate (Form F, FIG. 6). In a similar manner, a different desolvate, Form λ, is formed by heating the isopropyl acetate solvate (Form H, FIG. 27).

Form S+T was generated from the MIBK solvate of Compound A (Form G) by drying overnight at 30°C under vacuum (FIG. 12). In a similar manner, Form S was generated from Form H. Form U was generated from Form I. Form V was generated from Form L. Form W was generated from an ethyl acetate solvate, formed by slurrying Compound A in ethyl acetate at 50°C.

Form Y (FIG. 18), a desolvate, was generated by evaporating a saturated solution of Compound A in ethanol at 50 °C and atmospheric pressure to dryness. In a similar manner, the following forms were isolated from saturated solutions of Compound A: Form Z (FIG. 19) from acetic acid solution, and Form α (FIG. 20) from acetone solution.

About 50-70 mg of Compound A was accurately weighed in a 4 mL vial and heated to 60 °C. Then ethanol and a few drops of N-methyl pyrrolidone (NMP) were added. The contents were stirred with a magnetic stir bar until the solid was dissolved. The vial was placed in an ice/water mixture and kept cold until a precipitate formed. The solid was filtered to give Compound A Form β (FIG. 21), an NMP solvate. In a similar manner, Form χ (FIG. 22), a DMSO solvate, was obtained by crystallization from IPA:DMSO (96.5:3.5).

Form δ (FIG. 23), a possible THF solvate, was obtained when a solution of Compound A in THF at 60°C was added to either heptane or cyclohexane at room temperature. In a similar manner, Form C+ε (FIG. 24), a possible acetone solvate, was obtained by adding a solution of Compound A in ethanol: acetone (1:1) to heptane. Form ϕ (FIG. 25), and acetone solvate, was obtained by addition an acetone solution to heptane or cyclohexane.

The isopropyl alcohol (IPA) solvate Form η (FIG. 26) was generated by slurrying the MIBK solvate Form G of Compound A in IPA at room temperature.

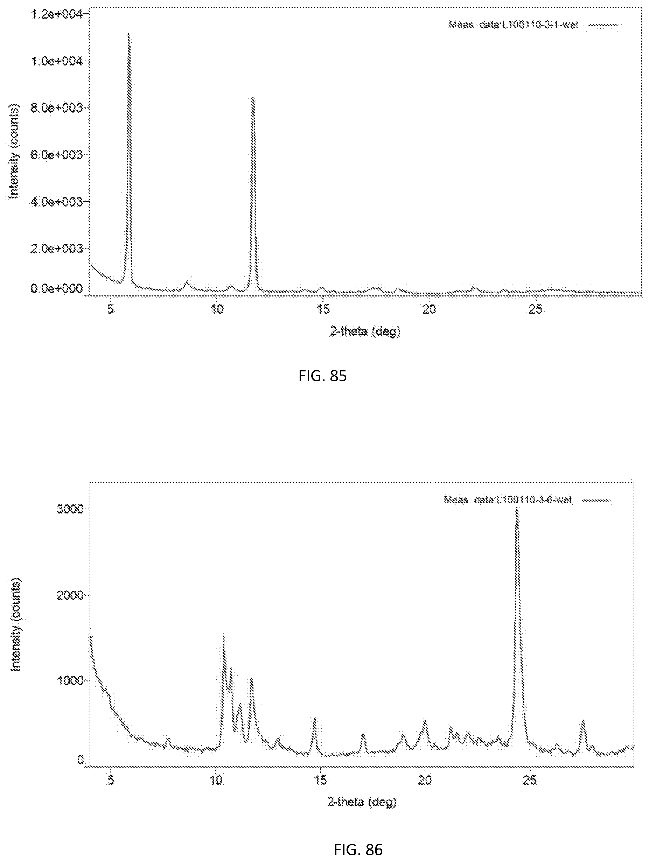

A mixture of Form B and Form M (FIG. 85) can be generated by slurring Form A in methanol at 50 °C.

A mixture of Form F and Form N (FIG. 86) can be generated by slurring Form A in ethyl acetate at 50 °C.

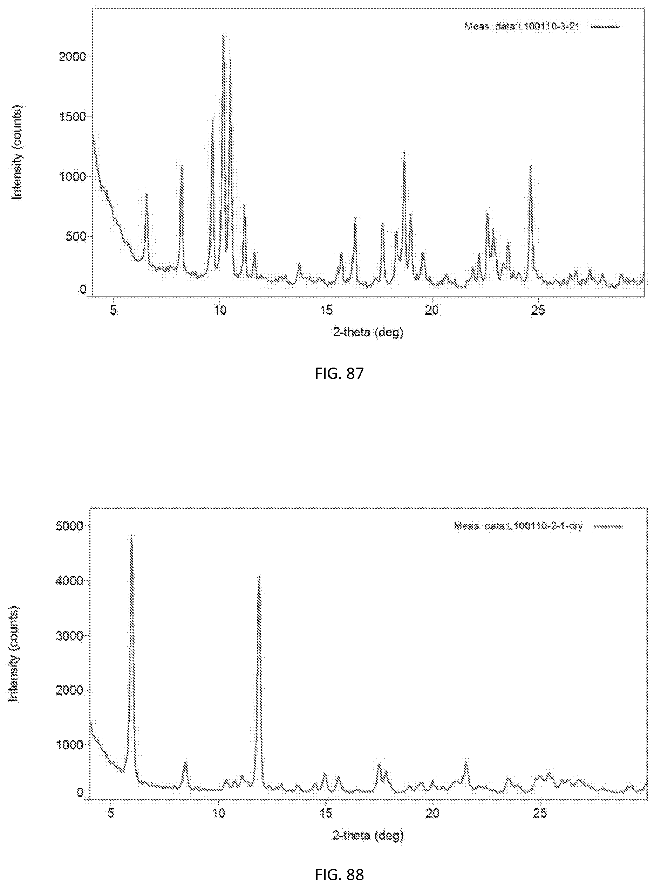

A mixture of Form A and Form O (FIG. 87) can be generated by slurring Form A in acetonitrile at 50 °C.

A mixture of Form B and Form P (FIG. 88) can be generated by drying methanol solvate at 30 °C under vacuum overnight.

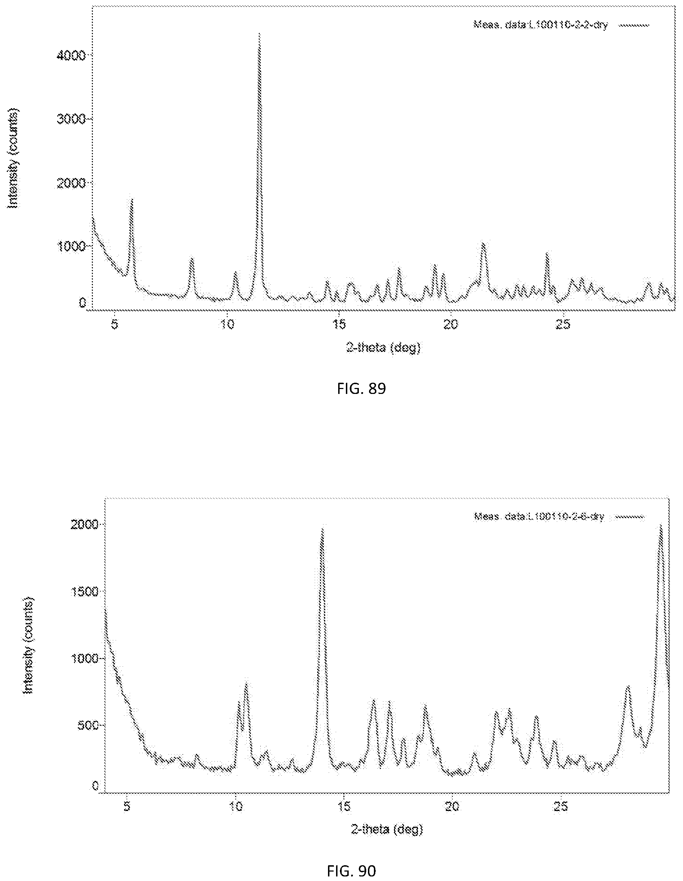

A mixture of Form C and Form Q (FIG. 89) can be generated by drying ethanol solvate at 30 °C under vacuum overnight.

A mixture of Form F and Form R (FIG. 90) can be generated by drying ethyl acetate solvate at 30 °C under vacuum overnight.

### Example 2. Amorphous Solid Dispersions of Compound A

The procedure used to prepare amorphous solid dispersions of Compound A is as follows.

The procedure for the experiments with 1:4 ratio of Compound A:Polymer: (1) Approximately 10 mg of Compound A was weighed in 4 mL vials and ~40 mg of the polymer is added to each of the respective vials; (2) Solvent was added to each of the respective vials for saturation; (3) Content of vials were stirred at room temperature, if dissolution was achieved at room temperature, the clear solution was transferred to 2 mL centrifuge tubes; (4) If complete dissolution was not achieved, vials were heated to 60°C to achieve dissolution. If still dissolution was not achieved at 60 °C, the vials were subjected to centrifugation and the supernatant was collected; (5) The solution/ supernatant was subjected to evaporation in SpeedVac at about 50 °C and under vacuum; (6) The resulting solid/gel was dried further in a vacuum oven for at least 3 hours which resulted in a solid; and (7) X-ray diffraction was performed on the resulting solid.

The polymers used were polyvinylpyrrolidone MW 10000 (PVP-10), polyvinylpyrrolidone MW 40000 (PVP-40), hypromellose or hydroxy propyl methyl cellulose (HPR), poly(1-vinylpyrrolidone-co-vinyl acetate) (PVP-Co-VA). The solvent systems used were THF:water 9:1, Ethanol:acetone 1:1, acetone, and acetic acid. In addition, amorphous solid dispersions were prepared using a 2:1 ratio of polymer to compound A and the ethanol:acetone 1:1 and acetic acid solvent systems.

### Example 3. Preparation of Co-crystals of Compound A

The generation of co-crystals was attempted through different modes such as solvent drop milling, evaporation of the Compound A/co-former solution, and co-melting beyond the melting point of the co-former. Form A of Compound A was used as the starting material in all the experiments.

The procedure for making a Compound A/glutaric acid co-crystal is described below.

Compound A (154.7 mg) was weighed in a 4 mL vial, and 56.7 mg of glutaric acid was added to the vial (~1.2 molar equivalents). The contents of the vial were well mixed with a spatula and heated up to 118 °C (20 °C higher than the melting point of co-former). The vial was kept for ~5 minutes and cooled to 88 °C, kept for 15-20 minutes and cooled down to RT. Partial conversion of Compound A to the co-crystal was observed by XRPD.

An additional 0.8 equivalents of glutaric acid were added to this solid. The vial was heated to 118 °C. The internal temperature of the system was also checked with a thermocouple and was found to be 108 °C. The system was kept at this temperature for 15-20 minutes with periodic mixing with a spatula, cooled down to 88 °C. XRPD analysis confirmed conversion to the cocrystal of Compound A and glutaric acid.

The solid that was obtained after adding extra glutaric acid followed by thermal treatment was washed with distilled water (4 X 500 µL ~13.5 vol. with respect to Compound A) and was subjected to XRPD, no traces of excess glutaric acid was seen. This solid was further dried in a vacuum oven at 50 °C for 3-4 hours.

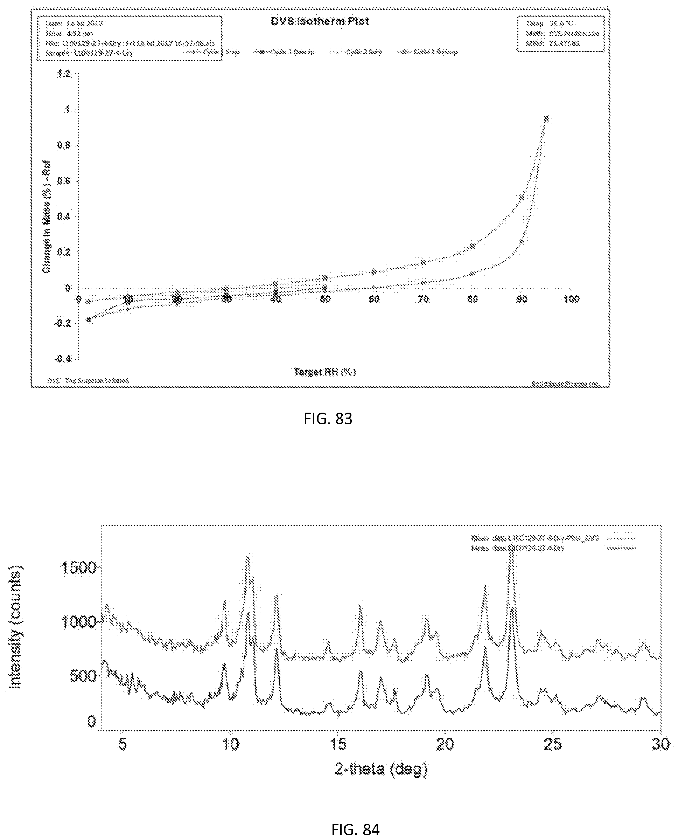

The co-crystal was not hygroscopic, with only 1.12% mass gain between 2% and 95% relative humidity (RH) environments and it was stable when subjected to 75% RH at 40 °C for 1 week. The isotherm is shown in FIG. 83. No difference in the XRPD pattern was seen after the DVS analysis, the XRPD patterns before and after DVS analysis are shown in FIG. 84.

The kinetic and thermodynamic solubility of the glutaric acid co-crystal was assessed in simulated fasted state gastric and intestinal fluids (FaSSGF and FaSSIF) and also in water. The pH of the system was also measured. The solubility assessment was also performed on Compound A for comparison. A small amount of Compound A and also the co-crystal were slurried in the respective fluid and a sample is collected after 1 hour for kinetic solubility assessment. Samples were collected after 24 hours for equilibrium solubility measurement. The solubility determination is done by HPLC through a short 20-minute generic method that was used during the salt screening project 100177FF. The solubility data is presented in Table 1.

**Table 1. Solubilities of the Compound A free acid (FA) and the glutaric acid co-crystal in the simulated fluids FaSSGF, FaSSIF and in water at 37 °C**

| **Experiment ID** | **Solvent** | **Compound** | **Kinetic/Equib** | **Area** | **Solubility (mg/mL)** | **pH** | **XRP D after slurry** |
|---|---|---|---|---|---|---|---|
| L100129-29-4 | FaSSGF | Form A | Kinetic - 1 hour | 6.148 | 0.0011 | | |
| L100129-29-5 | FaSSIF | Form A | Kinetic - 1 hour | 2654.464 | 0.4895 | | |
| L100129-29-6 | Water | Form A | Kinetic - 1 hour | 140.511 | 0.0259 | | |
| L100129-29-7 | FaSSGF | Glutaric acid co-crystal | Kinetic - 1 hour | 0.551 | 0.0001 | | |
| L100129-29-8 | FaSSIF | Glutaric acid co-crystal | Kinetic - 1 hour | 1301.012 | 0.2399 | | |
| L100129-29-9 | Water | Glutaric acid co-crystal | Kinetic - 1 hour | 1.583 | 0.0003 | | |
| L100129-29-4 | FaSSGF | Form A | Equilibrium - 24 hours | 6.160 | 0.0011 | 1.67 | Form-A |
| L100129-29-5 | FaSSIF | Form A | Equilibrium - 24 hours | 2994.47 | 0.5522 | 6.44 | Form-A |
| L100129-29-6 | Water | Form A | Equilibrium - 24 hours | 1046.449 | 0.1930 | 6.57 | Dihyd rate |
| L100129-29-7 | FaSSGF | Glutaric acid co-crystal | Equilibrium - 24 hours | 5.214 | 0.0010 | 1.66 | Dihyd rate |
| L100129-29-8 | FaSSIF | Glutaric acid co-crystal | Equilibrium - 24 hours | 2592.566 | 0.4781 | 6.31 | Dihyd rate |
| L100129-29-9 | Water | Glutaric acid co-crystal | Equilibrium - 24 hours | 8.556 | 0.0016 | 4.27 | Dihyd rate |

### Example 4. Salt Screening of Compound A

Anhydrous Form A of Compound A was used as the starting material in all the experiments.

Several different counter-ions (CIs) were used in an attempt to make salts with Compound A. Counter-ions Ca and Mg were used in two different ways, as their hydroxides, and also to produce hydroxide in-situ (in the form of CaO + Water or MgCl₂ + NaOH) to improve the chances of salt formation since the solubility of Ca and Mg hydroxides is poor in most of solvents/solvent systems. Several counter-ion systems used were: calcium hydroxide, calcium oxide (reacted with water in-situ), magnesium hydroxide, magnesium chloride (reacted with sodium hydroxide in-situ), sodium hydroxide, potassium hydroxide, ethanolamine, diethanolamine, triethanolamine, diethylamine, ethylenediamine, ethanol, 2-(diethylamine), choline hydroxide, L-arginine, L-histidine, L-lysine, meglumine. The IUPAC name for choline is 2-hydroxy-*N*,*N*,*N*-trimethylethan-1-aminium.

The initial salt screening was performed starting with 30 mg of Compound A and 1.1 equivalent of CI, both added as solutions in all cases (except in the case of the CIs Ca and Mg), to improve the salt formation. The vials were evaporated to dryness and slurries were made in five different process solvents to further improve the chances of salt formation. The slurries were filtered and the solids were analyzed by XRPD. Solids with unique patterns were dried and analyzed by XRPD again to see the effect of drying on solid form, all unique patterns were then exposed to saturated humidity environment at room temperature overnight and were re-subjected to XRPD. TGA/DSC was performed on all unique dry solids to identify their melting points and also to see the weight loss upon heating (to identify whether the solid is a solvate). H-NMR and HPLC analyses were performed on at least one version of the salt, to look for Compound A:CI stoichiometry, residual solvent content and also to look for signs of degradation of Compound A upon forming the salt.

All the counter-ions except ethylenediamine formed salts with Compound A. Use of ethylenediamine resulted in the degradation of Compound A, observed through HPLC. Scale-ups have been performed on all salts starting with ~200 mg of Compound A. Two experiments were also conducted with 0.5 Eq Ca and Mg hydroxides to test the likelihood of forming a hemi-salt. The experiment with 0.5 Eq Calcium Hydroxide resulted in a new pattern that is different from the patterns obtained with 1 Eq. CI, indicating the formation of a calcium hemi-salt. However, magnesium hydroxide resulted in a solid that had the same pattern as its counter-part with 1 Eq CI.

The solubility of one form of each salt in Fasted State Simulated Gastric Fluid (FaSSGF), Fasted State Simulated Intestinal Fluid (FaSSIF) and Water was measured at 37 °C. Normally, for each salt the solid form that showed relatively better stability in drying or humidity was scaled up and used in these experiments. In general, the effect of salt itself is more profound than individual solid forms of the salt when compared with the free molecule. However, the salts resulted in disproportionation in FaSSGF. The resulting solid was free molecule di-hydrate form by XRPD. However, the L-Histidine salt stayed intact. Several salts exhibited higher solubility in water. Notably, the sodium and potassium salts showed a solubility higher than 20 mg/mL in water. All the salts of Compound A obtained from the scale-up experiments were exposed to 75% relative humidity environment for 1 week for physical form stability assessment and changes were observed primarily in the case of the Hemi Ca and meglumine salts of Compound A when compared with their starting solids.

***Preparation of Potassium Salt Acetic Acid Solvate of Compound A*:** To a 20 L Stainless Steel pressure vessel was charged Int. G (950 g, 1 equiv., 1.97 moles), KOAc (213 g, 1.1 equiv., 2.17 moles) and THF (14.25 L, 15 Vol.). The vessel was closed and pressurized with 10 psig of nitrogen, agitated with an overhead stirrer at 1000 rpm and heated to 90 °C. During this time the pressure of the vessel rose to 41 psig, the reaction was continued at this pressure and at a temperature of 92 °C for approximately 12 h. The batch was then cooled to ambient temperature. The fine solids were filtered through an 18" neutsche filter set up with a tight weave polypropylene cloth under nitrogen. The batch was filtered for a period of two hours. Following the initial filtration, the cake was slurry washed with 5 volumes of THF (4.75 L) four times, then was conditioned under nitrogen for 12 h, transferred to trays and dried in a vacuum oven (45 °C) for 2 days. The final isolated Compound A K-salt (18AK0164H) weighed 732 g (79%) yield with an overall purity of 99.60% AUC by UPLC with N/D MGl-100171(UPLC method) and <20 ppm of the dimeric impurity. NMR of the batch showed a 1:1.1 ratio of Compound A K- Salt to AcOH. The material showed 5.7 wt% of THF and a potency of 117%, uncorrected.

***Conversion of Potassium-salt Acetic Acid Solvate of Compound A to THF solvate-hydrate:*** A 9 L carboy was charged with Compound A K-salt ( 620 g, 1 equiv., Lot # 18AK0164H), K2CO3 (72 g, 0.4 equiv.), THF (1.24 L, 2 vol., bulk quality) and water (3.72 L, 6 vol.). The batch was agitated with an air motor stirrer to dissolve all the solids. After 15 minutes of stirring, the orange solution was transferred into a 10 L jacketed reactor via a 10 micron polypropylene filter using a transfer pump over 5 minutes. The lines were rinsed with DI water (465 mL)/THF (175 mL) mixture followed by a DI water rinse. The batch temperature was adjusted to 20 °C and acetic acid (0.165 L, 2.20 equiv.) charged to the batch over 1 h. Slurry formation started after about a quarter of the acid charged into the batch. After 2 h hold time, DI water (1.86 L, 3.00 vol.) was charged to the batch over 2 h at 20 °C and the slurry aged at 20 °C overnight. The batch was filtered and the filter cake washed with 1:5 THF/water (2 x 2 vol.) then dried in a vacuum oven at 45 °C for 12 h to give 570 g (86% yield) of Compound A THF Solvate (Lot # 18AK0193C) of 99.81% purity by UPLC and with THF: H₂O molar ratio of 0.56: 0.67.

***Preparation of Potassium Salt Tetrahydrofuran Solvate of Compound A from Compound A DMAc solvate* (ASV-BO-194):** To a 500-mL jacketed reactor equipped with mechanical stirrer, temperature probe, condenser and N₂ inlet was charged Compound A DMAc solvate (24.5 g, 46.90 mmol, 1.00 equiv.), potassium carbonate (7.13 g, 51.59 mmol, 1.10 equiv.), MEK (245 mL, 10.0 vol.) and DI water (1.27 mL, 70.36 mmol, 1.50 equiv.). The slurry was heated to 45 °C over 1 hour and held at that temperature for 6 h. The batch was cooled to 20 °C over 3 h then held overnight at 20 °C. The batch was then cooled to 5-10 °C then aged at that temperature for 1 h 45 min before being filtered to collect the solid product, which was manually smoothened on the filter and conditioned to deliquor the cake. The wet cake was slurried in THF (49 mL, 2.0 vol.) and aged for 30 min. The slurry was then filtered under vacuum. This THF-slurry procedure was repeated four more times, giving a total of 5 x 2 vol. THF washes. After the fifth wash, the batch was conditioned until no further THF emerged for the cake, then dried under vacuum at 40 °C (on the filter) to afford Compound A K salt (22.47 g, 85% uncorrected, Lot # ASV-BO-194-12). ¹H NMR showed a Compound A K-salt / THF / water mole ratio: 1.00 : 0.22 : 0.51.

**Conversion of Compound A Potassium salt to Compound A Tetrahydrofuran Solvate:** Compound A potassium salt (16.50 g, 34.86 mmol, Lot # ASV-BP-6-8): Analysis: Compound A Potassium salt / DMAc / THF / water mole ratio: 1.00 : 0.02 : 0.16 : 0.65) on the filter frit was suspended in 60 mL of a THF/water solution prepared by mixing 99 mL (6 vol.) DI water and 33 mL (2 vol.) THF and the resultant slurry was stirred at room temperature, affording partial dissolution. The mixture was filtered, then the solid residue was suspended in ~30 mL of the THF/water solution. Further dissolution occurred after agitation. This mixture was then filtered. Again the solid residue was suspended in ~30 mL of the THF/water solution. The majority of the residual solid dissolved and this was then filtered. With the 500-mL jacketed reactor as the receiver under vacuum, the filtrate was transferred to the reactor through in-line filter (Whatman, 0.3 micron glass microfiber filter) into the reactor. During the transfer process, residual solid was observed in the filter line. This wash rinsed into the reactor with the aid of an additional 3:1 water/THF solution (1 vol.), followed by a DI water rinse (16 mL, 1.0 vol.). The batch temperature was adjusted to 20°C and acetic acid (4.4 mL, 2.20 molar equiv.) charged to the batch over 30 min. After a 1.25 hour hold, DI water (50 mL, 3.00 vol.) was charged to the tan slurry batch over 2 h at 20°C and the slurry aged at 20°C overnight. The batch was filtered and the filter cake washed with 1: 5 THF/Water (2 x 49 mL, 2 x 2 vol.) and dried in a vacuum oven at 41°C to afford Compound A THF solvate (13.08 g, Lot # ASV-BP-18-3). ¹H NMR analysis showed a Compound A : THF mole ratio of 1.00 : 0.95.

**Table 2. Solubility**

| ***Exp Id (L100-)*** | **CI** | **System** | **Area** | **Solubility, mg/mL** | **Resulting form** |
|---|---|---|---|---|---|
| L100110-3-2 | --- | FaSSGF | | 0.1 | Free form Anhydrous |
| L100110-19-5 | --- | FaSSGF | | 0.0067 | Free form dihydrate |
| 110-86-1 | Ca | FASSGF | 19.122 | 0.0010 | Dihydrate |
| 110-86-4 | Hemi Ca | FASSGF | 46.495 | 0.0025 | Dihydrate |
| 110-86-7 | Mg | FASSGF | 16.168 | 0.0009 | Dihydrate |
| 110-86-10 | Na | FASSGF | 14.098 | 0.0008 | Dihydrate |
| 110-86-13 | K | FASSGF | 22.635 | 0.0012 | Dihydrate |
| 110-86-16 | Hemi Mg | FASSGF | 14.186 | 0.0008 | Dihydrate |
| L100110-19-3 | --- | FaSSIF | | 4.5 | Free form Anhydrous |
| L100110-19-6 | --- | FaSSIF | | 2.9 | Free form dihydrate |
| 110-86-2 | Ca | FASSIF | 10714.18 | 0.58 | Amorphous |
| 110-86-5 | Hemi Ca | FASSIF | 14766.83 | 0.80 | Hydrate of Calcium salt primarily (2-D) |
| 110-86-8 | Mg | FASSIF | 29216.45 | 1.59 | Weakly hydrate of Magnesium salt (3-C) |
| 110-86-11 | Na | FASSIF | 45994.03 | 2.50 | Dihydrate+Extra * |
| 110-86-14 | K | FASSIF | 50008.67 | 2.72 | Dihydrate+Extra * |
| 110-86-17 | Hemi Mg | FASSIF | 25888.9 | 1.41 | Dihydrate |
| L100110-34-1 | --- | Water | | 0.2 | Free form Anhydrous |
| L100110-19-4 | --- | Water | | 0.1 | Free form dihydrate |
| 110-86-3 | Ca | Water | 6486.92 | 0.35 | Dihydrate |
| 110-86-6 | Hemi Ca | Water | 5912.01 | 0.32 | Dihydrate |
| 110-86-9 | Mg | Water | 12032.2 | 0.65 | Dihydrate |
| 110-86-12 | Na-100X dil | Water | 4615.626 | 0.25 | Clear solution (solubility > 25 mg/mL) |
| 110-86-15 | K-100X dil | Water | 3921.429 | 0.21 | Clear solution (solubility > 21.3 mg/mL) |
| 110-86-18 | Hemi Mg | Water | 12371.22 | 0.67 | Dihydrate |

### Example 5. Method for Obtaining XRPD Patterns

X-ray powder diffraction was done using a Rigaku MiniFlex 600. Samples were prepared on Si zero-return wafers. A typical scan is from 2θ of 4 to 30 degrees, with step size 0.05 degrees over five minutes with 40 kV and 15 mA. A high-resolution scan is from 2θ of 4 to 40 degrees, with step size 0.05 degrees over thirty minutes with 40 kV and 15 mA. Typical parameters for XRPD are listed below.

X-ray wavelength: Cu Kα1, 1.540598 Å; X-ray tube setting: 40 kV, 15 mA; Slit condition: variable + fixed slit system; Scan mode: continuous; Scan range (°2TH): 4-30; Step size (°2TH): 0.05; Scan speed (°/min): 5.

Tables 3-80 provide the major 2θ peaks and d-spacings for each of the crystalline forms disclosed herein.

**Table 3. XRPD Peak positions of Form A**

| 2-θ | d (A°) | Height (counts) | Relative Intensity, % |
|---|---|---|---|
| 8.27 | 10.68878 | 1607 | 37 |
| 10.56 | 8.37014 | 4379 | 100 |
| 11.21 | 7.8855 | 1470 | 34 |
| 15.81 | 5.59963 | 660 | 15 |
| 16.43 | 5.38989 | 1085 | 25 |
| 17.72 | 5.00087 | 1342 | 31 |
| 18.45 | 4.80591 | 1166 | 27 |
| 18.75 | 4.72983 | 3414 | 78 |
| 22.30 | 3.98389 | 772 | 18 |
| 22.70 | 3.91432 | 1990 | 45 |
| 22.99 | 3.86513 | 1803 | 41 |
| 23.63 | 3.7622 | 1491 | 34 |
| 24.72 | 3.59862 | 2192 | 50 |
| 26.57 | 3.35183 | 274 | 6 |
| 27.49 | 3.24143 | 268 | 6 |
| 29.00 | 3.07664 | 323 | 7 |
| 29.52 | 3.02378 | 259 | 6 |
| 30.13 | 2.96403 | 922 | 21 |
| 32.04 | 2.79156 | 414 | 9 |
| 32.33 | 2.76688 | 423 | 10 |
| 35.25 | 2.54415 | 265 | 6 |

**Table 4. XRPD Peak positions of Form B**

| 2-θ | d (A°) | Height (counts) | Relative Intensity, % |
|---|---|---|---|
| 5.92 | 14.91066 | 5642 | 100 |
| 11.80 | 7.49683 | 4906 | 87 |
| 14.95 | 5.9199 | 298 | 5 |
| 17.46 | 5.07401 | 502 | 9 |

**Table 5. XRPD Peak positions of Form C**

| 2-θ | d (A°) | Height (counts) | Relative Intensity, % |
|---|---|---|---|
| 5.74 | 15.39153 | 2067 | 27 |
| 10.37 | 8.51986 | 480 | 6 |
| 11.45 | 7.72222 | 7751 | 100 |
| 15.42 | 5.74041 | 484 | 6 |
| 15.83 | 5.59273 | 350 | 5 |
| 16.70 | 5.30287 | 510 | 7 |
| 17.19 | 5.15513 | 654 | 8 |
| 17.69 | 5.01081 | 892 | 12 |
| 19.27 | 4.60254 | 1010 | 13 |
| 19.66 | 4.5128 | 787 | 10 |
| 21.41 | 4.14655 | 1028 | 13 |
| 22.95 | 3.87215 | 474 | 6 |
| 23.26 | 3.82149 | 550 | 7 |
| 24.28 | 3.66251 | 1400 | 18 |
| 24.55 | 3.62315 | 550 | 7 |
| 25.81 | 3.44968 | 545 | 7 |
| 26.25 | 3.39277 | 456 | 6 |
| 26.54 | 3.35528 | 304 | 4 |
| 28.82 | 3.09492 | 560 | 7 |
| 29.39 | 3.03701 | 441 | 6 |

**Table 6. XRPD Peak positions of Form D**

| 2-θ | d (A°) | Height (counts) | Relative Intensity, % |
|---|---|---|---|
| 5.52 | 16.00195 | 1507 | 17 |
| 8.52 | 10.37128 | 2171 | 24 |
| 11.01 | 8.03145 | 9097 | 100 |
| 16.49 | 5.3729 | 1420 | 16 |
| 17.19 | 5.15457 | 417 | 5 |
| 18.25 | 4.85666 | 1357 | 15 |
| 20.13 | 4.40764 | 781 | 9 |
| 21.03 | 4.22172 | 873 | 10 |
| 21.20 | 4.18738 | 1401 | 15 |
| 23.38 | 3.80249 | 412 | 5 |
| 24.03 | 3.70114 | 1717 | 19 |
| 25.61 | 3.47527 | 543 | 6 |
| 28.51 | 3.12802 | 425 | 5 |

**Table 7. XRPD Peak positions of Form E**

| 2-θ | d (A°) | Height (counts) | Relative Intensity, % |
|---|---|---|---|
| 7.13 | 12.39225 | 2144 | 100 |
| 8.77 | 10.08041 | 286 | 13 |
| 10.06 | 8.78695 | 362 | 17 |
| 10.81 | 8.17815 | 993 | 46 |
| 11.45 | 7.71869 | 207 | 10 |
| 12.05 | 7.34104 | 410 | 19 |
| 12.34 | 7.16766 | 853 | 40 |
| 14.14 | 6.26049 | 1196 | 56 |
| 14.73 | 6.01006 | 2013 | 94 |
| 15.01 | 5.89719 | 180 | 8 |
| 15.47 | 5.72179 | 431 | 20 |
| 16.09 | 5.50291 | 1244 | 58 |
| 16.67 | 5.31374 | 340 | 16 |
| 17.46 | 5.074 | 477 | 22 |
| 18.10 | 4.89713 | 730 | 34 |
| 18.64 | 4.7562 | 281 | 13 |
| 19.88 | 4.46331 | 689 | 32 |
| 20.19 | 4.39405 | 635 | 30 |
| 21.04 | 4.21909 | 431 | 20 |
| 21.22 | 4.18406 | 965 | 45 |
| 21.63 | 4.10543 | 290 | 14 |
| 22.65 | 3.92339 | 1035 | 48 |
| 22.90 | 3.88116 | 819 | 38 |
| 24.36 | 3.65154 | 620 | 29 |
| 24.66 | 3.60676 | 202 | 9 |
| 25.34 | 3.51261 | 1159 | 54 |
| 26.34 | 3.38042 | 453 | 21 |
| 27.10 | 3.28758 | 264 | 12 |
| 27.28 | 3.26673 | 187 | 9 |
| 27.77 | 3.20938 | 362 | 17 |
| 28.77 | 3.10029 | 167 | 8 |
| 29.32 | 3.04372 | 302 | 14 |
| 29.60 | 3.0152 | 263 | 12 |

**Table 8. XRPD Peak positions of Form F**

| 2-θ | d (A°) | Height (counts) | Relative Intensity, % |
|---|---|---|---|
| 7.28 | 12.13064 | 156 | 13 |
| 10.12 | 8.73306 | 925 | 78 |
| 10.41 | 8.4933 | 920 | 78 |
| 11.37 | 7.77672 | 292 | 25 |
| 13.91 | 6.36341 | 1179 | 100 |
| 16.20 | 5.46847 | 262 | 22 |
| 16.38 | 5.40798 | 331 | 28 |
| 17.05 | 5.19679 | 456 | 39 |
| 18.41 | 4.81512 | 162 | 14 |
| 18.90 | 4.69209 | 77 | 7 |
| 19.27 | 4.60216 | 129 | 11 |
| 20.94 | 4.23938 | 107 | 9 |
| 22.00 | 4.03681 | 396 | 34 |
| 22.51 | 3.94607 | 226 | 19 |
| 23.21 | 3.82963 | 73 | 6 |
| 23.81 | 3.73378 | 424 | 36 |
| 25.30 | 3.51739 | 74 | 6 |
| 25.85 | 3.44375 | 81 | 7 |
| 27.97 | 3.18771 | 227 | 19 |
| 29.53 | 3.02271 | 676 | 57 |
| 29.87 | 2.98899 | 83 | 7 |

**Table 9. XRPD Peak positions of Form G**

| 2-θ | d (A°) | Height (counts) | Relative Intensity, % |
|---|---|---|---|
| 7.71 | 11.45694 | 321 | 6 |
| 9.50 | 9.29954 | 3351 | 64 |
| 9.80 | 9.02038 | 264 | 5 |
| 10.70 | 8.26425 | 335 | 6 |
| 11.42 | 7.74427 | 573 | 11 |
| 12.91 | 6.85398 | 1574 | 30 |
| 13.06 | 6.77304 | 630 | 12 |
| 14.16 | 6.25129 | 331 | 6 |
| 15.32 | 5.78082 | 242 | 5 |
| 16.39 | 5.40376 | 342 | 7 |
| 16.72 | 5.29747 | 1503 | 29 |
| 16.93 | 5.23349 | 953 | 18 |
| 17.28 | 5.12802 | 1583 | 30 |
| 18.26 | 4.85418 | 367 | 7 |
| 18.95 | 4.67825 | 651 | 13 |
| 19.52 | 4.545 | 1324 | 25 |
| 20.20 | 4.39282 | 1156 | 22 |
| 20.75 | 4.27707 | 290 | 6 |
| 21.17 | 4.19266 | 499 | 10 |
| 22.94 | 3.87415 | 805 | 15 |
| 24.11 | 3.68838 | 255 | 5 |
| 24.39 | 3.64666 | 481 | 9 |
| 25.59 | 3.47856 | 5204 | 100 |
| 26.91 | 3.31057 | 389 | 7 |
| 28.26 | 3.15574 | 3363 | 65 |
| 29.10 | 3.06569 | 428 | 8 |

**Table 10. XRPD Peak positions of Form H**

| 2-θ | d (A°) | Height (counts) | Relative Intensity, % |
|---|---|---|---|
| 9.22 | 9.57947 | 7600 | 100 |
| 11.80 | 7.49312 | 869 | 11 |
| 12.08 | 7.32216 | 348 | 5 |
| 13.17 | 6.71738 | 1220 | 16 |
| 15.63 | 5.6641 | 712 | 9 |
| 16.77 | 5.28195 | 1040 | 14 |
| 18.40 | 4.81891 | 1013 | 13 |
| 19.34 | 4.58495 | 1245 | 16 |
| 19.78 | 4.48413 | 2744 | 36 |
| 21.18 | 4.19151 | 919 | 12 |
| 21.38 | 4.15202 | 481 | 6 |
| 22.78 | 3.90049 | 1111 | 15 |
| 22.98 | 3.8673 | 646 | 9 |
| 23.58 | 3.77027 | 2990 | 39 |
| 24.18 | 3.67827 | 830 | 11 |
| 25.11 | 3.54312 | 586 | 8 |
| 25.87 | 3.44169 | 1788 | 24 |
| 27.01 | 3.29814 | 349 | 5 |
| 28.00 | 3.18407 | 1677 | 22 |
| 28.55 | 3.12393 | 351 | 5 |
| 29.01 | 3.07499 | 439 | 6 |

**Table 11. XRPD Peak positions of Form I**

| 2-θ | d (A°) | Height (counts) | Relative Intensity, % |
|---|---|---|---|
| 5.15 | 17.14998 | 234 | 5 |
| 5.77 | 15.29146 | 796 | 18 |
| 9.30 | 9.50135 | 1010 | 23 |
| 9.61 | 9.1997 | 262 | 6 |
| 10.23 | 8.64045 | 4364 | 100 |
| 11.55 | 7.65485 | 1675 | 38 |
| 15.33 | 5.77637 | 313 | 7 |
| 21.87 | 4.06139 | 707 | 16 |
| 22.29 | 3.9856 | 337 | 8 |
| 23.80 | 3.73541 | 375 | 9 |

**Table 12. XRPD Peak positions of Form K**

| 2-θ | d (A°) | Height (counts) | Relative Intensity, % |
|---|---|---|---|
| 8.42 | 10.48827 | 4822 | 100 |
| 10.36 | 8.53374 | 365 | 8 |
| 11.37 | 7.77279 | 2754 | 57 |
| 12.96 | 6.8237 | 817 | 17 |
| 13.67 | 6.47249 | 872 | 18 |
| 14.45 | 6.12352 | 983 | 20 |
| 15.56 | 5.69196 | 922 | 19 |
| 16.42 | 5.39359 | 285 | 6 |
| 18.04 | 4.9142 | 354 | 7 |
| 18.90 | 4.69153 | 988 | 21 |
| 20.46 | 4.33778 | 392 | 8 |
| 21.14 | 4.19865 | 1327 | 28 |
| 21.56 | 4.11889 | 1755 | 36 |
| 23.65 | 3.75927 | 574 | 12 |
| 23.96 | 3.71172 | 688 | 14 |
| 24.94 | 3.5668 | 226 | 5 |
| 25.32 | 3.51471 | 366 | 8 |
| 25.57 | 3.48028 | 819 | 17 |
| 26.03 | 3.42028 | 694 | 14 |
| 26.20 | 3.39821 | 284 | 6 |
| 26.66 | 3.34086 | 646 | 13 |
| 27.20 | 3.27634 | 293 | 6 |
| 29.25 | 3.0512 | 417 | 9 |

**Table 13. XRPD Peak positions of Form L**

| 2-θ | d (A°) | Height (counts) | Relative Intensity, % |
|---|---|---|---|
| 8.11 | 10.88728 | 170 | 12 |
| 10.53 | 8.39548 | 319 | 23 |
| 11.49 | 7.6921 | 904 | 64 |
| 11.87 | 7.45234 | 1402 | 100 |
| 12.27 | 7.20569 | 141 | 10 |
| 15.21 | 5.82222 | 794 | 57 |
| 15.65 | 5.6584 | 470 | 34 |
| 16.04 | 5.52206 | 591 | 42 |
| 16.88 | 5.24756 | 455 | 32 |
| 17.10 | 5.18216 | 849 | 61 |
| 17.70 | 5.00658 | 143 | 10 |
| 18.43 | 4.81125 | 278 | 20 |
| 18.71 | 4.73921 | 332 | 24 |
| 21.32 | 4.16467 | 128 | 9 |
| 22.04 | 4.02938 | 413 | 29 |
| 22.81 | 3.89506 | 695 | 50 |
| 23.45 | 3.79117 | 422 | 30 |
| 24.08 | 3.69229 | 206 | 15 |
| 24.72 | 3.59921 | 266 | 19 |
| 26.37 | 3.37757 | 328 | 23 |
| 29.03 | 3.07381 | 91 | 6 |
| 29.49 | 3.02679 | 70 | 5 |

**Table 14. XRPD Peak positions of Form S+T**

| 2-θ | d (A°) | Height (counts) | Relative Intensity, % |
|---|---|---|---|
| 7.42 | 11.91007 | 448 | 36 |
| 8.80 | 10.03632 | 152 | 12 |
| 9.36 | 9.44359 | 213 | 17 |
| 9.50 | 9.30169 | 121 | 10 |
| 10.50 | 8.4148 | 1239 | 98 |
| 11.25 | 7.86127 | 336 | 27 |
| 12.39 | 7.14051 | 450 | 36 |
| 12.91 | 6.8537 | 74 | 6 |
| 14.30 | 6.18723 | 1260 | 100 |
| 14.74 | 6.00598 | 244 | 19 |
| 15.32 | 5.77749 | 93 | 7 |
| 15.81 | 5.60115 | 718 | 57 |
| 16.75 | 5.28996 | 268 | 21 |
| 17.13 | 5.17217 | 136 | 11 |
| 17.72 | 5.00027 | 285 | 23 |
| 18.09 | 4.90081 | 312 | 25 |
| 18.39 | 4.82152 | 979 | 78 |
| 19.61 | 4.52303 | 64 | 5 |
| 20.09 | 4.41564 | 594 | 47 |
| 20.54 | 4.32074 | 281 | 22 |
| 21.10 | 4.20647 | 315 | 25 |
| 21.89 | 4.05726 | 364 | 29 |
| 22.53 | 3.94393 | 78 | 6 |
| 23.23 | 3.8257 | 1048 | 83 |
| 24.40 | 3.64512 | 208 | 17 |
| 25.48 | 3.49332 | 514 | 41 |
| 26.86 | 3.31655 | 262 | 21 |
| 27.78 | 3.20839 | 237 | 19 |
| 28.33 | 3.14737 | 388 | 31 |

**Table 15. XRPD Peak positions of Form S**

| 2-θ | d (A°) | Height (counts) | Relative Intensity, % |
|---|---|---|---|
| 7.39 | 11.95027 | 327 | 24 |
| 8.73 | 10.11806 | 359 | 26 |
| 9.18 | 9.62337 | 51 | 4 |
| 10.54 | 8.38858 | 1266 | 91 |
| 11.27 | 7.84766 | 278 | 20 |
| 12.34 | 7.16511 | 693 | 50 |
| 14.40 | 6.14604 | 1384 | 100 |
| 14.74 | 6.00469 | 188 | 14 |
| 15.40 | 5.7507 | 132 | 10 |
| 15.79 | 5.60785 | 737 | 53 |
| 16.70 | 5.30353 | 445 | 32 |
| 17.11 | 5.17788 | 231 | 17 |
| 17.44 | 5.08208 | 328 | 24 |
| 17.72 | 5.00006 | 1026 | 74 |
| 18.14 | 4.887 | 755 | 55 |
| 18.42 | 4.81251 | 1157 | 84 |
| 19.69 | 4.50542 | 108 | 8 |
| 20.06 | 4.42329 | 788 | 57 |
| 20.57 | 4.31465 | 534 | 39 |
| 21.17 | 4.19318 | 482 | 35 |
| 21.93 | 4.04945 | 562 | 41 |
| 22.60 | 3.93034 | 270 | 20 |
| 23.27 | 3.81985 | 1220 | 88 |
| 24.40 | 3.64441 | 459 | 33 |
| 24.72 | 3.59863 | 94 | 7 |
| 25.46 | 3.49626 | 606 | 44 |
| 25.77 | 3.45466 | 303 | 22 |
| 26.23 | 3.39484 | 154 | 11 |
| 26.94 | 3.30691 | 258 | 19 |
| 27.83 | 3.20304 | 514 | 37 |
| 28.26 | 3.15557 | 390 | 28 |
| 28.52 | 3.1267 | 320 | 23 |
| 28.87 | 3.08971 | 307 | 22 |
| 29.85 | 2.99094 | 248 | 18 |

**Table 16. XRPD Peak positions of Form U**

| 2-θ | d (A°) | Height (counts) | Relative Intensity, % |
|---|---|---|---|
| 5.79 | 15.25652 | 539 | 34 |
| 8.43 | 10.4795 | 1496 | 94 |
| 9.62 | 9.18534 | 167 | 10 |
| 10.38 | 8.51392 | 205 | 13 |
| 11.36 | 7.78383 | 1264 | 79 |
| 11.62 | 7.61171 | 1590 | 100 |
| 12.93 | 6.8429 | 214 | 13 |
| 13.68 | 6.46712 | 219 | 14 |
| 14.47 | 6.1181 | 547 | 34 |
| 15.59 | 5.67796 | 299 | 19 |
| 16.46 | 5.38212 | 156 | 10 |
| 17.01 | 5.20821 | 152 | 10 |
| 17.40 | 5.09199 | 134 | 8 |
| 18.07 | 4.90473 | 107 | 7 |
| 18.45 | 4.80391 | 51 | 3 |
| 18.91 | 4.6903 | 562 | 35 |
| 19.33 | 4.58723 | 89 | 6 |
| 20.50 | 4.32989 | 83 | 5 |
| 21.08 | 4.21039 | 702 | 44 |
| 21.56 | 4.11765 | 757 | 48 |
| 22.49 | 3.94958 | 80 | 5 |
| 23.21 | 3.82911 | 96 | 6 |
| 23.60 | 3.76677 | 216 | 14 |
| 23.97 | 3.71009 | 381 | 24 |
| 25.02 | 3.55676 | 57 | 4 |
| 25.49 | 3.49201 | 408 | 26 |
| 26.01 | 3.42279 | 275 | 17 |
| 26.74 | 3.33167 | 112 | 7 |
| 27.22 | 3.27325 | 82 | 5 |
| 27.47 | 3.24404 | 102 | 6 |
| 29.21 | 3.05524 | 166 | 10 |

**Table 17. XRPD Peak positions of Form V**

| 2-θ | d (A°) | Height (counts) | Relative Intensity, % |
|---|---|---|---|
| 6.35 | 13.90226 | 263 | 32 |
| 7.33 | 12.05205 | 199 | 24 |
| 7.98 | 11.07194 | 69 | 8 |
| 8.21 | 10.76694 | 42 | 5 |
| 8.73 | 10.12354 | 67 | 8 |
| 10.56 | 8.37353 | 833 | 100 |
| 11.21 | 7.88582 | 166 | 20 |
| 11.83 | 7.47628 | 45 | 5 |
| 12.31 | 7.18552 | 214 | 26 |
| 13.44 | 6.58433 | 62 | 7 |
| 14.37 | 6.15866 | 214 | 26 |
| 14.68 | 6.02788 | 211 | 25 |
| 15.65 | 5.65959 | 683 | 82 |
| 16.51 | 5.36422 | 263 | 32 |
| 16.83 | 5.26498 | 271 | 33 |
| 17.68 | 5.0125 | 205 | 25 |
| 18.34 | 4.83358 | 291 | 35 |
| 18.74 | 4.73121 | 197 | 24 |
| 20.06 | 4.42375 | 160 | 19 |
| 21.10 | 4.20759 | 161 | 19 |
| 21.89 | 4.05761 | 167 | 20 |
| 22.27 | 3.98886 | 58 | 7 |
| 22.65 | 3.92216 | 96 | 12 |
| 23.18 | 3.83439 | 114 | 14 |
| 23.68 | 3.75429 | 189 | 23 |
| 24.70 | 3.60133 | 223 | 27 |
| 25.43 | 3.49941 | 148 | 18 |

**Table 18. XRPD Peak positions of Form W**

| 2-θ | d (A°) | Height (counts) | Relative Intensity, % |
|---|---|---|---|
| 10.06 | 8.78727 | 435 | 39 |
| 10.38 | 8.51351 | 871 | 78 |
| 10.67 | 8.28753 | 425 | 38 |
| 11.08 | 7.98164 | 129 | 11 |
| 11.35 | 7.78861 | 168 | 15 |
| 11.74 | 7.53486 | 360 | 32 |
| 11.89 | 7.4355 | 202 | 18 |
| 12.50 | 7.0739 | 177 | 16 |
| 13.90 | 6.36748 | 1002 | 89 |
| 14.69 | 6.02387 | 145 | 13 |
| 16.24 | 5.45326 | 195 | 17 |
| 16.34 | 5.42066 | 156 | 14 |
| 17.06 | 5.19266 | 278 | 25 |
| 18.39 | 4.82085 | 93 | 8 |
| 18.69 | 4.74351 | 114 | 10 |
| 18.91 | 4.68901 | 121 | 11 |
| 21.98 | 4.04154 | 163 | 15 |
| 22.51 | 3.94719 | 102 | 9 |
| 23.79 | 3.73785 | 154 | 14 |
| 24.43 | 3.64088 | 1123 | 100 |
| 25.90 | 3.43783 | 88 | 8 |
| 27.56 | 3.23416 | 157 | 14 |
| 27.95 | 3.18928 | 249 | 22 |
| 28.54 | 3.12464 | 183 | 16 |
| 29.48 | 3.02756 | 631 | 56 |

**Table 19. XRPD Peak positions of Form X**

| 2-θ | d (A°) | Height (counts) | Relative Intensity, % |
|---|---|---|---|
| 6.57 | 13.43846 | 567 | 25 |
| 8.20 | 10.77946 | 923 | 41 |
| 9.66 | 9.14872 | 1358 | 60 |
| 10.17 | 8.69028 | 2260 | 100 |
| 10.50 | 8.41944 | 1741 | 77 |
| 11.17 | 7.9177 | 694 | 31 |
| 11.66 | 7.58578 | 209 | 9 |
| 13.73 | 6.44445 | 126 | 6 |
| 15.72 | 5.63451 | 274 | 12 |
| 16.39 | 5.40323 | 572 | 25 |
| 17.65 | 5.02051 | 576 | 26 |
| 18.32 | 4.83943 | 416 | 18 |
| 18.67 | 4.74992 | 1124 | 50 |
| 18.98 | 4.67231 | 575 | 25 |
| 19.57 | 4.53358 | 277 | 12 |
| 21.92 | 4.05128 | 118 | 5 |
| 22.21 | 3.99855 | 259 | 11 |
| 22.60 | 3.93073 | 629 | 28 |
| 22.88 | 3.88323 | 383 | 17 |
| 23.36 | 3.80557 | 145 | 6 |
| 23.56 | 3.77329 | 346 | 15 |
| 24.65 | 3.60914 | 1075 | 48 |
| 26.79 | 3.32498 | 119 | 5 |
| 27.43 | 3.24868 | 116 | 5 |

**Table 20. XRPD Peak positions of Form Y**

| 2-θ | d (A°) | Height (counts) | Relative Intensity, % |
|---|---|---|---|
| 6.51 | 13.56982 | 688 | 100 |
| 8.16 | 10.82987 | 35 | 5 |
| 10.68 | 8.27907 | 77 | 11 |
| 12.96 | 6.82732 | 685 | 100 |
| 13.34 | 6.6317 | 164 | 24 |
| 14.25 | 6.21038 | 48 | 7 |
| 16.31 | 5.43026 | 87 | 13 |
| 17.79 | 4.98166 | 49 | 7 |
| 18.92 | 4.6866 | 78 | 11 |
| 19.47 | 4.55556 | 245 | 36 |
| 22.38 | 3.96985 | 61 | 9 |
| 24.19 | 3.6757 | 172 | 25 |
| 26.02 | 3.42199 | 51 | 7 |
| 28.73 | 3.1048 | 40 | 6 |

**Table 21. XRPD Peak positions of Form Z**

| 2-θ | d (A°) | Height (counts) | Relative Intensity, % |
|---|---|---|---|
| 5.83 | 15.15971 | 78 | 8 |
| 8.20 | 10.76811 | 132 | 13 |
| 8.42 | 10.49873 | 185 | 18 |
| 10.63 | 8.31858 | 1014 | 100 |
| 11.23 | 7.87332 | 261 | 26 |
| 11.55 | 7.65819 | 450 | 44 |
| 11.97 | 7.38713 | 231 | 23 |
| 14.34 | 6.1711 | 250 | 25 |
| 15.59 | 5.67832 | 363 | 36 |
| 16.17 | 5.47742 | 333 | 33 |
| 17.29 | 5.12414 | 18 | 2 |
| 17.64 | 5.02504 | 240 | 24 |
| 18.05 | 4.91146 | 480 | 47 |
| 18.67 | 4.74926 | 333 | 33 |
| 21.56 | 4.11901 | 82 | 8 |
| 22.32 | 3.979 | 159 | 16 |
| 22.85 | 3.88914 | 100 | 10 |
| 23.57 | 3.77129 | 79 | 8 |
| 24.11 | 3.68778 | 411 | 40 |
| 24.31 | 3.65902 | 588 | 58 |
| 24.62 | 3.61317 | 174 | 17 |
| 25.32 | 3.51459 | 47 | 5 |
| 26.72 | 3.33401 | 86 | 9 |
| 27.59 | 3.23007 | 56 | 6 |
| 28.73 | 3.1048 | 148 | 15 |
| 29.17 | 3.05883 | 80 | 8 |

**Table 22. XRPD Peak positions of Form α**

| 2-θ | d (A°) | Height (counts) | Relative Intensity, % |
|---|---|---|---|
| 7.26 | 12.16109 | 356 | 100 |
| 10.06 | 8.78569 | 326 | 92 |
| 10.36 | 8.53276 | 289 | 81 |
| 10.62 | 8.32671 | 236 | 66 |
| 11.23 | 7.87415 | 95 | 27 |
| 11.90 | 7.42931 | 149 | 42 |
| 13.85 | 6.3878 | 222 | 62 |
| 15.79 | 5.60772 | 37 | 10 |
| 16.46 | 5.38026 | 134 | 38 |
| 17.31 | 5.11738 | 80 | 22 |
| 21.91 | 4.05326 | 109 | 31 |
| 22.43 | 3.96018 | 121 | 34 |
| 24.12 | 3.68699 | 135 | 38 |
| 27.87 | 3.19903 | 36 | 10 |
| 29.39 | 3.03621 | 74 | 21 |

**Table 23. XRPD Peak positions of Form β**

| 2-θ | d (A°) | Height (counts) | Relative Intensity, % |
|---|---|---|---|
| 7.36 | 11.99797 | 1320 | 49 |
| 10.52 | 8.40273 | 2711 | 100 |
| 11.20 | 7.89298 | 415 | 15 |
| 12.35 | 7.15982 | 438 | 16 |
| 14.25 | 6.21004 | 2210 | 82 |
| 14.67 | 6.03184 | 636 | 23 |
| 15.26 | 5.80059 | 297 | 11 |
| 15.73 | 5.62778 | 1766 | 65 |
| 16.72 | 5.29918 | 179 | 7 |
| 17.11 | 5.17935 | 128 | 5 |
| 17.78 | 4.98572 | 288 | 11 |
| 18.07 | 4.90535 | 609 | 22 |
| 18.27 | 4.85076 | 2030 | 75 |
| 19.50 | 4.54843 | 164 | 6 |
| 20.11 | 4.41112 | 753 | 28 |
| 20.43 | 4.34446 | 967 | 36 |
| 21.00 | 4.2262 | 1300 | 48 |
| 21.75 | 4.08293 | 822 | 30 |
| 22.04 | 4.02993 | 393 | 15 |
| 22.49 | 3.95055 | 216 | 8 |
| 23.16 | 3.83754 | 1882 | 69 |
| 23.59 | 3.76772 | 159 | 6 |
| 24.49 | 3.63263 | 164 | 6 |
| 25.45 | 3.49699 | 449 | 17 |
| 26.35 | 3.37971 | 124 | 5 |
| 26.76 | 3.32885 | 670 | 25 |
| 27.78 | 3.209 | 280 | 10 |
| 28.02 | 3.18207 | 322 | 12 |
| 28.32 | 3.14845 | 534 | 20 |
| 28.61 | 3.1179 | 440 | 16 |
| 29.13 | 3.06289 | 126 | 5 |
| 29.50 | 3.02506 | 620 | 23 |

**Table 24. XRPD Peak positions of Form χ**

| 2-θ | d (A°) | Height (counts) | Relative Intensity, % |
|---|---|---|---|
| 8.53 | 10.36124 | 434 | 22 |
| 11.16 | 7.92161 | 1937 | 100 |
| 16.72 | 5.29732 | 132 | 7 |
| 17.11 | 5.17685 | 134 | 7 |
| 18.38 | 4.82347 | 408 | 21 |
| 19.16 | 4.62832 | 152 | 8 |
| 20.14 | 4.40478 | 306 | 16 |
| 21.19 | 4.18862 | 221 | 11 |
| 21.38 | 4.15259 | 360 | 19 |
| 21.52 | 4.12508 | 228 | 12 |
| 22.41 | 3.96478 | 242 | 12 |
| 23.15 | 3.83851 | 92 | 5 |
| 24.18 | 3.67732 | 254 | 13 |
| 25.91 | 3.4365 | 257 | 13 |
| 28.79 | 3.09877 | 114 | 6 |

**Table 25. XRPD Peak positions of Form δ**

| 2-θ | d (A°) | Height (counts) | Relative Intensity, % |
|---|---|---|---|
| 10.90 | 8.11024 | 290 | 100 |
| 12.14 | 7.28726 | 105 | 36 |
| 13.22 | 6.68946 | 41 | 14 |
| 14.44 | 6.12953 | 124 | 43 |
| 14.70 | 6.02007 | 35 | 12 |
| 17.39 | 5.09676 | 53 | 18 |
| 18.14 | 4.886 | 68 | 24 |
| 19.55 | 4.5377 | 63 | 22 |
| 20.21 | 4.38972 | 18 | 6 |
| 22.66 | 3.92145 | 42 | 14 |
| 24.48 | 3.63399 | 215 | 74 |
| 26.98 | 3.3026 | 87 | 30 |

**Table 26. XRPD Peak positions of a mixture of Form C and a possible acetone solvate (Form ε)**

| 2-θ | d (A°) | Height (counts) | Relative Intensity, % |
|---|---|---|---|
| 5.73 | 15.41658 | 368 | 27 |
| 10.33 | 8.55984 | 128 | 10 |
| 11.41 | 7.75225 | 1348 | 100 |
| 11.67 | 7.57375 | 67 | 5 |
| 11.81 | 7.48709 | 87 | 6 |
| 15.39 | 5.75169 | 121 | 9 |
| 15.82 | 5.59883 | 75 | 6 |
| 16.60 | 5.33454 | 445 | 33 |
| 17.11 | 5.17708 | 115 | 9 |
| 17.64 | 5.02327 | 316 | 23 |
| 19.23 | 4.61134 | 109 | 8 |
| 19.62 | 4.52189 | 88 | 6 |
| 21.36 | 4.15655 | 161 | 12 |
| 21.84 | 4.06688 | 125 | 9 |
| 22.42 | 3.96186 | 87 | 6 |
| 22.88 | 3.88362 | 77 | 6 |
| 23.18 | 3.83343 | 263 | 19 |
| 23.60 | 3.76748 | 76 | 6 |
| 24.22 | 3.67188 | 249 | 18 |
| 25.74 | 3.45803 | 69 | 5 |
| 26.24 | 3.39337 | 111 | 8 |
| 28.74 | 3.10357 | 76 | 6 |

**Table 27. XRPD Peak positions of Form ϕ**

| 2-θ | d (A°) | Height (counts) | Relative Intensity, % |
|---|---|---|---|
| 6.95 | 12.69992 | 851 | 14 |
| 10.76 | 8.21266 | 220 | 4 |
| 11.06 | 7.99275 | 293 | 5 |
| 13.87 | 6.37839 | 1447 | 24 |
| 16.05 | 5.51793 | 346 | 6 |
| 16.48 | 5.3753 | 568 | 10 |
| 20.85 | 4.25639 | 5936 | 100 |
| 22.33 | 3.97807 | 944 | 16 |
| 22.71 | 3.91233 | 330 | 6 |
| 27.92 | 3.19264 | 1800 | 30 |
| 29.00 | 3.07696 | 627 | 11 |

**Table 28. XRPD Peak positions of Form η**

| 2-θ | d (A°) | Height (counts) | Relative Intensity, % |
|---|---|---|---|
| 6.87 | 12.85507 | 192 | 69 |
| 7.69 | 11.48644 | 209 | 75 |
| 12.06 | 7.32999 | 31 | 11 |
| 14.30 | 6.19077 | 65 | 23 |
| 16.32 | 5.42733 | 38 | 13 |
| 16.91 | 5.23944 | 76 | 27 |
| 18.17 | 4.87733 | 77 | 28 |
| 20.45 | 4.33961 | 220 | 79 |
| 21.08 | 4.21201 | 51 | 18 |
| 22.99 | 3.86525 | 279 | 100 |
| 23.85 | 3.72784 | 114 | 41 |
| 24.77 | 3.59205 | 49 | 18 |
| 25.46 | 3.49531 | 40 | 14 |
| 25.99 | 3.42553 | 34 | 12 |
| 26.56 | 3.35323 | 21 | 7 |
| 27.49 | 3.24178 | 49 | 18 |
| 28.17 | 3.16482 | 92 | 33 |
| 29.21 | 3.05472 | 31 | 11 |

**Table 29. XRPD Peak positions of Form λ,**

| 2-θ | d (A°) | Height (counts) | Relative Intensity, % |
|---|---|---|---|
| 8.85 | 9.97936 | 57 | 6 |
| 10.64 | 8.30722 | 834 | 94 |
| 11.24 | 7.86653 | 227 | 26 |
| 12.01 | 7.36601 | 291 | 33 |
| 14.33 | 6.1747 | 271 | 30 |
| 15.59 | 5.67972 | 261 | 29 |
| 16.20 | 5.46571 | 704 | 79 |
| 17.31 | 5.11846 | 206 | 23 |
| 17.60 | 5.03393 | 333 | 37 |
| 18.01 | 4.92036 | 528 | 59 |
| 19.56 | 4.53532 | 82 | 9 |
| 20.10 | 4.41393 | 151 | 17 |
| 22.34 | 3.97569 | 262 | 29 |
| 22.81 | 3.89618 | 50 | 6 |
| 24.26 | 3.66562 | 889 | 100 |
| 26.78 | 3.32625 | 117 | 13 |
| 27.68 | 3.22066 | 76 | 9 |
| 28.75 | 3.10234 | 183 | 21 |

**Table 30. XRPD Peak positions of the glutaric acid cocrystal**

| 2-θ | d (A°) | Height (counts) | Relative Intensity, % |
|---|---|---|---|
| 8.15 | 10.84284 | 194 | 7 |
| 9.74 | 9.07047 | 1061 | 39 |
| 10.78 | 8.1976 | 1982 | 74 |
| 11.04 | 8.00901 | 1835 | 68 |
| 12.17 | 7.26756 | 1645 | 61 |
| 14.59 | 6.06438 | 312 | 12 |
| 16.06 | 5.51456 | 1314 | 49 |
| 17.02 | 5.20511 | 799 | 30 |
| 17.62 | 5.03029 | 492 | 18 |
| 18.71 | 4.73919 | 326 | 12 |
| 19.15 | 4.63132 | 934 | 35 |
| 19.61 | 4.52359 | 389 | 14 |
| 21.47 | 4.13454 | 468 | 17 |
| 21.86 | 4.06253 | 1453 | 54 |
| 23.06 | 3.85374 | 2689 | 100 |
| 24.51 | 3.62834 | 470 | 17 |
| 25.13 | 3.54038 | 267 | 10 |
| 26.63 | 3.34464 | 216 | 8 |
| 27.10 | 3.28755 | 407 | 15 |
| 27.43 | 3.24858 | 267 | 10 |
| 27.99 | 3.1849 | 151 | 6 |
| 29.18 | 3.05837 | 321 | 12 |
| 32.84 | 2.72493 | 360 | 13 |
| 33.50 | 2.67298 | 206 | 8 |
| 37.17 | 2.41685 | 125 | 5 |

**Table 31. XRPD Peak positions of the Calcium salt of Compound A from the experiment L100110-68-1 (Form 1-A)**

| 2-θ | d (A°) | Height (counts) | Relative Intensity, % |
|---|---|---|---|
| 4.55 | 19.41373 | 1603 | 100 |
| 6.53 | 13.5336 | 987 | 62 |
| 7.25 | 12.18703 | 414 | 26 |
| 8.52 | 10.36821 | 324 | 20 |
| 23.37 | 3.80366 | 105 | 7 |
| 25.00 | 3.5593 | 121 | 8 |
| 29.51 | 3.02493 | 199 | 12 |

**Table 32. XRPD Peak positions of the Calcium salt of Compound A from the experiment L100110-68-3-Wet (Form 1-B)**

| 2-θ | d (A°) | Height (counts) | Relative Intensity, % |
|---|---|---|---|
| 4.02 | 21.95006 | 2299 | 100 |
| 6.99 | 12.64126 | 96 | 4 |
| 7.88 | 11.21031 | 473 | 21 |
| 8.73 | 10.12473 | 92 | 4 |
| 15.86 | 5.58166 | 95 | 4 |

**Table 33. XRPD Peak positions of the Calcium salt of Compound A from the experiment L100110-68-8 (Form 2-B)**

| 2-θ | d (A°) | Height (counts) | Relative Intensity, % |
|---|---|---|---|
| 5.94 | 14.87269 | 304 | 99 |
| 6.90 | 12.80769 | 162 | 53 |
| 7.79 | 11.3457 | 307 | 100 |
| 9.57 | 9.23139 | 19 | 6 |
| 11.98 | 7.38409 | 53 | 17 |
| 13.57 | 6.52017 | 24 | 8 |
| 14.19 | 6.2359 | 19 | 6 |
| 20.38 | 4.35414 | 37 | 12 |
| 25.25 | 3.52448 | 21 | 7 |

**Table 34. XRPD Peak positions of the Calcium salt of Compound A from the experiment L100110-68-10 after exposing to saturated humidity environment (Form 2-D)**

| 2-θ | d (A°) | Height (counts) | Relative Intensity, % |
|---|---|---|---|
| 4.51 | 19.57407 | 76 | 13 |
| 5.76 | 15.33611 | 118 | 21 |
| 7.44 | 11.87012 | 38 | 7 |
| 8.68 | 10.18068 | 496 | 88 |
| 10.79 | 8.1896 | 62 | 11 |
| 11.38 | 7.76999 | 241 | 43 |
| 14.12 | 6.26516 | 150 | 27 |
| 16.77 | 5.28335 | 563 | 100 |
| 18.57 | 4.7754 | 27 | 5 |
| 20.60 | 4.30778 | 38 | 7 |
| 21.61 | 4.1095 | 29 | 5 |
| 23.41 | 3.79764 | 325 | 58 |
| 26.29 | 3.38736 | 83 | 15 |
| 28.29 | 3.15205 | 136 | 24 |
| 29.50 | 3.02559 | 272 | 48 |

**Table 35. XRPD Peak positions of the Magnesium salt of Compound A from the experiment L100110-68-11-Dry (3-A).**

| 2-θ | d (A°) | Height (counts) | Relative Intensity, % |
|---|---|---|---|
| 4.06 | 21.73785 | 9654 | 100 |
| 8.04 | 10.98764 | 1168 | 12 |
| 8.80 | 10.03642 | 1012 | 10 |
| 9.27 | 9.52989 | 2027 | 21 |
| 11.00 | 8.03807 | 898 | 9 |
| 15.80 | 5.60268 | 1284 | 13 |
| 18.01 | 4.9209 | 740 | 8 |
| 18.64 | 4.75617 | 1288 | 13 |
| 19.37 | 4.5788 | 722 | 7 |
| 22.02 | 4.03289 | 1852 | 19 |
| 23.39 | 3.79958 | 899 | 9 |
| 38.06 | 2.36248 | 1271 | 13 |

**Table 36. XRPD Peak positions of the Magnesium salt of Compound A from the experiment L100110-68-11 upon exposing it to saturated humidity environment at room temperature**

| 2-θ | d (A°) | Height (counts) | Relative Intensity, % |
|---|---|---|---|
| 5.69 | 15.51348 | 470 | 31 |
| 8.87 | 9.96636 | 1541 | 100 |
| 10.87 | 8.132 | 244 | 16 |
| 11.46 | 7.71218 | 543 | 35 |
| 13.20 | 6.70116 | 245 | 16 |
| 14.24 | 6.21326 | 139 | 9 |
| 16.55 | 5.35354 | 291 | 19 |
| 18.60 | 4.76784 | 468 | 30 |
| 20.55 | 4.31771 | 74 | 5 |
| 21.85 | 4.06444 | 224 | 15 |
| 23.62 | 3.76347 | 299 | 19 |
| 24.41 | 3.64333 | 138 | 9 |
| 26.35 | 3.37926 | 301 | 20 |
| 27.76 | 3.21078 | 187 | 12 |
| 28.59 | 3.11989 | 112 | 7 |

**Table 37. XRPD Peak positions of the Magnesium salt of Compound A from the experiment L100110-68-13 before drying (3-B).**

| 2-θ | d (A°) | Height (counts) | Relative Intensity, % |
|---|---|---|---|
| 5.47 | 16.13545 | 131 | 25 |
| 6.93 | 12.73634 | 496 | 96 |
| 9.11 | 9.70297 | 30 | 6 |
| 10.72 | 8.24296 | 519 | 100 |
| 13.01 | 6.79699 | 32 | 6 |
| 14.92 | 5.93135 | 45 | 9 |
| 15.19 | 5.82791 | 62 | 12 |
| 16.08 | 5.50592 | 257 | 50 |
| 18.67 | 4.7492 | 302 | 58 |
| 20.63 | 4.30185 | 25 | 5 |
| 21.43 | 4.143 | 86 | 17 |

**Table 38. XRPD Peak positions of the Magnesium salt of Compound A from the experiment L100110-68-13, dried after deliquescing (3-D).**

| 2-θ | d (A°) | Height (counts) | Relative Intensity, % |
|---|---|---|---|
| 9.63 | 9.17326 | 682 | 100 |
| 10.75 | 8.22179 | 85 | 12 |
| 13.70 | 6.46016 | 118 | 17 |
| 15.70 | 5.63949 | 31 | 5 |
| 17.16 | 5.16179 | 54 | 8 |
| 18.63 | 4.75863 | 217 | 32 |
| 19.75 | 4.49151 | 73 | 11 |
| 21.70 | 4.09288 | 280 | 41 |
| 22.23 | 3.99595 | 32 | 5 |
| 26.27 | 3.38972 | 195 | 29 |

**Table 39. XRPD Peak positions of the Magnesium salt of Compound A from the experiment L100110-68-17, before drying (Form 4-B).**

| 2-θ | d (A°) | Height (counts) | Relative Intensity, % |
|---|---|---|---|
| 5.13 | 17.20435 | 705 | 100 |
| 6.04 | 14.6144 | 59 | 8 |
| 10.04 | 8.80367 | 213 | 30 |
| 15.42 | 5.74009 | 68 | 10 |
| 18.32 | 4.8385 | 43 | 6 |
| 27.44 | 3.24795 | 156 | 22 |

**Table 40. XRPD Peak positions of the Magnesium salt of Compound A from the experiment L100110-68-20, before drying (Form 4-D).**

| 2-θ | d (A°) | Height (counts) | Relative Intensity, % |
|---|---|---|---|
| 9.97 | 8.86232 | 20 | 10 |
| 11.08 | 7.97695 | 87 | 45 |
| 16.54 | 5.35375 | 76 | 39 |
| 18.57 | 4.7754 | 20 | 10 |
| 22.45 | 3.95625 | 67 | 34 |
| 27.48 | 3.2426 | 196 | 100 |

**Table 41. XRPD Peak positions of the Magnesium salt of Compound A from the experiment L100110-68-20, after drying (Form 4-E).**

| 2-θ | d (A°) | Height (counts) | Relative Intensity, % |
|---|---|---|---|
| 8.92 | 9.90545 | 140 | 54 |
| 10.59 | 8.35011 | 258 | 100 |
| 15.33 | 5.77434 | 101 | 39 |
| 15.88 | 5.57481 | 47 | 18 |
| 17.79 | 4.98152 | 116 | 45 |
| 20.45 | 4.33966 | 38 | 15 |
| 21.73 | 4.08729 | 170 | 66 |
| 27.39 | 3.25357 | 128 | 50 |
| 28.23 | 3.15834 | 29 | 11 |

**Table 42. XRPD Peak positions of the Sodium salt of Compound A from the experiment L100110-68-21 (Form 5-A).**

| 2-θ | d (A°) | Height (counts) | Relative Intensity, % |
|---|---|---|---|
| 4.04 | 21.82961 | 811 | 100 |
| 8.02 | 11.01682 | 125 | 15 |
| 10.11 | 8.74579 | 168 | 21 |

**Table 43. XRPD Peak positions of the Sodium salt of Compound A from the experiment L100110-68-24 (Form 5-B).**

| 2-θ | d (A°) | Height (counts) | Relative Intensity, % |
|---|---|---|---|
| 4.06 | 21.73785 | 474 | 65 |
| 7.76 | 11.38701 | 577 | 79 |
| 10.33 | 8.55811 | 733 | 100 |
| 20.83 | 4.26167 | 114 | 16 |
| 25.83 | 3.44676 | 56 | 8 |

**Table 44. XRPD Peak positions of the Sodium salt of Compound A from the experiment L100110-68-25 (Form 5-C).**

| 2-θ | d (A°) | Height (counts) | Relative Intensity, % |
|---|---|---|---|
| 5.72 | 15.42652 | 275 | 61 |
| 6.48 | 13.63258 | 448 | 100 |
| 8.49 | 10.40558 | 208 | 46 |

**Table 45. XRPD Peak positions of the Sodium salt of Compound A from the experiment L100110-68-25 after humidity exposure that resulted in substantial improvement in crystallinity (Form 5-D).**

| 2-θ | d (A°) | Height (counts) | Relative Intensity, % |
|---|---|---|---|
| 5.15 | 17.15855 | 10385 | 53 |
| 7.30 | 12.09301 | 19470 | 100 |
| 10.22 | 8.64785 | 1577 | 8 |
| 14.57 | 6.07274 | 8966 | 46 |
| 17.02 | 5.20512 | 2386 | 12 |
| 20.50 | 4.32901 | 3157 | 16 |
| 21.90 | 4.05459 | 2197 | 11 |

**Table 46. XRPD Peak positions of the Potassium salt of Compound A from the experiment L100110-68-26 after drying (Form 6-A).**

| 2-θ | d (A°) | Height (counts) | Relative Intensity, % |
|---|---|---|---|
| 4.13 | 21.36074 | 1068 | 100 |
| 8.42 | 10.49468 | 47 | 4 |
| 11.97 | 7.38706 | 145 | 14 |

**Table 47. XRPD Peak positions of the Potassium salt of Compound A from the experiment L100110-68-29 (Form 6-B).**

| 2-θ | d (A°) | Height (counts) | Relative Intensity, % |
|---|---|---|---|
| 4.15 | 21.25539 | 1912 | 55 |
| 6.73 | 13.11434 | 3466 | 100 |
| 9.54 | 9.26631 | 133 | 4 |
| 11.90 | 7.43037 | 150 | 4 |
| 17.83 | 4.96945 | 257 | 7 |
| 21.48 | 4.13371 | 129 | 4 |
| 31.42 | 2.84461 | 229 | 7 |

**Table 48. XRPD Peak positions of the Potassium salt of Compound A from the experiment L100110-68-30 after drying (Form 6-C).**

| 2-θ | d (A°) | Height (counts) | Relative Intensity, % |
|---|---|---|---|
| 8.24 | 10.71704 | 441 | 56 |
| 10.91 | 8.10348 | 299 | 38 |
| 22.17 | 4.00659 | 788 | 100 |
| 30.78 | 2.9028 | 47 | 6 |
| 38.86 | 2.31546 | 65 | 8 |

**Table 49. XRPD Peak positions of the Potassium salt of Compound A from the experiment L100110-68-30-H (Form 6-D).**

| 2-θ | d (A°) | Height (counts) | Relative Intensity, % |
|---|---|---|---|
| 8.91 | 9.9141 | 789 | 60 |
| 11.81 | 7.4864 | 258 | 20 |
| 14.09 | 6.28163 | 103 | 8 |
| 15.75 | 5.62047 | 163 | 12 |
| 17.30 | 5.12046 | 117 | 9 |
| 19.96 | 4.44385 | 134 | 10 |
| 21.98 | 4.03979 | 1312 | 100 |
| 23.22 | 3.82704 | 92 | 7 |
| 24.18 | 3.67732 | 190 | 15 |
| 25.92 | 3.43477 | 66 | 5 |
| 27.09 | 3.28884 | 95 | 7 |
| 28.46 | 3.13404 | 154 | 12 |

**Table 50. XRPD Peak positions of the Ethanolamine salt of Compound A from the experiment L100110-68-31 (Form 7-A).**

| 2-θ | d (A°) | Height (counts) | Relative Intensity, % |
|---|---|---|---|
| 5.04 | 17.51318 | 380 | 10 |
| 8.17 | 10.80774 | 1193 | 32 |
| 10.09 | 8.75893 | 3735 | 100 |
| 11.04 | 8.00809 | 1024 | 27 |
| 13.82 | 6.40101 | 279 | 7 |
| 14.94 | 5.92583 | 202 | 5 |
| 16.40 | 5.39914 | 429 | 11 |
| 18.61 | 4.76291 | 1020 | 27 |
| 19.86 | 4.46687 | 200 | 5 |
| 22.69 | 3.91561 | 732 | 20 |
| 23.49 | 3.78349 | 2 | 0 |
| 25.29 | 3.51929 | 153 | 4 |
| 25.59 | 3.47759 | 264 | 7 |
| 26.88 | 3.31475 | 281 | 8 |
| 27.76 | 3.21134 | 629 | 17 |
| 28.05 | 3.17894 | 135 | 4 |

**Table 51. XRPD Peak positions of the Ethanolamine salt of Compound A from the experiment L100110-68-32 after drying (Form 7-B).**

| 2-θ | d (A°) | Height (counts) | Relative Intensity, % |
|---|---|---|---|
| 8.41 | 10.50243 | 8644 | 94 |
| 10.21 | 8.65795 | 9198 | 100 |
| 13.15 | 6.72755 | 1713 | 19 |
| 16.31 | 5.42927 | 671 | 7 |
| 16.83 | 5.26504 | 3247 | 35 |
| 18.93 | 4.68344 | 605 | 7 |
| 20.32 | 4.36721 | 1177 | 13 |
| 20.68 | 4.29139 | 679 | 7 |
| 21.16 | 4.19515 | 573 | 6 |
| 24.61 | 3.61417 | 1034 | 11 |
| 25.04 | 3.55349 | 3272 | 36 |
| 26.03 | 3.41984 | 1693 | 18 |
| 27.74 | 3.21305 | 1965 | 21 |
| 29.24 | 3.0519 | 535 | 6 |
| 29.89 | 2.98695 | 909 | 10 |
| 34.12 | 2.62598 | 1233 | 13 |
| 35.63 | 2.51757 | 389 | 4 |

**Table 52. XRPD Peak positions of the Diethanolamine salt of Compound A from the experiment L100110-68-36 (Form 8-A).**

| 2-θ | d (A°) | Height (counts) | Relative Intensity, % |
|---|---|---|---|
| 6.62 | 13.34209 | 1970 | 100 |
| 7.99 | 11.0497 | 418 | 21 |
| 11.86 | 7.45418 | 1593 | 81 |
| 12.52 | 7.06306 | 207 | 11 |
| 14.14 | 6.25715 | 579 | 29 |
| 15.17 | 5.83499 | 86 | 4 |
| 16.06 | 5.51371 | 134 | 7 |
| 18.72 | 4.73628 | 122 | 6 |
| 19.29 | 4.59738 | 72 | 4 |
| 19.94 | 4.44815 | 226 | 12 |
| 21.27 | 4.17306 | 253 | 13 |
| 21.69 | 4.09351 | 507 | 26 |
| 22.15 | 4.00999 | 371 | 19 |
| 22.85 | 3.88855 | 653 | 33 |
| 23.65 | 3.75859 | 177 | 9 |
| 24.57 | 3.6204 | 701 | 36 |
| 25.65 | 3.46998 | 353 | 18 |
| 26.12 | 3.4093 | 484 | 25 |
| 28.47 | 3.13287 | 306 | 16 |

**Table 53. XRPD Peak positions of the Diethanolamine salt of Compound A from the experiment L100110-68-38 (Form 8-B).**

| 2-θ | d (A°) | Height (counts) | Relative Intensity, % |
|---|---|---|---|
| 6.80 | 12.98593 | 10954 | 100 |
| 9.60 | 9.20224 | 8979 | 82 |
| 11.35 | 7.78675 | 1374 | 13 |
| 13.18 | 6.71147 | 4006 | 37 |
| 16.36 | 5.41348 | 2290 | 21 |
| 17.99 | 4.92574 | 1324 | 12 |
| 18.20 | 4.87048 | 400 | 4 |
| 18.81 | 4.71456 | 544 | 5 |
| 19.20 | 4.61866 | 4211 | 38 |
| 20.14 | 4.40634 | 2275 | 21 |
| 20.44 | 4.34166 | 4914 | 45 |
| 21.38 | 4.15282 | 9437 | 86 |
| 22.78 | 3.90121 | 2427 | 22 |
| 24.42 | 3.64182 | 5115 | 47 |
| 24.83 | 3.58325 | 853 | 8 |
| 25.69 | 3.46464 | 909 | 8 |
| 26.64 | 3.34358 | 1757 | 16 |
| 27.10 | 3.2873 | 2199 | 20 |
| 27.74 | 3.21354 | 3515 | 32 |
| 28.91 | 3.08603 | 1134 | 10 |
| 29.15 | 3.0608 | 1739 | 16 |
| 29.56 | 3.01962 | 411 | 4 |
| 30.38 | 2.93984 | 908 | 8 |
| 30.89 | 2.89233 | 1139 | 10 |
| 31.61 | 2.82802 | 396 | 4 |
| 33.03 | 2.70994 | 837 | 8 |
| 34.12 | 2.62559 | 437 | 4 |
| 34.96 | 2.56427 | 466 | 4 |
| 36.59 | 2.45401 | 462 | 4 |

**Table 54. XRPD Peak positions of the Diethanolamine salt of Compound A from the experiment L100110-68-36 after subjecting to saturated humidity environment at RT (Form 8-C).**

| 2-θ | d (A°) | Height (counts) | Relative Intensity, % |
|---|---|---|---|
| 4.91 | 17.97329 | 564 | 30 |
| 5.09 | 17.35412 | 1877 | 100 |
| 6.51 | 13.5755 | 411 | 22 |
| 7.67 | 11.51328 | 144 | 8 |
| 7.91 | 11.17124 | 76 | 4 |
| 8.50 | 10.39291 | 193 | 10 |
| 8.95 | 9.87429 | 975 | 52 |
| 9.73 | 9.07873 | 1204 | 64 |
| 10.12 | 8.73284 | 1222 | 65 |
| 11.91 | 7.4269 | 236 | 13 |
| 15.30 | 5.78658 | 1091 | 58 |
| 17.75 | 4.99325 | 123 | 7 |
| 18.27 | 4.85216 | 112 | 6 |
| 19.25 | 4.60735 | 117 | 6 |
| 20.50 | 4.32805 | 171 | 9 |
| 21.17 | 4.19262 | 236 | 13 |
| 21.57 | 4.11737 | 190 | 10 |
| 23.00 | 3.86345 | 882 | 47 |
| 23.50 | 3.78192 | 330 | 18 |
| 25.66 | 3.46911 | 132 | 7 |
| 26.14 | 3.40577 | 149 | 8 |
| 29.60 | 3.01547 | 79 | 4 |

**Table 55. XRPD Peak positions of the Diethanolamine salt of Compound A from the experiment L100110-68-40 before drying (Form 8-D).**

| 2-θ | d (A°) | Height (counts) | Relative Intensity, % |
|---|---|---|---|
| 6.75 | 13.08575 | 1872 | 100 |
| 8.01 | 11.02396 | 143 | 8 |
| 12.11 | 7.30222 | 935 | 50 |
| 13.97 | 6.3334 | 341 | 18 |
| 14.46 | 6.12158 | 259 | 14 |
| 18.63 | 4.75847 | 81 | 4 |
| 20.24 | 4.38347 | 214 | 11 |
| 20.97 | 4.23276 | 143 | 8 |
| 21.57 | 4.11681 | 207 | 11 |
| 22.12 | 4.01613 | 209 | 11 |
| 22.64 | 3.92409 | 308 | 16 |
| 24.43 | 3.64133 | 167 | 9 |
| 25.12 | 3.54221 | 213 | 11 |
| 25.98 | 3.42708 | 321 | 17 |
| 28.69 | 3.10878 | 159 | 8 |

**Table 56. XRPD Peak positions of the Diethanolamine salt of Compound A from the experiment L100110-68-40 after drying followed by exposure to saturated humidity environment (Form 8-E) (Essentially Form 8-B with extra peaks).**

| 2-θ | d (A°) | Height (counts) | Relative Intensity, % |
|---|---|---|---|
| 4.88 | 18.1047 | 1278 | 6 |
| 6.84 | 12.91887 | 6782 | 31 |
| 9.62 | 9.1878 | 22046 | 100 |
| 11.38 | 7.76763 | 993 | 5 |
| 18.02 | 4.9176 | 841 | 4 |
| 19.23 | 4.61183 | 4971 | 23 |
| 20.45 | 4.3397 | 5404 | 25 |
| 25.75 | 3.45762 | 1125 | 5 |

**Table 57. XRPD Peak positions of the Triethanolamine salt of Compound A from the experiment L100110-68-42 (Form 9-A).**

| 2-θ | d (A°) | Height (counts) | Relative Intensity, % |
|---|---|---|---|
| 4.03 | 21.90272 | 937 | 15 |
| 7.75 | 11.39109 | 4762 | 76 |
| 10.35 | 8.53952 | 6300 | 100 |
| 10.86 | 8.14284 | 4254 | 68 |
| 11.87 | 7.44941 | 762 | 12 |
| 12.21 | 7.24113 | 1718 | 27 |
| 13.57 | 6.52209 | 321 | 5 |
| 14.53 | 6.08924 | 1901 | 30 |
| 15.47 | 5.72377 | 1291 | 20 |
| 15.81 | 5.60181 | 2918 | 46 |
| 16.68 | 5.3095 | 1210 | 19 |
| 17.11 | 5.17765 | 503 | 8 |
| 17.50 | 5.06374 | 677 | 11 |
| 17.94 | 4.93979 | 891 | 14 |
| 18.49 | 4.79524 | 2588 | 41 |
| 19.67 | 4.51008 | 925 | 15 |
| 20.59 | 4.30963 | 3642 | 58 |
| 21.36 | 4.15644 | 4267 | 68 |
| 21.73 | 4.08589 | 1844 | 29 |
| 22.46 | 3.95474 | 3714 | 59 |
| 22.76 | 3.90369 | 1071 | 17 |
| 23.85 | 3.72826 | 1461 | 23 |
| 24.05 | 3.69751 | 2086 | 33 |
| 24.45 | 3.63748 | 1062 | 17 |
| 26.06 | 3.41707 | 548 | 9 |
| 26.40 | 3.37284 | 1402 | 22 |
| 26.62 | 3.3462 | 1189 | 19 |
| 26.95 | 3.30598 | 915 | 15 |
| 27.35 | 3.2588 | 982 | 16 |
| 28.09 | 3.17414 | 853 | 14 |
| 28.60 | 3.11887 | 351 | 6 |
| 29.29 | 3.04674 | 1189 | 19 |
| 29.73 | 3.00264 | 1600 | 25 |
| 30.25 | 2.9524 | 356 | 6 |
| 30.90 | 2.89137 | 415 | 7 |
| 31.85 | 2.80749 | 552 | 9 |
| 32.21 | 2.77673 | 451 | 7 |
| 32.64 | 2.74161 | 284 | 5 |
| 33.90 | 2.64223 | 512 | 8 |
| 34.61 | 2.58931 | 514 | 8 |
| 36.06 | 2.48854 | 331 | 5 |
| 38.29 | 2.34861 | 277 | 4 |

**Table 58. XRPD Peak positions of the Triethanolamine salt of Compound A from the experiment L100110-68-44 (Form 9-B).**

| 2-θ | d (A°) | Height (counts) | Relative Intensity, % |
|---|---|---|---|
| 7.38 | 11.96556 | 5277 | 65 |
| 9.80 | 9.01543 | 390 | 5 |
| 10.92 | 8.09534 | 4218 | 52 |
| 12.23 | 7.23046 | 1276 | 16 |
| 12.88 | 6.86771 | 988 | 12 |
| 14.69 | 6.02464 | 1947 | 24 |
| 15.42 | 5.74026 | 2803 | 35 |
| 16.32 | 5.42746 | 3141 | 39 |
| 16.85 | 5.25854 | 990 | 12 |
| 17.76 | 4.99134 | 563 | 7 |
| 18.10 | 4.89692 | 874 | 11 |
| 18.75 | 4.72947 | 326 | 4 |
| 19.12 | 4.63815 | 1555 | 19 |
| 19.77 | 4.48789 | 1384 | 17 |
| 20.35 | 4.35999 | 1615 | 20 |
| 21.48 | 4.13368 | 8083 | 100 |
| 22.59 | 3.9329 | 4036 | 50 |
| 23.51 | 3.78062 | 794 | 10 |
| 24.05 | 3.69679 | 641 | 8 |
| 25.98 | 3.42691 | 1015 | 13 |
| 26.36 | 3.37815 | 1606 | 20 |
| 26.64 | 3.34315 | 1666 | 21 |
| 27.33 | 3.26019 | 830 | 10 |
| 29.61 | 3.01471 | 1116 | 14 |
| 30.13 | 2.96366 | 976 | 12 |
| 30.80 | 2.90049 | 437 | 5 |
| 31.62 | 2.82771 | 825 | 10 |
| 34.05 | 2.6308 | 453 | 6 |

**Table 59. XRPD Peak positions of the Triethanolamine salt of Compound A from the experiment L100110-68-41 after drying and subjecting it to saturated humidity environment at RT (Form 9-C).**

| 2-θ | d (A°) | Height (counts) | Relative Intensity, % |
|---|---|---|---|
| 4.65 | 18.97718 | 27633 | 100 |
| 5.90 | 14.98024 | 2297 | 8 |
| 9.24 | 9.56562 | 16687 | 60 |
| 11.76 | 7.52087 | 1280 | 5 |
| 13.83 | 6.40025 | 8526 | 31 |
| 23.14 | 3.8407 | 1237 | 4 |

**Table 60. XRPD Peak positions of the Triethanolamine salt of Compound A from the experiment L100110-68-44 after drying, subjecting it to saturated humidity environment at RT (Form 9-D).**

| 2-θ | d (A°) | Height (counts) | Relative Intensity, % |
|---|---|---|---|
| 4.67 | 18.91096 | 1331 | 82 |
| 5.89 | 14.98856 | 1237 | 76 |
| 7.51 | 11.7633 | 346 | 21 |
| 8.31 | 10.62569 | 1619 | 100 |
| 8.89 | 9.94186 | 373 | 23 |
| 9.23 | 9.57156 | 740 | 46 |
| 11.39 | 7.76057 | 466 | 29 |
| 11.72 | 7.54559 | 656 | 41 |
| 12.46 | 7.1005 | 1137 | 70 |
| 12.81 | 6.90253 | 125 | 8 |
| 13.81 | 6.40901 | 327 | 20 |
| 15.24 | 5.80955 | 238 | 15 |
| 16.65 | 5.32042 | 390 | 24 |
| 17.72 | 5.00127 | 206 | 13 |
| 18.76 | 4.72621 | 189 | 12 |
| 20.30 | 4.37048 | 137 | 8 |
| 20.57 | 4.31356 | 173 | 11 |
| 21.04 | 4.21902 | 100 | 6 |
| 21.78 | 4.07639 | 87 | 5 |
| 22.53 | 3.94262 | 694 | 43 |
| 23.28 | 3.81761 | 263 | 16 |
| 24.42 | 3.64237 | 134 | 8 |
| 24.81 | 3.58583 | 87 | 5 |
| 26.11 | 3.41021 | 170 | 11 |
| 26.82 | 3.32193 | 189 | 12 |
| 28.09 | 3.1742 | 118 | 7 |
| 29.33 | 3.04314 | 248 | 15 |

**Table 61. XRPD Peak positions of the Triethanolamine salt of Compound A from the experiment L100110-68-45 (Form 9-E).**

| 2-θ | d (A°) | Height (counts) | Relative Intensity, % |
|---|---|---|---|
| 7.89 | 11.19788 | 3398 | 100 |
| 10.50 | 8.41706 | 972 | 29 |
| 11.23 | 7.87285 | 1747 | 51 |
| 11.78 | 7.50953 | 413 | 12 |
| 12.57 | 7.03403 | 862 | 25 |
| 13.86 | 6.38651 | 245 | 7 |
| 15.76 | 5.61774 | 356 | 10 |
| 16.51 | 5.36604 | 840 | 25 |
| 17.38 | 5.09913 | 1466 | 43 |
| 19.00 | 4.66637 | 1515 | 45 |
| 20.25 | 4.38149 | 405 | 12 |
| 20.89 | 4.24944 | 216 | 6 |
| 21.36 | 4.1573 | 494 | 15 |
| 22.21 | 3.99951 | 396 | 12 |
| 23.57 | 3.77205 | 680 | 20 |
| 24.16 | 3.68124 | 219 | 6 |
| 25.21 | 3.52941 | 301 | 9 |
| 26.39 | 3.37443 | 210 | 6 |
| 27.11 | 3.28616 | 512 | 15 |
| 28.88 | 3.08853 | 179 | 5 |
| 29.63 | 3.01231 | 124 | 4 |
| 30.16 | 2.96059 | 323 | 10 |
| 31.73 | 2.81796 | 170 | 5 |
| 36.31 | 2.47234 | 122 | 4 |

**Table 62. XRPD Peak positions of the Diethylamine salt of Compound A from the scale-up experiment L100110-85-9 (Form 10-A).**

| 2-θ | d (A°) | Height (counts) | Relative Intensity, % |
|---|---|---|---|
| 7.02 | 12.58251 | 84 | 8 |
| 8.40 | 10.52239 | 130 | 12 |
| 9.04 | 9.77431 | 441 | 40 |
| 9.81 | 9.00901 | 534 | 49 |
| 11.66 | 7.58328 | 45 | 4 |
| 12.10 | 7.3101 | 163 | 15 |
| 12.34 | 7.16522 | 406 | 37 |
| 13.70 | 6.45893 | 113 | 10 |
| 14.76 | 5.99498 | 1091 | 100 |
| 18.56 | 4.77732 | 57 | 5 |
| 19.87 | 4.46419 | 86 | 8 |
| 20.91 | 4.24543 | 55 | 5 |
| 21.72 | 4.08808 | 63 | 6 |
| 23.14 | 3.84024 | 138 | 13 |
| 24.25 | 3.66686 | 182 | 17 |
| 26.36 | 3.3785 | 73 | 7 |

**Table 63. XRPD Peak positions of the Diethylamine salt of Compound A from the experiment L100110-68-46 followed by drying (Form 10-C).**

| 2-θ | d (A°) | Height (counts) | Relative Intensity, % |
|---|---|---|---|
| 6.16 | 14.34705 | 912 | 34 |
| 8.99 | 9.83108 | 247 | 9 |
| 10.01 | 8.83204 | 1422 | 53 |
| 12.40 | 7.13058 | 379 | 14 |
| 14.31 | 6.18306 | 1150 | 43 |
| 15.09 | 5.86776 | 2671 | 100 |
| 17.07 | 5.1892 | 502 | 19 |
| 20.95 | 4.23783 | 590 | 22 |
| 21.75 | 4.08304 | 121 | 5 |
| 23.25 | 3.82268 | 800 | 30 |
| 24.11 | 3.68828 | 134 | 5 |
| 26.34 | 3.3803 | 169 | 6 |
| 27.86 | 3.20014 | 121 | 5 |
| 29.00 | 3.07656 | 178 | 7 |
| 30.40 | 2.93771 | 240 | 9 |
| 32.92 | 2.71843 | 133 | 5 |

**Table 64. XRPD Peak positions of the Diethylamine salt of Compound A from the experiment L100110-68-49 (Form 10-B + extra minor peaks).**

| 2-θ | d (A°) | Height (counts) | Relative Intensity, % |
|---|---|---|---|
| 6.28 | 14.0724 | 600 | 34 |
| 7.10 | 12.44613 | 841 | 47 |
| 8.32 | 10.62079 | 630 | 36 |
| 8.96 | 9.86277 | 1394 | 79 |
| 9.71 | 9.1011 | 872 | 49 |
| 12.00 | 7.36715 | 1020 | 57 |
| 12.31 | 7.18294 | 417 | 23 |
| 13.58 | 6.5176 | 347 | 20 |
| 14.74 | 6.0039 | 1774 | 100 |
| 18.43 | 4.81058 | 289 | 16 |
| 21.76 | 4.0812 | 423 | 24 |
| 22.93 | 3.87536 | 363 | 20 |
| 24.16 | 3.68081 | 251 | 14 |
| 25.12 | 3.54196 | 268 | 15 |
| 26.24 | 3.39324 | 161 | 9 |
| 28.54 | 3.12516 | 137 | 8 |
| 29.68 | 3.00722 | 159 | 9 |
| 30.68 | 2.91168 | 159 | 9 |
| 31.58 | 2.83112 | 68 | 4 |

**Table 65. XRPD Peak positions of the Ethanol-2-diethylamine salt of Compound A from the experiment L100110-68-56 (Form 12-A).**

| 2-θ | d (A°) | Height (counts) | Relative Intensity, % |
|---|---|---|---|
| 4.82 | 18.33542 | 3585 | 99 |
| 9.61 | 9.19525 | 2275 | 63 |
| 10.44 | 8.46428 | 695 | 19 |
| 11.23 | 7.87607 | 2935 | 81 |
| 11.52 | 7.67196 | 778 | 21 |
| 12.26 | 7.21549 | 737 | 20 |
| 13.40 | 6.60445 | 557 | 15 |
| 14.01 | 6.31512 | 3624 | 100 |
| 14.47 | 6.11723 | 1416 | 39 |
| 14.85 | 5.95909 | 2576 | 71 |
| 15.81 | 5.60104 | 568 | 16 |
| 16.47 | 5.37784 | 286 | 8 |
| 17.68 | 5.01192 | 1555 | 43 |
| 18.90 | 4.69128 | 1993 | 55 |
| 19.28 | 4.59981 | 385 | 11 |
| 19.72 | 4.49874 | 1176 | 32 |
| 19.95 | 4.44596 | 1335 | 37 |
| 20.31 | 4.36967 | 1503 | 41 |
| 20.93 | 4.24163 | 1275 | 35 |
| 21.59 | 4.11201 | 2345 | 65 |
| 22.65 | 3.92246 | 660 | 18 |
| 23.15 | 3.8388 | 1501 | 41 |
| 23.75 | 3.74355 | 655 | 18 |
| 24.55 | 3.62312 | 357 | 10 |
| 25.00 | 3.55876 | 1101 | 30 |
| 25.65 | 3.46996 | 934 | 26 |
| 26.10 | 3.41201 | 978 | 27 |
| 26.51 | 3.35989 | 685 | 19 |
| 27.27 | 3.26723 | 976 | 27 |
| 28.16 | 3.16683 | 996 | 27 |
| 28.62 | 3.11687 | 363 | 10 |
| 29.45 | 3.03042 | 352 | 10 |
| 30.70 | 2.90964 | 438 | 12 |
| 31.82 | 2.80995 | 355 | 10 |
| 32.78 | 2.73024 | 333 | 9 |
| 33.31 | 2.6875 | 161 | 4 |
| 34.38 | 2.60642 | 263 | 7 |
| 35.17 | 2.54987 | 231 | 6 |
| 38.46 | 2.33859 | 194 | 5 |

**Table 66. XRPD Peak positions of the Ethanol-2-diethylamine salt of Compound A from the experiment L100110-68-60 (Form 12-B).**

| 2-θ | d (A°) | Height (counts) | Relative Intensity, % |
|---|---|---|---|
| 4.79 | 18.44824 | 1410 | 100 |
| 9.10 | 9.70753 | 729 | 52 |
| 12.32 | 7.17607 | 92 | 7 |
| 15.61 | 5.67238 | 85 | 6 |
| 16.39 | 5.40362 | 298 | 21 |
| 22.87 | 3.8849 | 82 | 6 |
| 26.80 | 3.32352 | 57 | 4 |

**Table 67. XRPD Peak positions of the Ethanol-2-diethylamine salt of Compound A from the experiment L100110-68-60 after subjecting it to saturated humidity environment at RT (Form 12-C).**

| 2-θ | d (A°) | Height (counts) | Relative Intensity, % |
|---|---|---|---|
| 4.40 | 20.07702 | 83 | 34 |
| 5.86 | 15.08102 | 246 | 100 |
| 8.63 | 10.23481 | 99 | 40 |
| 9.01 | 9.80211 | 27 | 11 |
| 11.78 | 7.50911 | 142 | 58 |
| 15.40 | 5.74964 | 12 | 5 |
| 17.67 | 5.01424 | 18 | 7 |
| 19.73 | 4.49648 | 11 | 4 |
| 22.53 | 3.94246 | 32 | 13 |
| 23.73 | 3.74702 | 20 | 8 |

**Table 68. XRPD Peak positions of the Choline hydroxide salt of Compound A from the experiment L100110-68-64 (Form 13-A).**

| 2-θ | d (A°) | Height (counts) | Relative Intensity, % |
|---|---|---|---|
| 5.36 | 16.48646 | 1214 | 27 |
| 8.62 | 10.25278 | 3256 | 74 |
| 10.53 | 8.39598 | 1222 | 28 |
| 12.28 | 7.19929 | 794 | 18 |
| 14.64 | 6.04675 | 1023 | 23 |
| 14.88 | 5.95004 | 2588 | 58 |
| 17.53 | 5.05496 | 4427 | 100 |
| 18.49 | 4.79444 | 1660 | 37 |
| 19.02 | 4.66273 | 343 | 8 |
| 20.04 | 4.42651 | 1484 | 34 |
| 20.83 | 4.26197 | 2404 | 54 |
| 21.60 | 4.11151 | 1316 | 30 |
| 24.02 | 3.70146 | 657 | 15 |
| 25.40 | 3.50333 | 1003 | 23 |
| 26.71 | 3.33426 | 529 | 12 |
| 27.57 | 3.23237 | 311 | 7 |
| 28.37 | 3.14285 | 583 | 13 |
| 28.96 | 3.08034 | 869 | 20 |
| 30.46 | 2.93267 | 238 | 5 |
| 31.55 | 2.8338 | 201 | 5 |
| 32.98 | 2.71412 | 227 | 5 |
| 33.94 | 2.63946 | 263 | 6 |
| 36.61 | 2.45263 | 159 | 4 |

**Table 69. XRPD Peak positions of the L-Arginine salt of Compound A from the experiment L100110-68-66 (Form 14-A).**

| 2-θ | d (A°) | Height (counts) | Relative Intensity, % |
|---|---|---|---|
| 6.99 | 12.62765 | 5901 | 100 |
| 7.93 | 11.13591 | 5751 | 97 |
| 9.74 | 9.07485 | 2946 | 50 |
| 13.38 | 6.6107 | 5532 | 94 |
| 13.98 | 6.32999 | 1790 | 30 |
| 14.91 | 5.93592 | 1086 | 18 |
| 16.13 | 5.49144 | 2027 | 34 |
| 18.25 | 4.85601 | 1740 | 29 |
| 18.83 | 4.70865 | 546 | 9 |
| 19.42 | 4.56677 | 1325 | 22 |
| 19.79 | 4.48263 | 2178 | 37 |
| 20.49 | 4.33104 | 571 | 10 |
| 20.86 | 4.25455 | 1613 | 27 |
| 22.21 | 3.99997 | 454 | 8 |
| 22.67 | 3.91846 | 1078 | 18 |
| 23.90 | 3.72061 | 2028 | 34 |
| 25.31 | 3.51592 | 502 | 9 |
| 25.84 | 3.44493 | 345 | 6 |
| 26.71 | 3.33517 | 238 | 4 |
| 28.20 | 3.16206 | 866 | 15 |
| 31.08 | 2.87541 | 334 | 6 |
| 32.61 | 2.74333 | 251 | 4 |
| 33.24 | 2.69275 | 214 | 4 |
| 36.01 | 2.49181 | 262 | 4 |

**Table 70. XRPD Peak positions of the L-Arginine salt of Compound A from the experiment L100110-68-68 (Form 14-B).**

| 2-θ | d (A°) | Height (counts) | Relative Intensity, % |
|---|---|---|---|
| 7.02 | 12.57436 | 2969 | 55 |
| 7.99 | 11.05088 | 5413 | 100 |
| 9.14 | 9.66904 | 2398 | 44 |
| 9.59 | 9.21739 | 2602 | 48 |
| 13.26 | 6.67243 | 2652 | 49 |
| 13.97 | 6.33294 | 1452 | 27 |
| 15.13 | 5.85036 | 466 | 9 |
| 15.79 | 5.60747 | 960 | 18 |
| 16.49 | 5.37226 | 1496 | 28 |
| 18.48 | 4.79717 | 3018 | 56 |
| 19.20 | 4.61895 | 518 | 10 |
| 20.20 | 4.39182 | 1717 | 32 |
| 21.00 | 4.22667 | 1198 | 22 |
| 21.30 | 4.16729 | 594 | 11 |
| 22.40 | 3.96495 | 458 | 8 |
| 22.99 | 3.86461 | 1490 | 28 |
| 23.65 | 3.75831 | 1403 | 26 |
| 24.00 | 3.70474 | 1702 | 31 |
| 25.29 | 3.51851 | 415 | 8 |
| 26.07 | 3.41562 | 586 | 11 |
| 26.45 | 3.36662 | 531 | 10 |
| 27.36 | 3.25689 | 331 | 6 |
| 28.08 | 3.17565 | 373 | 7 |
| 28.57 | 3.1222 | 880 | 16 |
| 30.35 | 2.94229 | 355 | 7 |
| 31.20 | 2.86416 | 636 | 12 |
| 34.23 | 2.6172 | 281 | 5 |

**Table 71. XRPD Peak positions of the L-Arginine salt of Compound A from the experiment L100110-68-69 (Form 14-C).**

| 2-θ | d (A°) | Height (counts) | Relative Intensity, % |
|---|---|---|---|
| 4.10 | 21.52352 | 143 | 21 |
| 5.50 | 16.05026 | 669 | 100 |
| 7.05 | 12.52711 | 358 | 53 |
| 7.50 | 11.78467 | 259 | 39 |
| 8.91 | 9.91629 | 198 | 30 |
| 9.48 | 9.32647 | 76 | 11 |
| 10.68 | 8.2747 | 106 | 16 |
| 12.42 | 7.12201 | 59 | 9 |
| 15.85 | 5.58547 | 451 | 67 |
| 16.32 | 5.42607 | 38 | 6 |
| 16.77 | 5.28335 | 34 | 5 |
| 18.09 | 4.90053 | 129 | 19 |
| 19.40 | 4.57114 | 65 | 10 |
| 20.30 | 4.37134 | 187 | 28 |
| 21.70 | 4.0916 | 132 | 20 |
| 24.26 | 3.66567 | 117 | 17 |
| 28.05 | 3.17894 | 91 | 14 |

**Table 72. XRPD Peak positions of the L-Arginine salt of Compound A from the experiment L100110-68-70 after drying (Form 14-E).**

| 2-θ | d (A°) | Height (counts) | Relative Intensity, % |
|---|---|---|---|
| 6.95 | 12.7084 | 765 | 100 |
| 9.03 | 9.78635 | 695 | 91 |
| 9.68 | 9.12658 | 84 | 11 |
| 10.90 | 8.11047 | 406 | 53 |
| 13.34 | 6.63191 | 139 | 18 |
| 14.20 | 6.23263 | 267 | 35 |
| 14.55 | 6.08369 | 411 | 54 |
| 19.45 | 4.55982 | 517 | 68 |
| 20.03 | 4.4292 | 71 | 9 |
| 21.07 | 4.21385 | 144 | 19 |
| 23.36 | 3.80567 | 188 | 25 |
| 24.09 | 3.69069 | 57 | 7 |
| 26.59 | 3.34973 | 78 | 10 |
| 28.41 | 3.13937 | 258 | 34 |

**Table 73. XRPD Peak positions of the L-Histidine salt of Compound A from the experiment L100110-68-71 after drying (Form 15-A).**

| 2-θ | d (A°) | Height (counts) | Relative Intensity, % |
|---|---|---|---|
| 6.91 | 12.79054 | 3644 | 100 |
| 7.72 | 11.4422 | 1797 | 49 |
| 7.91 | 11.17124 | 655 | 18 |
| 9.43 | 9.36766 | 287 | 8 |
| 10.36 | 8.53008 | 167 | 5 |
| 12.06 | 7.33127 | 186 | 5 |
| 13.80 | 6.41071 | 241 | 7 |
| 14.34 | 6.17179 | 724 | 20 |
| 15.30 | 5.78822 | 183 | 5 |
| 16.91 | 5.23987 | 241 | 7 |
| 18.19 | 4.87352 | 253 | 7 |
| 18.96 | 4.67646 | 2265 | 62 |
| 20.38 | 4.35314 | 1019 | 28 |
| 21.14 | 4.19991 | 1195 | 33 |
| 22.17 | 4.00622 | 212 | 6 |
| 22.47 | 3.95426 | 166 | 5 |
| 23.03 | 3.85829 | 1255 | 34 |
| 24.01 | 3.70303 | 825 | 23 |
| 25.53 | 3.48635 | 140 | 4 |
| 26.00 | 3.42485 | 151 | 4 |
| 27.51 | 3.2394 | 199 | 5 |
| 28.21 | 3.16054 | 344 | 9 |
| 29.25 | 3.05057 | 164 | 5 |
| 29.84 | 2.99196 | 149 | 4 |

**Table 74. XRPD Peak positions of the L-Histidine salt of Compound A from the experiment L100110-68-71 after drying, followed by subjecting it to saturated humidity environment at RT (Form 15-B).**

| 2-θ | d (A°) | Height (counts) | Relative Intensity, % |
|---|---|---|---|
| 5.88 | 15.0202 | 2317 | 82 |
| 9.49 | 9.31552 | 244 | 9 |
| 10.16 | 8.69923 | 237 | 8 |
| 11.75 | 7.52658 | 1143 | 41 |
| 13.13 | 6.73636 | 126 | 4 |
| 14.57 | 6.07544 | 394 | 14 |
| 15.28 | 5.79368 | 137 | 5 |
| 18.96 | 4.67646 | 2818 | 100 |
| 21.16 | 4.19451 | 672 | 24 |
| 22.61 | 3.92956 | 349 | 12 |
| 23.25 | 3.82284 | 232 | 8 |
| 24.15 | 3.68265 | 477 | 17 |
| 25.25 | 3.52418 | 370 | 13 |
| 26.30 | 3.38575 | 455 | 16 |
| 26.68 | 3.33798 | 311 | 11 |
| 27.52 | 3.23858 | 218 | 8 |

**Table 75. XRPD Peak positions of the L-Histidine salt of Compound A from the experiment L100110-68-72 after drying (Form 15-C).**

| 2-θ | d (A°) | Height (counts) | Relative Intensity, % |
|---|---|---|---|
| 9.50 | 9.30504 | 142 | 12 |
| 15.32 | 5.77725 | 292 | 25 |
| 18.99 | 4.66879 | 1169 | 100 |
| 21.14 | 4.19944 | 1171 | 100 |
| 22.12 | 4.01482 | 91 | 8 |
| 22.54 | 3.94098 | 115 | 10 |
| 24.16 | 3.68131 | 700 | 60 |
| 29.88 | 2.98821 | 226 | 19 |

**Table 76. XRPD Peak positions of the L-Histidine salt of Compound A from the experiment L100110-68-75 before drying (Form 15-D).**

| 2-θ | d (A°) | Height (counts) | Relative Intensity, % |
|---|---|---|---|
| 9.40 | 9.40551 | 76 | 13 |
| 10.35 | 8.5374 | 111 | 19 |
| 11.47 | 7.7104 | 369 | 64 |
| 15.33 | 5.77342 | 347 | 60 |
| 15.85 | 5.5869 | 244 | 42 |
| 16.82 | 5.26627 | 294 | 51 |
| 18.97 | 4.67518 | 525 | 91 |
| 21.19 | 4.1889 | 578 | 100 |
| 21.85 | 4.06511 | 168 | 29 |
| 24.17 | 3.67863 | 399 | 69 |
| 29.87 | 2.98917 | 64 | 11 |

**Table 77. XRPD Peak positions of the L-Histidine salt of Compound A from the experiment L100110-68-75 (Form 15-E).**

| 2-θ | d (A°) | Height (counts) | Relative Intensity, % |
|---|---|---|---|
| 5.94 | 14.87186 | 633 | 24 |
| 6.36 | 13.89127 | 1184 | 45 |
| 8.05 | 10.97998 | 643 | 25 |
| 9.50 | 9.3007 | 339 | 13 |
| 10.50 | 8.41928 | 2219 | 85 |
| 11.16 | 7.91943 | 394 | 15 |
| 11.78 | 7.50728 | 267 | 10 |
| 14.59 | 6.06685 | 484 | 19 |
| 15.37 | 5.7587 | 969 | 37 |
| 17.67 | 5.01613 | 227 | 9 |
| 18.14 | 4.88751 | 481 | 18 |
| 18.66 | 4.75027 | 99 | 4 |
| 18.98 | 4.67148 | 1986 | 76 |
| 21.15 | 4.19792 | 2605 | 100 |
| 22.54 | 3.94077 | 262 | 10 |
| 24.13 | 3.68452 | 1676 | 64 |
| 24.63 | 3.61185 | 915 | 35 |
| 25.38 | 3.50607 | 820 | 31 |
| 29.87 | 2.98839 | 397 | 15 |
| 32.16 | 2.78137 | 215 | 8 |
| 33.82 | 2.648 | 121 | 5 |
| 37.76 | 2.38075 | 524 | 20 |

**Table 78. XRPD Peak positions of the L-Lysine salt of Compound A from the experiment L100110-68-79 (Form 16-A).**

| 2-θ | d (A°) | Height (counts) | Relative Intensity, % |
|---|---|---|---|
| 7.12 | 12.40428 | 932 | 18 |
| 8.70 | 10.15282 | 2290 | 43 |
| 9.22 | 9.58923 | 5315 | 100 |
| 11.33 | 7.80251 | 1586 | 30 |
| 14.21 | 6.22779 | 448 | 8 |
| 15.89 | 5.57162 | 223 | 4 |
| 17.04 | 5.19801 | 2428 | 46 |
| 17.42 | 5.08617 | 708 | 13 |
| 18.36 | 4.82873 | 967 | 18 |
| 19.06 | 4.65208 | 1000 | 19 |
| 20.42 | 4.34592 | 952 | 18 |
| 21.45 | 4.14016 | 310 | 6 |
| 21.89 | 4.0566 | 788 | 15 |
| 22.56 | 3.93721 | 263 | 5 |
| 23.60 | 3.76691 | 399 | 8 |
| 24.02 | 3.70199 | 454 | 9 |
| 24.80 | 3.58779 | 1433 | 27 |
| 25.73 | 3.45912 | 805 | 15 |
| 27.00 | 3.3 | 333 | 6 |
| 27.52 | 3.23908 | 232 | 4 |
| 28.26 | 3.15499 | 204 | 4 |
| 34.15 | 2.62379 | 258 | 5 |
| 35.10 | 2.55487 | 214 | 4 |

**Table 79. XRPD Peak positions of the Meglumine salt of Compound A from the experiment L100110-68-82 (Form 17-A).**

| 2-θ | d (A°) | Height (counts) | Relative Intensity, % |
|---|---|---|---|
| 7.34 | 12.03989 | 2788 | 100 |
| 9.83 | 8.99314 | 155 | 6 |
| 10.72 | 8.2425 | 504 | 18 |
| 12.98 | 6.81658 | 138 | 5 |
| 15.56 | 5.69055 | 1896 | 68 |
| 16.77 | 5.28219 | 432 | 15 |
| 18.44 | 4.80783 | 433 | 16 |
| 19.24 | 4.60992 | 416 | 15 |
| 21.59 | 4.11228 | 1376 | 49 |
| 22.25 | 3.99202 | 580 | 21 |
| 26.25 | 3.3926 | 114 | 4 |
| 29.06 | 3.06999 | 175 | 6 |
| 37.08 | 2.42261 | 104 | 4 |

**Table 80. XRPD Peak positions of the Meglumine salt of Compound A from the experiment L100110-68-85 (Form 17-B).**

| 2-θ | d (A°) | Height (counts) | Relative Intensity, % |
|---|---|---|---|
| 7.50 | 11.77025 | 2296 | 100 |
| 11.04 | 8.01083 | 465 | 20 |
| 11.46 | 7.71675 | 604 | 26 |
| 12.22 | 7.23718 | 224 | 10 |
| 13.32 | 6.64298 | 310 | 14 |
| 15.60 | 5.67428 | 254 | 11 |
| 17.19 | 5.15324 | 598 | 26 |
| 17.95 | 4.9379 | 275 | 12 |
| 19.29 | 4.59784 | 449 | 20 |
| 20.32 | 4.36618 | 153 | 7 |
| 22.19 | 4.00331 | 691 | 30 |
| 22.84 | 3.89095 | 212 | 9 |
| 24.15 | 3.68285 | 246 | 11 |
| 26.36 | 3.37794 | 350 | 15 |
| 28.38 | 3.14239 | 564 | 25 |
| 29.85 | 2.99034 | 91 | 4 |

### Equivalents

While the present invention has been described in conjunction with the specific embodiments set forth above, many alternatives, modifications and other variations thereof will be apparent to those of ordinary skill in the art. All such alternatives, modifications and variations are intended to fall within the spirit and scope of the present invention.

### Numbered Embodiments:

1. A crystalline salt of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile ("Compound A").
2. The crystalline salt of Embodiment 1, characterized as having a counter-ion, wherein the counter-ion is selected from L-lysine, L-arginine, 2-hydroxy-N,N,N-trimethylethan-1-aminium, diethylamine, ethanolamine, ethanol-2-diethylamine, Na⁺, Mg²⁺, K⁺, Ca²⁺, diethanolamine, triethanolamine, L-histidine, and meglumine.
3. The crystalline salt of Embodiment 1, wherein the counter-ion is L-lysine.
4. The crystalline salt of Embodiment 1, wherein the counter-ion is L-arginine
5. The crystalline salt of Embodiment 1, wherein the counter-ion is 2-hydroxy-N,N,N-trimethylethan-1-aminium.
6. The crystalline salt of Embodiment 3, characterized by an X-ray powder diffraction pattern including peaks at about 8.70, 9.22, 11.3, 17.0, and 24.8 degrees 2θ, wherein the x-ray powder diffraction pattern is obtained using a Cu Kα radiation source (1.54 Å).
7. The crystalline salt of Embodiment 3, having an X-ray diffraction pattern substantially similar to that set forth in FIG. 76.
8. The crystalline salt of Embodiment 3, having a melting point of about 250 °C.
9. The crystalline salt of Embodiment 4, having an X-ray diffraction pattern substantially similar to that set forth in any one of FIGs. 67-70.
10. The crystalline salt of Embodiment 4, having a melting point of about 200 °C.
11. The crystalline salt of Embodiment 5, having an X-ray diffraction pattern substantially similar to that set forth in FIG. 66.
12. The crystalline salt of Embodiment 5, having a melting point of about 229 °C.
13. The crystalline salt of any one of Embodiments 1-12, having purity of Compound A of greater than 90% by weight.
14. The crystalline salt of any one of Embodiments 1-12, having purity of Compound A of greater than 95% by weight.
15. The crystalline salt of any one of Embodiments 1-12, having purity of Compound A of greater than 99% by weight.
16. A pharmaceutical composition comprising the crystalline salt of any one of Embodiments 1-15.
17. A morphic form (Form B) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 5.92, 11.8, and 17.5 degrees 2θ, wherein the x-ray powder diffraction pattern is obtained using a Cu Kα radiation source (1.54 Å).
18. The morphic form of Embodiment 17, having an X-ray diffraction pattern substantially similar to that set forth in FIG. 2.
19. A morphic form (Form C) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 5.74, 11.5, 17.7, 19.3, 19.7, 21.4, 24.3 degrees 2θ, wherein the x-ray powder diffraction pattern is obtained using a Cu Kα radiation source (1.54 Å).
20. The morphic form of Embodiment 19, having an X-ray diffraction pattern substantially similar to that set forth in FIG. 3.
21. A morphic form (Form D) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 5.52, 8.52, 11.0, 16.5, 18.3, 21.0, 21.2, and 24.0 degrees 2θ, wherein the x-ray powder diffraction pattern is obtained using a Cu Kα radiation source (1.54 Å).
22. The morphic form of Embodiment 21, having an X-ray diffraction pattern substantially similar to that set forth in FIG. 4.
23. A morphic form (Form E) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 7.13, 10.8, 12.3, 14.1, 14.7, 15.5, 16.1, 17.5, 18.1, 19.9, 20.2, 21.0, 21.2, 22.7, 22.9, 24.4, 25.3, and 26.3 degrees 2θ, wherein the x-ray powder diffraction pattern is obtained using a Cu Kα radiation source (1.54 Å).
24. The morphic form of Embodiment 23, having an X-ray diffraction pattern substantially similar to that set forth in FIG. 5.
25. A morphic form (Form F) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.1, 10.4, 11.4, 13.9, 16.2, 16.4, 17.1, 22.0, 23.8, and 29.5 degrees 2θ, wherein the x-ray powder diffraction pattern is obtained using a Cu Kα radiation source (1.54 Å).
26. The morphic form of Embodiment 25, having an X-ray diffraction pattern substantially similar to that set forth in FIG. 6.
27. A morphic form (Form G) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 9.50, 12.9, 16.7, 17.3, 19.5, 20.2, 25.6, and 28.3 degrees 2θ, wherein the x-ray powder diffraction pattern is obtained using a Cu Kα radiation source (1.54 Å).
28. The morphic form of Embodiment 27, having an X-ray diffraction pattern substantially similar to that set forth in FIG. 7.
29. A morphic form (Form H) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 9.22, 19.8, 23.6, 25.9, and 28.0 degrees 2θ, wherein the x-ray powder diffraction pattern is obtained using a Cu Kα radiation source (1.54 Å).
30. The morphic form of Embodiment 29, having an X-ray diffraction pattern substantially similar to that set forth in FIG. 8.
31. A morphic form (Form I) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 5.77, 9.30, 10.2, 11.6, and 21.9 degrees 2θ, wherein the x-ray powder diffraction pattern is obtained using a Cu Kα radiation source (1.54 Å).
32. The morphic form of Embodiment 31, having an X-ray diffraction pattern substantially similar to that set forth in FIG. 9.
33. A morphic form (Form K) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 8.42, 11.4, 14.5, 18.9, 21.1, and 21.6 degrees 2θ, wherein the x-ray powder diffraction pattern is obtained using a Cu Kα radiation source (1.54 Å).
34. The morphic form of Embodiment 33, having an X-ray diffraction pattern substantially similar to that set forth in FIG. 10.
35. A morphic form (Form L) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.5, 11.5, 11.9, 15.2, 15.7, 16.0, 16.9, 17.1, 18.4, 18.7, 22.0, 22.8, 23.5, and 26.4 degrees 2θ, wherein the x-ray powder diffraction pattern is obtained using a Cu Kα radiation source (1.54 Å).
36. The morphic form of Embodiment 35, having an X-ray diffraction pattern substantially similar to that set forth in FIG. 11.
37. A morphic form (Form S+T) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 7.42, 10.5, 11.3, 12.4, 14.3, 15.8, 16.8, 17.7, 18.1, 18.4, 20.1, 20.5, 21.1, 21.9, 23.2, 25.5, 26.9, and 28.3 degrees 2θ, wherein the x-ray powder diffraction pattern is obtained using a Cu Kα radiation source (1.54 Å).
38. The morphic form of Embodiment 37, having an X-ray diffraction pattern substantially similar to that set forth in FIG. 12.
39. A morphic form (Form S) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.5, 12.3, 14.4, 15.8, 16.7, 17.7, 18.1, 18.4, 20.1, 20.6, 21.2, 21.9, 23.3, 24.4, 25.5, and 27.8 degrees 2θ, wherein the x-ray powder diffraction pattern is obtained using a Cu Kα radiation source (1.54 Å).
40. The morphic form of Embodiment 39, having an X-ray diffraction pattern substantially similar to that set forth in FIG. 13.
41. A morphic form (Form U) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 5.79, 8.43, 11.4, 11.6, 14.5, 18.9, 21.1, and 21.6 degrees 2θ, wherein the x-ray powder diffraction pattern is obtained using a Cu Kα radiation source (1.54 Å).
42. The morphic form of Embodiment 41, having an X-ray diffraction pattern substantially similar to that set forth in FIG. 14.
43. A morphic form (Form V) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 6.35, 10.6, 15.6, 16.5, 16.8, and 18.3 degrees 2θ, wherein the x-ray powder diffraction pattern is obtained using a Cu Kα radiation source (1.54 Å).
44. The morphic form of Embodiment 43, having an X-ray diffraction pattern substantially similar to that set forth in FIG. 15.
45. A morphic form (Form W) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.1, 10.4, 10.7, 11.7, 13.9, 24.4, and 29.5 degrees 2θ, wherein the x-ray powder diffraction pattern is obtained using a Cu Kα radiation source (1.54 Å).
46. The morphic form of Embodiment 45, having an X-ray diffraction pattern substantially similar to that set forth in FIG. 16.
47. A morphic form (Form X) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 9.66, 10.2, 10.5, 11.2, 18.7, and 24.7 degrees 2θ, wherein the x-ray powder diffraction pattern is obtained using a Cu Kα radiation source (1.54 Å).
48. The morphic form of Embodiment 47, having an X-ray diffraction pattern substantially similar to that set forth in FIG. 17.
49. A morphic form (Form Y) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 6.51, 13.0, 13.3, 19.5, and 24.2 degrees 2θ, wherein the x-ray powder diffraction pattern is obtained using a Cu Kα radiation source (1.54 Å).
50. The morphic form of Embodiment 49, having an X-ray diffraction pattern substantially similar to that set forth in FIG. 18.
51. A morphic form (Form Z) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.6, 11.2, 11.6, 12.0, 14.3, 15.6, 16.2, 17.6, 18.1, 18.7, 24.1, and 24.3 degrees 2θ, wherein the x-ray powder diffraction pattern is obtained using a Cu Kα radiation source (1.54 Å).
52. The morphic form of Embodiment 51, having an X-ray diffraction pattern substantially similar to that set forth in FIG. 19.
53. A morphic form (Form α) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 7.26, 10.1, 10.4, 10.6, 11.9, 13.9, 16.5, 21.9, 22.4, and 24.1 degrees 2θ, wherein the x-ray powder diffraction pattern is obtained using a Cu Kα radiation source (1.54 Å).
54. The morphic form of Embodiment 53, having an X-ray diffraction pattern substantially similar to that set forth in FIG. 20.
55. A morphic form (Form β) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 7.36, 10.5, 14.3, 15.7, 18.3, 20.4, 21.0, 21.8, and 23.2 degrees 2θ, wherein the x-ray powder diffraction pattern is obtained using a Cu Kα radiation source (1.54 Å).
56. The morphic form of Embodiment 55, having an X-ray diffraction pattern substantially similar to that set forth in FIG. 21.
57. A morphic form (Form χ) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 8.53, 11.2, 18.4, 20.1, and 21.4 degrees 2θ, wherein the x-ray powder diffraction pattern is obtained using a Cu Kα radiation source (1.54 Å).
58. The morphic form of Embodiment 57, having an X-ray diffraction pattern substantially similar to that set forth in FIG. 22.
59. A morphic form (Form δ) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.9, 12.1, 14.4, 18.1, 19.6, 24.5, and 27.0 degrees 2θ, wherein the x-ray powder diffraction pattern is obtained using a Cu Kα radiation source (1.54 Å).
60. The morphic form of Embodiment 59, having an X-ray diffraction pattern substantially similar to that set forth in FIG. 23.
61. A morphic form (Form ε) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 5.73, 11.4, 16.6, 17.6, 23.2, and 24.2 degrees 2θ, wherein the x-ray powder diffraction pattern is obtained using a Cu Kα radiation source (1.54 Å).
62. The morphic form of Embodiment 61, having an X-ray diffraction pattern substantially similar to that set forth in FIG. 24.
63. A morphic form (Form ϕ) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 6.95, 13.9, 20.9, 22.3, and 27.9 degrees 2θ, wherein the x-ray powder diffraction pattern is obtained using a Cu Kα radiation source (1.54 Å).
64. The morphic form of Embodiment 63, having an X-ray diffraction pattern substantially similar to that set forth in FIG. 25.
65. A morphic form (Form η) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 6.87, 7.69, 20.5, 23.0, 23.9, and 28.2 degrees 2θ, wherein the x-ray powder diffraction pattern is obtained using a Cu Kα radiation source (1.54 Å).
66. The morphic form of Embodiment 65, having an X-ray diffraction pattern substantially similar to that set forth in FIG. 26.
67. A morphic form (Form λ) of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile, characterized by an X-ray powder diffraction pattern including peaks at about 10.6, 12.0, 14.3, 16.2, 17.6, 18.0, and 24.3 degrees 2θ, wherein the x-ray powder diffraction pattern is obtained using a Cu Kα radiation source (1.54 Å).
68. The morphic form of Embodiment 67, having an X-ray diffraction pattern substantially similar to that set forth in FIG. 27.
69. A co-crystal of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile and glutaric acid, characterized by an X-ray powder diffraction pattern including peaks at about 9.74, 10.8, 11.0, 12.2, 16.1, 17.0, 19.2, 21.9, and 23.1 degrees 2θ, wherein the x-ray powder diffraction pattern is obtained using a Cu Kα radiation source (1.54 Å).
70. The co-crystal of Embodiment 69, having an X-ray diffraction pattern substantially similar to that set forth in FIG. 28.
71. An amorphous solid dispersion of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile ("Compound A"), wherein the amorphous solid dispersion comprises a polymer.
72. The amorphous solid dispersion of Embodiment 71, wherein the polymer is polyvinylpyrrolidone.
73. The amorphous solid dispersion of Embodiment 71, wherein the weight ratio of Compound A over the polymer is about 1:2.
74. The amorphous solid dispersion of Embodiment 71, wherein the weight ratio of Compound A over the polymer is about 1:4.
75. A method for treating a resistance to thyroid hormone (RTH) syndrome in a subject having at least one TRβ mutation, the method comprising administering to the subject a therapeutically effective amount of the crystalline salt, morphic form, co-crystal, or amorphous solid dispersion of any one of Embodiments 1-74.
76. A crystalline salt, morphic form, co-crystal, or amorphous solid dispersion of any one of Embodiments 1-74 for the manufacture of a medicament for treating a resistance to thyroid hormone (RTH) syndrome in a subject having at least one TRβ mutation, wherein the crystalline salt, morphic form, co-crystal, or amorphous solid dispersion of any one of Embodiments 1-74 is for administration to the subject in at least one therapeutically effective amount.
77. A crystalline salt, morphic form, co-crystal, or amorphous solid dispersion of any one of Embodiments 1-74 for use in treating a resistance to thyroid hormone (RTH) syndrome in a subject having at least one TRβ mutation, wherein the crystalline salt, morphic form, co-crystal, or amorphous solid dispersion of any one of Embodiments 1-74 is for administration to the subject in at least one therapeutically effective amount.
78. The method of any of Embodiments 75-77, wherein the subject has obesity, hyperlipidemia, hypercholesterolemia, heterozygous familial hypercholesterolemia, diabetes, non-alcoholic steatohepatitis, fatty liver, fatty liver disease, bone disease, thyroid axis alteration, atherosclerosis, a cardiovascular disorder, tachycardia, hyperkinetic behavior, hypothyroidism, goiter, attention deficit hyperactivity disorder, dyslipidemia, learning disabilities, mental retardation, hearing loss, delayed bone age, neurologic or psychiatric disease or thyroid cancer.
79. The method of any of Embodiments 75-78, wherein the TRβ mutation is selected from the group consisting of a substitution of threonine (T) for the wild type residue alanine (A) at amino acid position 234 of SEQ ID NO: 1 (A234T); a substitution of glutamine (Q) for the wild type residue arginine (R) at amino acid position 243 of SEQ ID NO: 1 (R243Q); a substitution of histidine (H) for the wild type residue arginine (R) at amino acid position 316 of SEQ ID NO: 1 (R316H); and a substitution of threonine (T) for the wild type residue alanine (A) at amino acid position 317 of SEQ ID NO: 1 (A317T).
80. The method of any one of Embodiments 75-79, wherein the crystalline salt, morphic form, co-crystal, or amorphous solid dispersion is administered daily.
81. A method for treating non-alcoholic steatohepatitis in a subject in need thereof, the method comprising administering to the subject a therapeutically effective amount of the crystalline salt, morphic form, co-crystal, or amorphous solid dispersion of any one of Embodiments 1-74.
82. A crystalline salt, morphic form, co-crystal, or amorphous solid dispersion of any one of Embodiments 1-74 for the manufacture of a medicament for treating non-alcoholic steatohepatitis in a subject in need thereof, wherein the crystalline salt, morphic form, co-crystal, or amorphous solid dispersion of any one of Embodiments 1-74 is for administration to the subject in at least one therapeutically effective amount.
83. A crystalline salt, morphic form, co-crystal, or amorphous solid dispersion of any one of Embodiments 1-74 for use in treating non-alcoholic steatohepatitis in a subject in need thereof, wherein the crystalline salt, morphic form, co-crystal, or amorphous solid dispersion of any one of Embodiments 1-74 is for administration to the subject in at least one therapeutically effective amount.
84. The method of any of Embodiments 81-83, wherein the crystalline salt, morphic form, co-crystal, or amorphous solid dispersion is administered daily.
85. A method for treating familial hypercholesterolemia in a subject in need thereof, the method comprising administering to the subject a therapeutically effective amount of the crystalline salt, morphic form, co-crystal, or amorphous solid dispersion of any one of Embodiments 1-74.
86. A crystalline salt, morphic form, co-crystal, or amorphous solid dispersion of any one of Embodiments 1-74 for the manufacture of a medicament for treating familial hypercholesterolemia in a subject in need thereof, wherein the crystalline salt, morphic form, co-crystal, or amorphous solid dispersion of any one of Embodiments 1-74 is for administration to the subject in at least one therapeutically effective amount.
87. A crystalline salt, morphic form, co-crystal, or amorphous solid dispersion of any one of Embodiments 1-74 for use in treating familial hypercholesterolemia in a subject in need thereof, wherein the crystalline salt, morphic form, co-crystal, or amorphous solid dispersion of any one of Embodiments 1-74 is for administration to the subject in at least one therapeutically effective amount.
88. The method of any of Embodiments 85-87, wherein the familial hypercholesterolemia is homozygous or heterozygous.
89. The method of any of Embodiments 85-88, wherein the crystalline salt, morphic form, co-crystal, or amorphous solid dispersion is administered daily.
90. A method for treating fatty liver disease in a subject in need thereof, the method comprising administering to the subject a therapeutically effective amount of the crystalline salt, morphic form, co-crystal, or amorphous solid dispersion of any one of Embodiments 1-74.
91. A crystalline salt, morphic form, co-crystal, or amorphous solid dispersion of any one of Embodiments 1-74 for the manufacture of a medicament for treating fatty liver disease in a subject in need thereof, wherein the crystalline salt, morphic form, co-crystal, or amorphous solid dispersion of any one of Embodiments 1-74 is for administration to the subject in at least one therapeutically effective amount.
92. A crystalline salt, morphic form, co-crystal, or amorphous solid dispersion of any one of Embodiments 1-74 for use in treating fatty liver disease in a subject in need thereof, wherein the crystalline salt, morphic form, co-crystal, or amorphous solid dispersion of any one of Embodiments 1-74 is for administration to the subject in at least one therapeutically effective amount.
93. The method of any of Embodiments 90-92, wherein the crystalline salt, morphic form, co-crystal, or amorphous solid dispersion is administered daily.
94. A method for treating dyslipidemia in a subject in need thereof, the method comprising administering to the subject a therapeutically effective amount of the crystalline salt, morphic form, co-crystal, or amorphous solid dispersion of any one of Embodiments 1-74.
95. A crystalline salt, morphic form, co-crystal, or amorphous solid dispersion of any one of Embodiments 1-74 for the manufacture of a medicament for treating dyslipidemia in a subject in need thereof, wherein the crystalline salt, morphic form, co-crystal, or amorphous solid dispersion of any one of Embodiments 1-74 is for administration to the subject in at least one therapeutically effective amount.
96. A crystalline salt, morphic form, co-crystal, or amorphous solid dispersion of any one of Embodiments 1-74 for use in treating dyslipidemia in a subject in need thereof, wherein the crystalline salt, morphic form, co-crystal, or amorphous solid dispersion of any one of Embodiments 1-74 is for administration to the subject in at least one therapeutically effective amount.
97. The method of any of Embodiments 94-96, wherein the crystalline salt, morphic form, co-crystal, or amorphous solid dispersion is administered daily.
98. The method of any one of Embodiments 75-97, wherein the subject is human.

## Claims

1. A morphic Form λ of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (Compound A), **characterized by** an X-ray powder diffraction pattern including peaks at about 10.6, about 12.0, about 14.3, about 16.2, about 17.6, about 18.0, and about 24.3 degrees 2θ, wherein the x-ray powder diffraction pattern is obtained using a CuKα radiation source (1.54 Å).

2. The morphic form according to claim 1, wherein the X-ray powder diffraction pattern includes one or more additional peaks at about 11.2, about 15.6, about 17.3, and about 22.3 degrees 2θ.

3. The morphic form according to claim 1 or claim 2, having an X-ray powder diffraction pattern substantially similar to that set forth in FIG. 27.

4. The morphic form according to any one of the preceding claims, wherein the morphic form has a purity of greater than 90% by weight, optionally wherein the morphic form has a purity of greater than 95% by weight, further optionally wherein the morphic form has a purity of greater than 99% by weight.

5. A method of forming the morphic Form λ of Compound A as defined in any one of claims 1-4, wherein the method comprises (i) stirring a suspension of Compound A and isopropyl acetate for 2 days, and then filtering the suspension to obtain a solvate, and (ii) heating the solvate in (i).

6. A morphic Form F of 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (Compound A), **characterized by** an X-ray powder diffraction pattern including peaks at about 10.1, about 10.4, about 11.4, about 13.9, about 16.2, about 16.4, about 17.1, about 22.0 about 23.8, and about 29.5 degrees 2θ, wherein the x-ray powder diffraction pattern is obtained using a Cu Kα radiation source (1.54 Å).

7. The morphic form according to claim 6, wherein the X-ray powder diffraction pattern includes one or more additional peaks at about 18.4, about 19.3, about 22.5, and about 28.0 degrees 2θ.

8. The morphic form according to claim 6 or claim 7, having an X-ray powder diffraction pattern substantially similar to that set forth in FIG. 6.

9. The morphic form according to any one of claims 6-8, wherein the morphic form has a purity of greater than 90% by weight, optionally wherein the morphic form has a purity of greater than 95% by weight, further optionally wherein the morphic form has a purity of greater than 99% by weight.

10. A method of forming the morphic Form F of Compound A as defined in any one of claims 6-9, wherein the method comprises (i) stirring a suspension of Compound A and ethyl acetate for 2 days, and then filtering the suspension to obtain a solvate, and (ii) heating the solvate in (i) to 100 °C for 1 hour.

11. A pharmaceutical composition comprising the morphic form as defined in any one of claims 1-4 and 6-9 and at least one pharmaceutically acceptable excipient or carrier.

12. The pharmaceutical composition of claim 11, wherein the pharmaceutical composition is a tablet.

13. A morphic form or pharmaceutical composition as defined in any one of claims 1-4, 6-9, 11 and 12, for use in treating non-alcoholic steatohepatitis in a subject.
